# EUROPEAN PATENT SPECIFICATION

(11) **EP 3 077 510 B1**
(45) Date of publication and mention of the grant of the patent: **06.05.2020**
(21) Application number: 14867872.5
(22) Date of filing: 02.12.2014
(51) Int. Cl.: C12N 15/113, A61K 31/7088

(54) **ANTISENSE COMPOUNDS AND USES THEREOF**
ANTISENSE-VERBINDUNGEN UND VERWENDUNGEN DAVON
COMPOSÉS ANTISENS ET LEURS UTILISATIONS

(30) Priority: 02.12.2013 US 201361910871 P
(43) Date of publication of application: 12.10.2016
(73) Proprietor: Ionis Pharmaceuticals, Inc., Carlsbad, CA 92010 (US); Rosalind Franklin University of Medicine and Science, North Chicago, IL 60064 (US)
(72) Inventor: RIGO, Frank, Carlsbad, CA 92010 (US); HASTINGS, Michelle, L., North Chicago, IL 60064 (US)
(74) Representative: Carpmaels & Ransford LLP
(86) International application number: PCT/US2014/068225
(87) International publication number: WO 2015/084884

(56) References cited:
- WO-A1-97/08308
- WO-A1-2013/020097
- WO-A2-2013/066438
- WO-A2-2013/066438
- WO-A2-2013/118120
- Svetlana N Rylova ET AL: "The CLN3 Gene is a Novel Molecular Target for Cancer Drug Discovery 1", CANCER RESEARCH, 1 February 2002 (2002-02-01), pages 801-808, XP055375663, Retrieved from the Internet: URL:http://cancerres.aacrjournals.org/cont ent/62/3/801.full-text.pdf [retrieved on 2017-05-24]
- GOLABEK ET AL: "CLN3 disease process: missense point mutations and protein depletion in vitro", EUROPEAN JOURNAL OF PAEDIATRIC NEURO, SAUNDERS, PHILADELPHIA, US, vol. 5, 1 January 2001 (2001-01-01), pages 81-88, XP005749602, ISSN: 1090-3798, DOI: 10.1053/EJPN.2000.0440
- SUMEER DHAR ET AL: "Flupirtine blocks apoptosis in batten patient lymphoblasts and in human postmitotic CLN3- and CLN2-deficient neurons", ANNALS OF NEUROLOGY., vol. 51, no. 4, 1 April 2002 (2002-04-01), pages 448-466, XP055375147, BOSTON, US ISSN: 0364-5134, DOI: 10.1002/ana.10143
- MARIA KOUSI ET AL: "Update of the mutation spectrum and clinical correlations of over 360 mutations in eight genes that underlie the neuronal ceroid lipofuscinoses", HUMAN MUTATION, vol. 33, no. 1, 16 November 2011 (2011-11-16), pages 42-63, XP055347464, US ISSN: 1059-7794, DOI: 10.1002/humu.21624
- J. N. MILLER ET AL: "The role of nonsense-mediated decay in neuronal ceroid lipofuscinosis", HUMAN MOLECULAR GENETICS, vol. 22, no. 13, 28 March 2013 (2013-03-28), pages 2723-2734, XP55235382, gb ISSN: 0964-6906, DOI: 10.1093/hmg/ddt120
- MILLER ET AL.: 'The role of nonsense-mediated decay in neuronal ceroid lipofuscinosis' HUM MOL GENET vol. 22, no. 13, 28 March 2013, pages 2723 - 2734, XP055235382
- MARTIN ET AL.: 'The sequence and analysis of duplication-rich human chromosome 16' NATURE vol. 432, 23 December 2004, pages 988 - 994, XP055347460 DOI: 10.1038/NATURE03187
- KOUSI ET AL.: 'Update of the mutation spectrum and clinical correlations of over 360 mutations in eight genes that underlie the neuronal ceroid lipofuscinoses' HUM MUTAT. vol. 33, no. 1, 01 January 2012 - 16 November 2011, pages 42 - 63, XP055347464 DOI: 10.1002/HUMU.21624

### SEQUENCE LISTING

<110> Isis Pharmaceuticals, Inc.
   Rosalind Franklin University of Medicine and Science
<120> ANTISENSE COMPOUNDS AND USES THEREOF
<130> CORE0120WO
<150> 61/910,871
   <151> 2013-12-03
<160> 127
<170> PatentIn version 3.5
<210> 1
   <211> 17021
   <212> DNA
   <213> Homo sapiens
<400> 1
   agcactttgg gaagccaagg caggtggatt gcttgagctc aggagtttga ggccaccctg 60
   ggcaacgtgg caaaatccag tctctacaaa aaacacaaaa attagctggg cacagtggtg 120
   cgcacctgct gtcccagcta ctcgggaggc tgaggtggga ggatcacctg agcctgggag 180
   gtcaaggttg cagcaaacca agatcctgcc actgcactcc agcctgggca acagagcaaa 240
   accctgtcaa aaaaaaaaaa aaaatctggt ctaagcacca cctttgatgg aggcaggaat 300
   cctgcagcag cctggagcca acccaatggc tccctgcctc tggtgctcta tttcttatca 360
   ccatacaggt ctctaaggtc acctttaggt ctaacattgt atgaatgatt gtcagcaact 420
   ttccaatttt tttcatatac atatattttt tttattttat tttttacttt ttcttttttt 480
   gagacaggat ctcactctgt cacccaggct ggagtgcagt ggcgtgatca tagctcactg 540
   tagcttcaac ctcctgggca caagtgatcc tcccacctca gcttccaagg tacctgtgac 600
   tacaggaatg cgccaccatg cccggctaat tttgttatat atatatatat atatatttgt 660
   accttggttt tctcatctgt aacaccaggg gttgaagtgc tgtgatattt tttggttcta 720
   ggataatttg gaaataggga tctcttaaca tcttggatac tgccaataga tcattcacga 780
   aatcccttta gatgagtgtt atgtttactt aggtctctaa aacaatttgt aaggtcaaca 840
   gatggggata agaatgacct gaagctggtc gtctttgttc gcaaccagaa acagtcctgc 900
   ctcaaaaaaa aaaaaaaaaa aaagggagta ggcaggtggc catatttgtt tctggcaagt 960
   gagtgctgaa ggaaaggagc tgaagcctcc cacagtcata actggtgctg gcaggctact 1020
   gtctcggtct tgggcgccac tgatctaagg tcacggctct gcttgctgct cccacccgct 1080
   ccagtttaaa acctgcggtt ccagggttct ccagcccctc cctttttcac gctccgaagc 1140
   cgagaaggcc caaagcgaag acagagagga cccggaagta gggaaaacct ctgagcacgt 1200
   gatgggggaa cacgcgggtg ctgtcacgtg atccgacaaa cggcctctgc atagtgcaga 1260
   acattctgct gctcttaaag gtacaggcct cagggtccct gctgtagacg gggcggggga 1320
   gagtacgatg ggtggggcgt ggtgggtcgt agggcgctcg agatggagcc cccagcttcc 1380
   ttgatggatc gcggggcgcg agtgccctag acaagccgga gctgggaccg gcaatcgggc 1440
   gttgatcctt gtcacctgtc gcagaccctc atccctcccg tgggagcccc ctttggacac 1500
   tctatgaccc tggaccctcg ggggacctga acttgatgcg atgggaggct gtgcaggctc 1560
   gcggcggcgc ttttcggatt ccgagggtga gtattcccgc ccaccctcat ggaacgacca 1620
   cctctggctc gcagcccacc tgaggggaat gagagctgac tcggccccgg gaggacacgc 1680
   aaggagcggt cgtgcacctg agagtaggtg ggggtcatgc cctcttctcg ctctcccagg 1740
   ggaggagacc gtcccggagc cccggctccc tctgttggac catcagggcg cgcattggaa 1800
   gaacgcggtg ggcttctggt gagtggcctg agacttcagc gagtgacaat ggcatgagaa 1860
   gagggaagtg accggaggaa agggggaaga aagagttaag ctgcgcaaag gagctgaacc 1920
   cacaaatatt tactgaatcc actggtttgc cccaggggca gcatgtaaaa tttccaaact 1980
   tcactcctta tcaccttccg gatttagcaa gatcctcata cctaccacct gtaggattat 2040
   ttactttttt cttccagcca attcagcctt aacgtttttt agtgcacata tttttaaaaa 2100
   gaaaacttta aatcacgact ccacttggaa aaccagcgtt ttcctagtga acgtttaaat 2160
   aaatgcatca ctactaaaac ttttttattt ttttaattta ttttatttta tcttattttt 2220
   ttttcttttt gagacagggt cttgctctgt ttcccaggtt ggaatgcagt agtgcaatca 2280
   cagctccctg cagcctggaa ctcctgggct caagtgattc tcccacctca gcctccaagt 2340
   aggtgtggct acaggtgtgc accaccgcat ctgcctaatt tttatatttt tttatagaga 2400
   tgggggtctc actatgggac ctgggctggt ctcaaactcg tggcctcaag tgatcctcct 2460
   acctcaacct tccaaagtgc tgagattaca agcatgagcc accatgccgg gcctgaacct 2520
   tttttttttt tttttttttt gagacggagt ctctctctgt cctccaggct ggagtgcagt 2580
   ggcgcaatct cggctcactg caaactccgc ctcccaggtt cacaccattc ttttgcctca 2640
   gcctccggag tagctgagac tacaggcgcc tgccaccacg cccggctatt tttttgtatt 2700
   tttagtagag atggggtttc accgcgttat ccaggatggt ctcgatctcc tgacctcgtg 2760
   atccgcccgc ctcggcctcc caaagtgctg ggattacagg tgtgagccac cgcgcccagc 2820
   tttttttttt ttttttttta attttatttt atttttgaga cagagtctca ctctatcacc 2880
   caggctggag agcagtggca caatctcggc tcactgaaac ctccacctcc aaggttcaag 2940
   ctattctcct gtctcagctc cccgagtagc tgggattaca ggtgcgtgcc accagacaca 3000
   gctaattttt tgtattttta gtaaagacag gatttcacca tgttggtcag gctggtctag 3060
   aactcctgac cttaggtgat ccgcttgcct ccacttccca aagtgctggg attacaggcg 3120
   tgagccaccg tgccccgccc tgacataggg gctttgggat cacagacttg gattcacttc 3180
   cagcctccaa ggcctctccc agaaactctc tgtgaccact ctcctttttt tttttttttt 3240
   ttgagataga gtctcgctct tgtagcccag gctggagtgc aatggcacaa tctcggctta 3300
   ctgcaacctc cgcctttcgg gttcaagtgg ttctcctgcc tcagcctcct gagtagctag 3360
   gattacaggc atgtgcaact tcgcccagct aattttgtat tttttagtag agacggggtt 3420
   tctccatatt gctcaggctg gtctcgaact cctgacctca ggcgatccgc ccgcctcggc 3480
   ccctcaaagt gctggaatta caggtgtgag ccactgcact cggcctcttc ctttttattt 3540
   tttatttttg tgacagggtc ttgctctgtc acccaggcta gagtgcagtg gcatgatcac 3600
   agttcactgc agcctctgac tcctggactc aagcaatcct cccacctcag cctcccaagt 3660
   agctgggact acagtgcaag ccaccacacc tggctaattt ttaaattttt tgtagagatg 3720
   ggggtctcac tatcttgccc aagcaccaaa gcctgtattt ttgaccccaa cattgaatga 3780
   ggatgggatc tggtgataga gggaaaaaaa agagggggct gattgaaggg cacaggtaag 3840
   ggaaggtttg gcacaaaggc tacagatggc tgcaggatga ggagggagtt gagacctgtc 3900
   ctgctccttc caggctgctg ggcctttgca acaacttctc ttatgtggtg atgctgagtg 3960
   ccgcccacga catccttagc cacaagagga catcgggaaa ccagagccat gtaagtgact 4020
   ctctaccacc accaccatgg ttagtccctg tgggaagatg agggggtggg acaaggtggg 4080
   gtaaagtttc cagtctctgg ctgggcacag tggctcacac ctgtaatccc agcagtttgg 4140
   gaggctgagg cgggcggatc acttgaagtc aggagttcga gaccagcctg gccaacatgg 4200
   tgaaactccg actctactaa aaatacaaaa actacctggg tgtggtggca cacacctgta 4260
   gtctgagcta ttcaggaggc tgaggcagga gaattgcttg aagccaggag gtggaagttg 4320
   caatgagcca agatcacacc actgcactct agcttgggca acagagtgag acaccatctc 4380
   aaaaaaaaga aagctcttga atgtgttatc taaacaaagt ccatttacag ggccatctgt 4440
   ggcctgtggt ctagtagtgt gagactcata tggaaacccc ctcttcattg tgcatgcaga 4500
   gacgctgagg tcctgagaag tcagtcactg ctgacccaaa gccatccttc aagtgaaggc 4560
   agagctggga cgggagccag gctctgtgtg tctatccctc tgcctccagg gtgaaagaca 4620
   tgcttttctc ccattaggtg gacccaggcc caacgccgat cccccacaac agctcatcac 4680
   gatttgactg caactctgtc tctacggctg tgcgtactca tctcacctgg tccttgcctg 4740
   acccagggcc cctgggtcta tagaccccac tcccagccct tcactaccca gctgggactg 4800
   tcatgattaa aacagttgag acctgggctg ggcgcactgg ctcacacttg taatcccagc 4860
   actttgggag accaaggtgg gaggatcact tgagcctagg agtttgagac cagcctgggc 4920
   agtatagtga gacccccatc ttaaagaaaa aaattaaaaa atatatatat ataaaataat 4980
   tgagacctta atataatttc tgaggctgag gcggatggat cacttgagac caggagttca 5040
   agaccagcct ggccaacatg gtgaaacccc atctgtacta aaaatacaaa aattagccag 5100
   gcatggtggt gcacgcctgt aatcccagct actcaggagg ctgaggcagg agaatcactt 5160
   gaacccagga ggtggaggtt gtagtgagcc aagatcgagc cactacactc cagcctgggt 5220
   ggcagaatga gactcactct caaaatatat atatatatag tttctgagtc ctttctgtct 5280
   gcaccatata ttagctcatt taagccacac agcagtcctg tgagctaggt gctatgatat 5340
   tcccattttc cagatgagga aactgaagct cagagagtat aaactcttga cacacaacta 5400
   aggagtggga gagctgagac ttgaacccag gcgtgcctga ctccagagcc tgtgtttgta 5460
   gcaggcctgt ttggccagct cctgcctctc cttggccacg tggttgggag ggttgtcccc 5520
   tggaagctct gcggtctcac tctattctcc tgtcccaggc tgtgctcctg gcggacatcc 5580
   tccccacact cgtcatcaaa ttgttggctc ctcttggcct tcacctgctg ccctacaggt 5640
   ctgggtgagg gtagtgggag gcagggtggg caggagctga gaaaggggag gctgggatgg 5700
   ctgagatgct gagagtagag accgaccttc cccctccctt cccttctcac cccctcagcc 5760
   cccgggttct cgtcagtggg atttgtgctg ctggaagctt cgtcctggtt gccttttctc 5820
   attctgtggg gaccagcctg tgtggtgagt gtgtggttct gtgtcagatg gggagccccg 5880
   aggaaccaca tcagagcatt tgtgggaaga gtctccccag cctcccagag gaaagggatt 5940
   cattctgtca cccttagaag cctgctaggg ctatcagcag taggcgatgg gagactggga 6000
   caatttggag gggtaggcag tggaggagat gggagaaaat ggatgaatta gatggagatt 6060
   gaggtgaaca aagtcaagac tctgtgatgg accaggcaca gtgactcatg cctataatcc 6120
   cagcactttg gaaagccaag gcaggcagat cacctgaggt caggagttcg aaaccagcct 6180
   ggccaacatg gagaaacccc gtctctacca aaaatacaaa aattagctgg gtgtggtggc 6240
   aggagcctgt aatcccagct actcgggagg ctgaggcagg agaatctctt gaacctggga 6300
   ggcagaggtt gcagtgagcc gagatcacgc cactgtactc cagcctgggt gacagggcga 6360
   gactccgtct ccaaaaagaa aagaaaagaa aaagactgat gaaggggcag agacatcaag 6420
   ggtgcatgtc tgcccctggt ctgataactg ggtggatgga ggtgccactc tccatgaagg 6480
   gacacgcagg ggagtggggc tctgcttcag acctggaacc tggcctatgc atgggatcta 6540
   ttggagcctc tatgagctga tactgaggag gccatggcca gacacattag aggcctgggc 6600
   agtgtggcaa ggtgtggtgt gaccatccca gtgcttgtcc tccccccagg tgtggtcttc 6660
   gctagcatct catcaggcct tggggaggtc accttcctct ccctcactgc cttctacccc 6720
   aggtaagcag gtggagcagg gagtgtgggg agaggctgtc ccatggtcag cctaggtcct 6780
   cctgaatgtt cctgtgttct ccttcccagg gccgtgatct cctggtggtc ctcagggact 6840
   gggggagctg ggctgctggg ggccctgtcc tacctgggcc tcacccaggc cggcctctcc 6900
   cctcagcaga ccctgctgtc catgctgggt atccctgccc tgctgctggc caggtgagct 6960
   gccctgagcc gggagggaga ggggtccaag gagagaaaac ttggccatgg ctgggtgtgg 7020
   tggctcacgc ctgtaatccc agcactttgg gaggccaagg agggcagatc gcctaaggtc 7080
   aggaaaccag cctggccaac atggtgaaac cccgtctcta ctaaaaatac aacaattagc 7140
   caggtgtggt ggcgggtgcc tgtagtccca actactcagg aggctaaggc aggagaatcg 7200
   cttgaacccc ggaggcagtg gtggcagtga gccgagatcg tgccattgca ctccagcccg 7260
   ggcgacagag ttagactctg tctcaggaag aaaaaaaaaa aaagaaagaa aagaaaactt 7320
   gattatgatt gcaatcttca agtccctacc ttgctgtgaa gggaggcgga atctggactc 7380
   tgatagcccc aggtgtgagt cctggagctg ccacttttta gctttgtagc gttgaacaag 7440
   ttactccacc tctctgaacc ctcagtttcc ccatatctca aatggcagtt gttcttgctt 7500
   tccttggagg tgatgagggt aatgcattca gcacagtgtg gttcccaagg tgattagaag 7560
   taggatgagg gtggacttta tttgattagt tccttttttt tttttttttt tttttaatca 7620
   gtgttgacca ggttggcctc gaatgtgtag ccttgcctgc ctgagtgcca gggcaacagg 7680
   cctgaaccat ggcgactccc tttttttttt tttttttttt ttttttttga gacggagtct 7740
   catggtgtca ctcaggctgg agtgcagtga ctggtgtgat ctcagctcac tgcagcctcg 7800
   gcctcccggg ctctagtgat tctcctgcct cagcctcccg agtagctggg actacaggcc 7860
   catgccacca tgcctggcta attttttata tttttagtag agacggggtt tcactgtatt 7920
   ggccaggctg gtctcaaact cctgacctca agtgatccac ctgccttggc ctcccataat 7980
   gctaggaata caggcgtgag ccaccgcgcc cggcctgatt agttctgtgt attttcatgc 8040
   atattacaaa acactttggc cgggcatggt ggctcacatc tgtaatccca gcactttggg 8100
   aggccgaggc gggcgcacca cgaggtcagg agtttgagac cagcctggcc aatatggtga 8160
   aaccccgtct ctactaaaaa tacaaaaatt agccaggctt ggtggcgctt gcctgtagtc 8220
   ccagctactt gggaggctga ggagaagaat cgcttgaacc caggaggcag aggttgcagt 8280
   gagccgtgat tgtgccactg cactccaggc tgggcaacag agcgagactc cgtctaaaaa 8340
   aaaaaaaaac acacactttg aactagccag acacacctgg cccttcacaa gattagtgct 8400
   taggacaagt ctaagtagaa aaagtgagtc catttaaaga aaaatattaa gtaaaaatat 8460
   atatatacag gaggaatatg gatatggcaa ataacatgaa gccagaacct aagtgactaa 8520
   aatcgaggaa gttctggcct agcacagagt gaacgtataa ttaatgggag ctagagcgac 8580
   cattagaata ataatggcca gaaaacagaa ctgactcgct aggtatgagc cagcagtccg 8640
   aacaagggcc tgctgagcac agtggctcac agctgtaatc ccagtgcttt aggaggctga 8700
   ggctggagga ccactagagg ctagaaattt gagaccagcc tgggcaacat agcgagactc 8760
   catttataca aaaaatggaa aatatgaggc gggcttggtg gcacgtgcct gtagtccccg 8820
   ctacttggga ggctgaggcg ggaggattgc ttgagcctgg gaggtcgcgg ctgcagtgag 8880
   ctatgattgc accactgcat tccagcctgg atgctggaga aagaccctgt ctctgaaaaa 8940
   aatcaaaaca aaatgaaggc ccatgaatag gaataaggag ataaattcag gtccaaagaa 9000
   agaagccttt gtccacaatt ggagctctcc cgcacagcgt aggagcctta gaggcagtga 9060
   gctacccatc tttgaaagtg ttcaaaggta gaggtgttcc ctgggaaaag tggcctagat 9120
   ggtccctggg gaccttccca gccagtaggg ttttctgacc ctgccttcat cctactccta 9180
   gctatttctt gttgctcaca tctcctgagg cccaggaccc tggaggggaa gaagaagcag 9240
   agagcgcagc ccggcagccc ctcataagaa ccgaggcccc ggagtcgaag ccaggtagga 9300
   gacacagacc ctcagagagg tcactttctt tctctctggg tttggccttt tcctctctgc 9360
   aataggcaaa gttaagaggg gaaagagagt gagtgtactg gttatgggaa aagcctttgt 9420
   ctatttgaga aatacttgtt gaggacgagc acagtggctc acgcctgtaa tatcccagca 9480
   ctttgggagg ctgaggcagg aggaccactt gagctcagaa gtctgagtcc agcctgggca 9540
   acagagcaag accttgtcgc aaaaaaaaaa aaaaaaaaaa aaaaaagaaa agaaaggaag 9600
   gaagggaggg agagaagaag ggatattaat tgagtactta ccatgtgcca ggcattccaa 9660
   tcacaagtgc tgaggccctg cggtggggat aagctgggat gttctagaac cagagaatgg 9720
   ccagtgagac tgggcagggt gggccaatgg ccatctgtgg agctgtacaa gagacattgg 9780
   ccgggcgtgg tggttcatac ctgtaatccc agcactttgg gaggctgagg cagatggatc 9840
   atttgagatc aagagtttga gaccagtctg gccaacatgg taaaaccccg tctctactaa 9900
   aaataaaaaa attagccagg tgtggtggtg catgcctgta gtcccagcta cttgggaagc 9960
   tgaggcacga gaatcacttg aacccaggag gcagaggctg cagtgagctg agattgcacc 10020
   actgcactgc agcctgggca actgagtgag actctgtctc aaaaaataaa aaaaattaaa 10080
   aatcaaagag acgtgaagag gtctcacctg gttagctttt attttcatca tgataaagta 10140
   tgtatattac ttgaagttta ccattttatt attttttatc ttactttatt tttttgaggt 10200
   ggagtttcgc tcttgttgcc caggctggag tgcaatggcg caatctcagc tcactacaac 10260
   ctctgcctcc tgggttcaag tgattctcct gcctcagcca cccgagtagc tgggactaca 10320
   gacacctgcc accacacatg gctaattttt gtatttttag tagagatggg gtttctccat 10380
   gttggccagg gtggtcttga actcctgacc tcaggtgatc cgcccacctc agtctcccaa 10440
   ggtgctggga ttacaagcat gagccactgc gcccagccat tttaaccatt tttaagtgtc 10500
   caatccagtg gcatggaagt gagttcccac tgttgcgtgg tctggttaat tctgtcatac 10560
   cggtgcctct ttgccgagtc ttcagtgtga aaacttcatc tgcccttctc tctctgttcc 10620
   cctgcaggct ccagctccag cctctccctt cgggaaaggt ggacagtgtt caaggttcgg 10680
   atgatggctg gggtatgtcc ctgtggggct tgctcacctc caggcccccc gcttatatct 10740
   tctgcctttt ccagggtctg ctgtggtaca ttgttccctt ggtcgtagtt tactttgccg 10800
   agtatttcat taaccaggga cttgtaagtg aggggtgcta ggaggggtgt ggaggtggcg 10860
   attggggctg ggacccacac agccccgtcc atctcccctg tctggtattt gttgcagttt 10920
   gaactcctct ttttctggaa cacttccctg agtcacgctc agcaataccg ctggtaagag 10980
   gagcgagggc agtgggctgg gagggcgccg tggtgatgca gctgccctgc ccagtaggca 11040
   ccggggggag cgggatggtc cctggaggag cctcctctcc tccccccaac cctaacctca 11100
   ggtaccagat gctgtaccag gctggcgtct ttgcctcccg ctcttctctc cgctgctgtc 11160
   gcatccgttt cacctgggcc ctggccctgc tgcaggtacc aaacccctgc ccctcacttc 11220
   actcccacct tggctcccag cttggctccc agcttcccca aaccccctgc ttcccactat 11280
   cagtgggaag tgtaaatttt tttttttttt tttgagacag agtctcgctc tgtcgtccag 11340
   gctggagtgc agtagcgcaa tcttggctta ctgcaacctc tgcctcccgg gttcaagtga 11400
   ctctcctgcc tcagtctcct gagtagctgg gattacaggc gcccgccacc atacctggct 11460
   aatttttgta tttttagtag agatgggatt ttgccatatt ggctggtctt gaactcttga 11520
   cctcaggtga tctgccggcc tcagcctccc aaagtgctgg gattacaggg aagtgtaaat 11580
   tgtacactat catttcttag cacagggaac caaagtccag cagagctcca tgtaaaatgt 11640
   atagcctcag gagccaagca aaactgagct tcagtactca gccactgtgt gacttagggc 11700
   aggtctcaga acctctctga gcctcaattt cctcatgtat aaattgggtg tggccaatac 11760
   ctatttttca gggtagttgt aagaaataga gatgagagaa agaatcgaag aacaagattt 11820
   tgtagtgtgt gtgtgtgtgt gtgtgcgtgt gtgtgtgtgt gtgtgtgtgt gtgtgtgtgt 11880
   gttttaagca ttgggattgg gccgggccag gtggctcatg cctgtaatcc cagcactatg 11940
   ggaggctgag gcaggtggat catttgagct tggtagttca agtccagcct gggcaacatg 12000
   gcacaatcac atctctacaa aaattagcca ggtgcagtgg cgcacacctg tagtcttagc 12060
   tactgggaag ctgaggtggg aggatcactt gaacccatga ggtcaaggct gcagtaacca 12120
   aggtcatgcc actgcactcc agcctaggca atagagcgag accctgtctc ccagaaaaag 12180
   aaaaaagaaa ggaaaagtcg gaaccttcct ctgtcgccta ggctggagta cagtggcatg 12240
   atcataggtc atttacctca ggcaaagaga caggctttca aataatgttt ctaactataa 12300
   gaaaatgtac agttctttta ttctagatag ttaattataa tagaattggt gactttgaag 12360
   tcagtgggtt atttatcttg agggtggtga gcaaattcaa ctgcccttgg caaacagtag 12420
   tttacagctt taagggcgca aacacccttc cttacaacag tctaataaaa ggagtttcat 12480
   ataaccagca ttcattgttt caaggttaca aatccacagc ccctgcaggt aactgttaca 12540
   ataaactgtc aactgtgaca ggaaagtctt ctgcctttga aatttaggaa cttggccagt 12600
   cctggtggct cacacctgta atcccagcac tttgggaggc caaggcaggt gaatcacctg 12660
   aggtcaggat caggagtttg agaccagtct ggccaacaca gtgaaacccc gtctctatta 12720
   aaaatgcaaa aattagttgg gcgtggtggc acctgcctgt catctcagct actcgctagg 12780
   ctgaggcagg agaattgctt gaactccgga tatggaggtt gcagtgagcc gagattgcag 12840
   tactgtactc cagcctgggc cacagagcga gactccatct ccaaaaaaaa agacttagga 12900
   actcaattct gtgatagcaa aatacagaaa tagagattat actttaggct aggtgttgtg 12960
   gctcacacct gtaatcccaa tgcttttgga ggctagggtg ggaggatcac tcgaagccag 13020
   gagcttgaga ccagactgga caacatagtg agacccccca cctctacaaa aaattaaaaa 13080
   aaaaaataag ccaggtggga gaattgcttg agcccagaag tttgagagag cagcctgggt 13140
   aacatcacga gacctcatct ctacaaaaaa caacaacagg gtgtggtgac attcacctgt 13200
   aatcccagct actcgggagg ctgaggcagg aggatcgctt tagcccagga gttccaggct 13260
   gcagtgagcc ataatcatgc cactgtaccc cagcctgggt gagaccctat ctcaaaaaaa 13320
   aagattacac tttcatatta tggcaaaagc atgtaaatat cctccagcct ccttccttat 13380
   ttatctgagc agtgctgcaa gcccagggtt tgtacccaca aaaccccaaa tttataactc 13440
   agggtgcttg tagataccac tgaaacatgt agcttatggt tttaagctac atgtttttag 13500
   ctataatgtt ttttgttttg ttttgttttt tgagatggag tctcgctctg ttgcccaggc 13560
   tggaatgtag cggtgcaatc ttggctcact gcaacccctg cctcccagat tcaagcgatt 13620
   ctcaagcctc agccttccga gtagctggga ttacaggtgc gccaccacgc ccagcaaatt 13680
   ttttgttttg ttttgttttg ttttgttttt tagagacaga gttttgctct tgtagctcag 13740
   gctagagtac aatggcgtga tcttggctca ctgcaacctc cgcctcccag gttcaggcga 13800
   ttcttctgcc tcagcctcct gcgtagctgg gattacaggc acgtgccacc acacctggct 13860
   aatttttgta ttttttagta gagatggggt ttcgccatgt tggccaggct ggtcttgaac 13920
   tcctgacctc aggtgatcca cccgccttgg ccttccaaag tgctgggatt acaggtgtga 13980
   gccaccacac ctggctagag gtgtgtcctt tttaatgctc atctttaagt ctacctcaga 14040
   tttaatgaat taggacctct agggtaagcc ccgcttgggg catttgtacc tagatcccca 14100
   ggcaattctt acatatacga aaagtatgaa ccgccacgag gaggcctaca cactgcacaa 14160
   gaggcaatgt ggcgtggtca gtgctgggag gatggcacct ccttgagaga ctggggcaca 14220
   gaggtgggac ctacaccctg gggtggggat cgaagaggct tcctggccag gtgcggtggc 14280
   tcctgcctat aatcccagca ctttgggagg ctgaggcagg ggggcagcga ctgcttgaga 14340
   ccaggagttc gagcccagct gaataggatg cccggctact cccaccttgt tttaccatag 14400
   tgaaccaacg gtttttccaa taggggcaat tttgctgccc agcggatgtt tggcaacgtc 14460
   tgcaggcatg ctttggttgt cacaactgag ggatgctatg ggcatctagc gggcagaggc 14520
   cagggatcct gctaaatgcg ctgccatata caggacagcc ctcaccatgc agaacaacca 14580
   gcaccaaacg ccaagagtgc tgcaattgag aaatcctggt ttcgatggtc ccacttcctt 14640
   taagaagcct ccattttttt tttttttttt tttttttttt ttatgagaca ggatcttgct 14700
   ctgtcaccca ggctggagtg cagtggcaca atcatagctt actgcagcct ccacctccta 14760
   ggttcaagca atcctcctgt ctcagactcc cgagtagctg ggactgcagg tgcgtgccac 14820
   catgctcaac taatttttaa attttttgta gagacggagt tttgttatgt tgcccaggct 14880
   ggtctcgaac tcctggcctc aagtgatcat cccaccttgg tctcccaatg tgctggtatt 14940
   acctgcatga gccactgcac caggctgagg ttgagaattt tttttttttt tttttgggat 15000
   ggagtctctc tctgttgccc aggctggagt gcagtgctgc gatcttggct cactgcagcc 15060
   tctgcctccc aggttcaagt gattctcctg cctcagcctt ccaagtagct gggactacag 15120
   aaccactaca cccagataat ttttgtattt ttagttgaga tggggtttta ccatgtttag 15180
   tagagaccag gccaggctgg tctcgaaatc ctgacctcag gtgatctgcc cacctcagcc 15240
   tcccaaagtg ctgggattac aggtgtgagc caccacaccg ggctggtgtt gagattttat 15300
   ctagacctgg cagccctctc cttcaccagt aactcctaaa accagggacc cctggagggg 15360
   aggccacctc tccttccctg ccccgccctg gtcccaggct tagcctctcc ccctgttcac 15420
   agtgcctcaa cctggtgttc ctgctggcag acgtgtggtt cggctttctg ccaagcatct 15480
   acctcgtctt cctgatcatt ctgtatgagg ggctcctggg aggcgcagcc tacgtgaaca 15540
   ccttccacaa catcgccctg gaggtcagca ttggccgggc aagggctggg ggtggcctgt 15600
   ccagggacac ccagggcagg gatgtctggg actgaagcct cacccctgct ctctgccctc 15660
   ccagaccagt gatgagcacc gggagtttgc aatggcggcc acctgcatct ctgacacact 15720
   ggggatctcc ctgtcggggc tcctggcttt gcctctgcat gacttcctct gccagctctc 15780
   ctgatactcg ggatcctcag gacgcaggtc acattcacct gtgggcagag ggacaggtca 15840
   gacacccagg cccaccccag agaccctcca tgaactgtgc tcccagcctt cccggcaggt 15900
   ctgggagtag ggaagggctg aagccttgtt tcccttgcag gggggccagc cattgtctcc 15960
   cacttgggga gtttcttcct ggcatcatgc cttctgaata aatgccgatt ttatccatgg 16020
   acttcttata tcgtttttgt ctctaaaaag aaacttttat tatgaaagta atacatgccc 16080
   actttcctat atatagacag cataaagaag gtatgtcagc agatttctcc cttttttgtt 16140
   tgtttgtttg tttgtttgtt tgttttgaca gagtctcact ctgtcaccta ggctggagtg 16200
   caatggcgtg atcttggctc actgcaacct ccacttcccg gttcaagcaa ttctcctgcc 16260
   tcagcctccc gagtagctgg gattacaggt ggtcactacc atgtctggct aatttttata 16320
   tttttagtac agacgacatt ttgccatgtt ggccaggctg gtctcaaact catgacctca 16380
   ggtgatccgc ccaccttggc ctcccaaagt gctgagatta caggtgtgag ccactgtacc    16440
   tggcctctgc catctttgag gccttacgta ttcattcatt catgcattca ttgtttggga    16500
   caatctgtaa acaatcatgt tggacattct ttccagcatg aaagttttca gagaagtgga    16560
   aagaattgtg cagtgaatca tcctgagtat gcgccaccct gttctatgtt aacattttgt    16620
   tacagttgtt ttatcatgta gtccagcccc catcaaatct atgtattcac tgttccatca    16680
   gtgagtagat agagctaatg aatggcaggg ttggcacctg aggcatttta ttttattatt    16740
   tttttttatt tttagagaca gggttgcact ctgtcaccca ggctgaagta cagtggcaca    16800
   gtcatggctc actgtagcct tgacctccta gactcaaaca atcctcacac cttggcctcc    16860
   caagcagctg ggactgcaag tgcatgccac caagcccagc taattttttt tttttttttt    16920
   gtagagacgc gatcttccta tgttacccag gctggtcttg aactcttgag ctcaagcagt    16980
   cctgccttgg cctcccaaag tactgagatt acaagcatga g                        17021
<210> 2
   <211> 14881
   <212> DNA
   <213> Mus musculus
<400> 2
   tggacgtctg ctaagggacc ccgacaaggc ctagcttgaa taagtgggta cacttgtaga      60
   ggcaggagat tgaaacccag gaaggactgg gctaggagtc tggctgactg taggaaaggg     120
   agctcaggct tcaggagagg ctgtaaggga gaggaaaggt gagactacga ggaatggtgg     180
   aagtttcggg tatctctctg cccctctcac cagtgctccc ctcaaagcct gatgtagccc     240
   agggagacga agccggagaa aatccatcct gcaggtctct gtgtgtccag aaagctcaca     300
   atgaccatcc gtccccaccc acgaagtcct ccccagcacc caagacactt cctcttcccc     360
   tgactctccc tgacaaatct ccaccccagg ccctgtgcac ctcccccctg ctcatggatg     420
   acttcatgac aggcaggggg atggcttaac agtgtccagg tgactcagac tcgggtgcac     480
   atctggaact aaagggcaga ggatcgggcc agaaagggga aagggaaaaa aaaagtggaa     540
   acaggagacc tcagaattgg gtatggggga gggggcattc ggcaagtgca gcctgagtga     600
   ctcaggtctt gtattcgcac ctcagccatt tccgcctcct ccaagccaca gccttcagtg     660
   agaggaagct agggtttgtg gttcctgatt tatttggggg agaagctgta gaagtgagga     720
   taaggccact ggaagttctg ggttcccatt ttcaacgcca ttaagatttt tctggggatg     780
   aaaggtctgg attggtgctc aatgcccaca ccctcaccta tggttattct tgtactgtgg     840
   tcatgtgtcc tcacagctta ctgtgagttc cttaaggaca gaggctgcag aatttctaga     900
   gactcgtttg ggtaggagga tcctttttct cactgtttga gtgatctagg aagaacacca     960
   caggagggca tatttcaaac ggcaaatctt aatgacaggt ggttgaaaag acccgggaga    1020
   agtgggcaga ggctgctgga atccatagca gaggaatagg cttgatgaag aatggtcaag 1080
   atgctgccct gccatcgagt ggaaggtaag ggaaggactc cttatttgga actgtgaggt 1140
   gacacattcc aactatcctc tcagcctcat tctcctaact tccaattagg acaaacggct 1200
   gacatcacag accaggaaaa acacccatag cagctttctc agtgaactta gcacctttgc 1260
   tctatttctt actgctgagt ctctgaagtc agctaaaagt ctagcctctg ttgcacagtg 1320
   gggcggaggg accttggttt tctcttgtaa aaccaggggt tggacaactt tcgtggtatt 1380
   tgttgttcta ggatagtaag gcaaagggga tttaaccgta acatcgtgaa tattgccaca 1440
   gataattcat agaatgctgt tttgtgggtg tttattttta atcgatctac tctcagctac 1500
   ttcccagatg cttattatag gtctttacaa taggtctgta aggacaacag ataacacttg 1560
   aggctggtcg cctttgcgct caagcagaac ttgtccttgg gacgttgggt ggggtagtgg 1620
   gacatccgca gcaaagtagc cataattgtt tcaggcaagt aagagctgca ggaaagaagc 1680
   tgaagccttc cgcgccatga ctagtactgg cagatttggt cggcgtctag agcgccgctt 1740
   ttcatggctc tacttgtggc tcccttcgac tccaattcaa aatcagtgct tccagcgttc 1800
   tctcgaactc tccttctttt attctccgaa gtttgagaag tccaaaggcg aaaaagagaa 1860
   aaaaggaccc ggaagtaaga agcaaccctc aagcacgtga tggaaggctc acgggtactg 1920
   tcacgtgacc cccaacctgc tcgtctacct tgcggagagt tcagctgctc ttaaaggtac 1980
   aggcctgggg gcgggggctt tgctttagag gggcaggggg cggggtcagg cgggtggggc 2040
   gtgggtgaac acgagctgga gatctgggga tccttcgacg gacatgtcgt gtatagcaga 2100
   cagcggaacc tgggactgac cgcggggcat tgatccttcg cacccacctg tcccagactt 2160
   taatctgttt tcttgaagct agctcggaac acacgctgac tttgggccct ttgggggacc 2220
   cgaactcaat gttatgggaa gttctgcggg ctcgtggagg cgccttgagg attctgagag 2280
   tgagtgctct catcggttgc cttgggaatg accacctctg cccgcggctc tcccgaggag 2340
   ttgggatgct ggccctgccc cctcaacacc atgcgggata ggcggggcct tcgtgtaccc 2400
   gagtgagtgg ggggtcatgc tttctctacc tcccagggga ggagaccgac tcagagcccc 2460
   aggcccctcg gttggatagt cggagtgtcc tttggaagaa tgcagtgggt ttctggtaag 2520
   tggtctgaga gttgtgcaaa agtaaaatgc cagctgggcg tggtggcaca cgcctttaat 2580
   cccagcattt gggaggcaga ggcaggcgga tttctgagtt cgaggccagc ctggtctaca 2640
   gagtgagttc caggacagcc agggctacac agagaaaccc tgtctcgaaa aacaaacaaa 2700
   caaacaaaca aacaaagtaa agtgcgattg gagcaaagat gagagccaag ttgtgaaaag 2760
   cagttgaata cacagatact taatcaactg tgaactttcc aagttccatt aaattcccac 2820
   cttcacctat tgatcagatc ttagtatctg tagagttatt tattaaatgt tttcctccag 2880
   ctaactttgt tttttttcct cttccttcct ttctttcttt ctttctttct tcctcttcct 2940
   cctcctcctc ttcctcttct ttttcttctt ttttttggaa aggtctttaa accctacaag 3000
   cagtaagggc ttactttgac ctttctaatc ctgcttttgc cttccaggtg ctgcagttac 3060
   aggccctact gtagcacaat gcccagaaca cccattgttt ctgtttgcag tgcagggtat 3120
   taaaagcagg tccttcccgg gctcacgcac tgttgtacaa gtgagctctg tctccagcca 3180
   atttgctttt gccaagttct gagcacagct ctgggagatg aaaataagaa caatatgccc 3240
   tgctattatt ttggttccct tgagatcagc aggtgaaaat ttgtctccgt tttgcacagg 3300
   aatacgctag atatttgcat tgtaccacag tgtttccgtt gatgagaaat gcccagggat 3360
   cagaatctta gtgtaagcgt gggagaaata gtgcttagga ggacttggca cttggccatg 3420
   agctggtcta atctcaaggc ctgttttgat agtggagaag gaaaaaaaaa aggtgtatct 3480
   ttaaggactg taagtggata gaaagccaga cagtgaagat gtgtcctcat ttctaggatc 3540
   ttgggtcttt gcaacaattt ctcatatgtg gtgatgctga gcgctgccca tgacatcctc 3600
   aagcaggagc aggcgtctgg aaaccagagc catgtaagta agcccctctt caccaccacc 3660
   ataggtcagt aagtgagccc ctctgtacca ctaccatagg tcagtccgca tggggaatac 3720
   taagaaccca ctcagggcca gaggacccct gaaaaactcc tagagtagct ctgggtggga 3780
   ttggggagct aaaggtctta gtctcccaca tcctcatggc aggagggatc ttaaagagag 3840
   caacgggtac caggttggtg aactcacata actggctggg taagacctta ttttgtggtg 3900
   tgttttgtca ttgtaccctg ttgacatttg aggtcaggtg actgttgtga gtctgtcctg 3960
   catgttgtag atgtttagta gcatcccatg ccccttgtca accaaaagtg acagccaaaa 4020
   atgtctccag atgttacaaa tgctcctgcc aggtaacatt acccaggtgg agagcctttg 4080
   gcggcagtga gctagagcac gcaggcccta agaggcgcag cctccacttt gcaggttcac 4140
   taggaaattt gacccaagct gatattttcc agaaacccag atttctatat gaatgctctt 4200
   aaacatggtg catccatctt ggggggctgt ctttggcctc tggcccaaag tttgagatgc 4260
   ttataaacac tctcttcatt gtggacattg aaaaactaag gccccctaga agccttgcct 4320
   acccacagcc atttgtgaag ttaggacaga actggacctg ggccttggtt agcctcctgc 4380
   cgctttgtcg gaaagaccct cttctctccc ataggtggaa ccaggcccaa cacccacacc 4440
   ccacaacagc tcatctcgat ttgactgcaa ctccatctcc acagctgtga gtgtccatgt 4500
   ggggacccca cgatccttgc ctttgcaatc tcgtttcccc acccccaccc ccatccagtt 4560
   gggaatgttg ggattgcagt gatgaatata aatatgtaaa tagtaattat tagttcaggt 4620
   gccccccttc ccccagcttt gtgagtgaaa cactgtaagt ttccctcttt ggatgaggaa 4680
   actgaaggtc agagtgtata aatatgccct gacagccatt tgaggagccc cagagcaggg 4740
   aatgaatgga gatctatcta actccagagc ctaacttaca gcaggtctca ctggccggct 4800
   ccccggctgg gttgggaagg ttgtctccgg gaaggtctag gctctcactg tgttctcctg 4860
   tctcaggcgg tgctcctagc agacatcctt cccacccttg tcatcaaact cctggcgcct 4920
   cttggccttc acttgctgcc ttacaggtct gggtcggggt ggcaggaggg aggcagggtg 4980
   ggaggaagcg ggagtctgga gagtctcgca tggaggtggc cttctgagtt cctcctcatc 5040
   tcttctcagc ccccgggtgc tcgtcagtgg agtttgttct gctgggagct ttgttctggt 5100
   tgccttctct cagtcagtgg ggttaagcct gtgtggtgag tacgaagctg ggagaggttg 5160
   atggactcaa cagagtgatg tgctgtagtc tccccagctt cctgtgtgtg tgtgtgtgtg 5220
   tgtgtgtgtg tgtgtgtgtg tgtgtgtgtg agagagagag agagagagag agagagagac 5280
   acacacttgg gagattgtga atttgagatt agttggggcc acatagtgaa ttcaagccta 5340
   tcctaagctg cacagcaaaa ccctatctca aaaaacaaag acaaaaaaaa accaaaaaaa 5400
   caaggagagg gccagtccag gctgatttac atagcaagtt ccaggccagc ctggactaca 5460
   tccccgagac tttgtctcaa agacaaacct cctagagctt ccctaaccag aagtgagctc 5520
   taggaacaat tggagggtgt caataggaag gaatggggag actgagtata ggtggggatg 5580
   agcagggttg aataattggg aattattaac tgaatgaaga tgggggtaca gtctcggaga 5640
   ggacatgtgg aggagggtgt ggcttttcag atgagaccct agcctgtctg ttcagtgaca 5700
   gatctgttct tagccctgct gagctgatgg agttgaggct gtaaacaaac caccggggac 5760
   tgcggctttc agttaaaggt cactgtttgc tgtttgtcct cccaggagtg gttttggcca 5820
   gcatctcctc agggctaggg gaggtcacct tcctctcact gactgccttc taccccaggt 5880
   aagcggcctg ggccaggagg actgggagac gagtgccccc tgctggcttc catcttcctc 5940
   agtgctcttg tcttcttttc agtgctgtga tctcatggtg gtcttcgggt accgggggtg 6000
   cagggcttct tggatcgctg tcttacctgg gactcaccca ggctggcctc tccccgcagc 6060
   acaccctact ttctatgttg gggatccctg ttctgctgct agccaggtga gtgtccttag 6120
   gcccaaagga tgaaagatgg aaggaggagc ttgatgctaa tccaatcttc agagctcttc 6180
   agccttgttg tgaaggggtg tggactctgg actctcgaca cccctcaggt ggaatctcag 6240
   agtggggctg tgttagctgt gcgactctgg acaacttcat tcctgacctt cagttgtctc 6300
   tgtctcaggc tggttctgtc tcatccgcac aggtcggctc ctgagactgg ggctgggact 6360
   gagtgcaccc cggtttattt cttctgtgtg tatttgtata ttttagaaaa cattattaag 6420
   ccaggtgtag tggtacatgc ctttcatccc agcactctgg aggcatcatg ttatagctta 6480
   gggctattgt ggggtatcca ccagaaagac ggctcagaca tgggtttaaa cccagtggaa 6540
   agccgattag tcacctggta actacacact gcatgttcag tggcatttcc attggagtca 6600
   gttgtcctaa ggtccggtgg cctgttacaa ggctatcttg aagaaaagga gtcagtatgt 6660
   gtgtaatgct gaacacccac agtcctggct cttgggaggt gatggaaaag gctcatgtgt 6720
   ttgaagccta cctgggatac acgtggagac cctggctcaa aagctaactg agataattga 6780
   acatatggca aatgcctcta atccaagccc tcaggaggca ggaggatctc tgtgagtttg 6840
   gggtcagcct ggtctacatc ttgagctcca ggccagccag agctgcatag ttaagaccct 6900
   gtctcaaaaa aaaaaaaaaa gaaagaaaga aataaagcaa tcccatgatc atacagaccc 6960
   tgggtaaact cagtcacaac acgaaacaaa aagacactga gtatgagaaa gggacctgtg 7020
   atgtgggaaa ggtcacaggg tgtggggacc cgagggggtg gggagagtca ccagagggca 7080
   gtatatatgt gaataaaaat gtcaagaaac acatttagta agaatctctg tgaagtggtg 7140
   aatagatgag aagtactgag aacagggaaa cgagcaggtg ctgtgactga gtcaaatgag 7200
   gtccctgggt agaaacaaag aagcttttcc agcacagggt acctagaggc tgggagcacc 7260
   ccatgcaagt gtgaagggcc tggaggggcg ggcctgcagg acctctgggg cctttcccga 7320
   caagagcccc ctgactcgct tctccattcc atcccagcta tttcttgttg ctcacgtctc 7380
   ctgaacccct ggaccctgga ggggaaaacg aggcagagac tgctgcccgg cagcctctca 7440
   taggcaccga gaccccagag tcaaagccag gtaggatgca aaggccctcc catcccaggg 7500
   ccactttccg ccttggggtt ttggtctcaa cagtggacac agttgaggga aacaggctgc 7560
   taggtgtaca ggtctgggtc aaagcagtca tgtatactta ctaactactg actggatgcc 7620
   agacattccg attgctggga atggtagtgc tccagatgaa ggaatgagag cattttcaca 7680
   ggacataaaa ctgcgatggg gcccagaggg gtggcgcaca ccttgaatcc caacactccg 7740
   gaggcagcca caatcagatc tctatgattt caaggccggt tgggctacat agtaagaccc 7800
   tgcacagacc cagaaagtaa cggttaagga aatggggtgg ggatggctaa gggagctgct 7860
   ttacaggagg ttccaagtgt gggggttttg ttgttgtttt gttttttctt caagtttttg 7920
   cttattggtt tttaaatagt ctctctgtat agccctagct ggcctagaaa tcactgtgta 7980
   ctctcgaatg gcctcaacct cacaaatcca cctgcctctg cctctcaaat gctaggagta 8040
   aacgcatatg ccaccataca cctggctagg atttattttt ctatttattg atgtgtatgt 8100
   gtatttgtga acttataccg cggatgacca gcagagggca tcagatgcct tggagttgca 8160
   gttacaggca gttgggagct ggccgatgtt gccaggattc aaacgtggac cccctcatgg 8220
   ttgagcaacc agtgctcgct cgctctttct ttccttttct ctctttcttt ctttctttct 8280
   ttctttcttt ctttctttct ttctttcttt ctttctttct ttctttcttt ctttcttcct 8340
   tccttccttc cttcctttct ttctttcttt ctttcggatt tatttattta tttaatgtat 8400
   atgagcacaa tgtagttgtc ttcagacaca ccagaagagg gcaagagggc atcagatctc 8460
   ataacagatg gttgtgagcc accatgtggt tgctgggaat tgaactcagg acctctggaa 8520
   gagcagtcag tgctcttaac cactgagcca tctctccagc ccctctttct ttttttttta 8580
   ttgtttgttt gttttgtttt tgttttgttt tttgtttttt tgttgttgtt gttgaggcag 8640
   agcttctctg tgtagctctg gctgtcctgg aactcactct ctagaccaga agtcaaaaat 8700
   tctcctgcct ctgctttcca tgcgctggga ttcagggcgt gtgccgccac ttaaccccaa 8760
   gccacctctc caactctgag aaaggctcta cttatttatt taattttgta attatttgtt 8820
   tgtttgtttg cttgcttttc gaggaagggt ttctctgtgt agccctggct gtcctggaac 8880
   tcacactgta tgtaatccag gctgccctca aactttcaga gatccacctg cctcagcctc 8940
   ccaagtgggg attaaaggca tgcgccacca ctgcccagcg atttgtctat ttttatttta 9000
   tgtgcattgg tgttttgcct gtgtgcatgt ctatgggagg gtgttggatc ccctggaact 9060
   ggagttacag acagttgtga gctgccacgt gggtgctggg aattgaggtc cgctggaaga 9120
   gcagccatta ctcttaacca ctgagccatc tcttgagccc ccgagagaac ccccaacaac 9180
   aaacctttta tttttgagaa tggagctgtc tgggcaagag cgtgtgcaca agtgtgaggt 9240
   ctgacgttct gaggattgag ctctgctcat tcagactcgg cagtgagtgc cctcatccac 9300
   cgagcatcca tcagcccaag acagcctttt gaaggatatc tctgggctga aacttggctg 9360
   tcaaggtgtc agctttgcaa atgcctaggt gatcatgttc caggcacagg aaacagctca 9420
   gggtgaggcc ttatgtgaga aaaggagcct ggttgttcta gaaatgaaag gcttagccag 9480
   agtctgggat ggagtcggta gataaaagct gtaccctgat ctatagtctt gctcattaaa 9540
   aaaaaaaaaa aaaagtttta taagtatatg tgtgtatata catatgtata cagcgcgaag 9600
   tttaccattt tagcgactac taagtgtgca ctaagtggca tttaaatcca cacactgctg 9660
   tgctggctgg ttgcttgctg catgctggca ccttagctaa gcgttcagcg tgcaacctcc 9720
   tccccttccc cctgctctgt aggtgccagc tgggacctct ccctccagga aaggtggaca 9780
   gtgttcaagg tttgaatggt ggctgcgggt gccccgtgag gatcctctgc ctcttgcctt 9840
   ccacttacat ctcccgactc ttccagggtc tcttgtggta catcatccct ctggtgctgg 9900
   tctactttgc agaatacttt atcaaccagg gacttgtgag tgaggggcgc cggggaggga 9960
   tgtgggagga gagatagggc tgaggttccg ccatctccat ctgtctggtc ttcgctgcag 10020
   ttcgagctcc tgtttttccg gaacacttcc ctaagccatg ctcagcagta ccgatggtga 10080
   gagaagttgg caggtgggca gtaggctggg aggatccctg atgttggggt cctggggttg 10140
   accccaaaaa gtgccctgag agcctcttcc tcttctctgc cccaacctta ggtaccagat 10200
   gctataccag gctggtgtgt tcgcctcccg ctcttctctc caatgttgcc gaatacggtt 10260
   cacctgggtc ctagccctgc tccaggtact gagtgctgcc ctgtttgttc acacccacct 10320
   cggcctcctg agtttcttac attagtgtac tccagcgttc ctaagtacag cactaaaggc 10380
   cagctggact ccatataaaa tgtgtggtgg caccaggagg aaccagtcat acttgtgggt 10440
   aaattccagt ttctgttctc aagagctgtg tgactcagag cagtctctga acctctattg 10500
   agcctcagtt ttcctatcag gaagtcagat ggggtggagc aggattgtgc acctagcctg 10560
   tctgaggctg tgggtcccat ccctagtact gcaaaaatta aaatatttaa tagctgtggg 10620
   caacacctat ttctcagtgt ttgtatagca atgttgagaa aaaaaaggaa gaacaagata 10680
   ttgttttgat ttattttggt tttgttcttg tctgctttat ttttttttct ttgtttgttt 10740
   ttaaggtagg gtctcactgt gtagtcctgg gtggcctgga atccgatatg tagactaggc 10800
   tagcctgaag ctcacagatc tgcttcatga atgcgggcgt tgagggcctg tgcctcgtgc 10860
   ctggccgtcg tgctttgttc tcacagtcgt tctgactgca cctggggcct tgttcctgct 10920
   ggtccatgct gattgttacg aaacttctta agccctcaag cttgctttca tttacttact 10980
   tatttacttt aaagatttgt ttgtttatat ataaacaaac tgtagctgtg ttcagacaca 11040
   ccagaagagg gcatcagcta gccgggcgtg gtggcgcacg cctttaatcc cagcactcgg 11100
   gaggcagagg caggcggatt tctgagttcg aggccagcct ggtctgaaaa gtgagctcca 11160
   ggacagccag ggctacacag agaaaccctg tctcgaaccc ccccccaaaa aaaaaaaaca 11220
   aaaaaaaaca aaaaaaaaaa acagaagagg gcatcagatc ccatcataga tggttgtgag 11280
   ccaccatgtg gttgctggga atcgaactca ggacctctgg cagagcagtc agtgctctta 11340
   accgctgagc catcgctcta gccctattta tttattttat acaatttaaa taaacacact 11400
   tatatttata tttatttatt ggtgggaggg agacacttgc caagccgtgt gtatggaggt 11460
   cagaggacag cgtgcaggat tggttccctt cttcaacgtg tgggttctgg tgatcaaact 11520
   caggtcatac aggcaagtac ctgtagctga gccatctctt cagcccaaac agggtttctc 11580
   tgtatagccc tggctgtcct ggaactcact ctgtggacca ggctggcctc gaactcagaa 11640
   atctgcctgc ctctgcctcc caagtgctgg gtttaaaggc gtgcgccacc actgcccagc 11700
   tcagcccaaa ctttttattt ggaggcaaag tcttcaaagc tggccttgaa cctacatctg 11760
   aggcccacat aagccttgac cattgtttag tttatatata cacaaacctt tgtctatgtg 11820
   tatatatgtg aaccacctgt gtgcctggtg cccatggagg ccagagaggg tatcagatcc 11880
   ctggaactgg aactacagat aggtgtggac cgccctgtgg gtgctgtgga agaacagcgg 11940
   gtgcctttaa ctactgagcc atctttccag tccaaaattt tgtactcttg acatttgcag 12000
   ctagatcatt ctgatgtgtt acttgctgta ggtgtttagc agtatcccta gctggtgaga 12060
   cggcaagtag gtctccagac attgtcaaat gacccattga tttcagtgca gtaattaggg 12120
   gagaacaggt gatgttcagg gggtccttgg gtgctgagga taggactctg gtagagcatt 12180
   tgcctctgaa tcacaagctc tcgtgttagc tctgcagtgt atgtgtgtgt gtgtgtgtgt 12240
   gtgtgtgtgt gtgtgtgtgt gtgtgtttgt gtgtgtgata cacatggtag gggtgtctag 12300
   gtgttaggat tcatgccatt aaaaaagaaa caaaaaggca agaaggagag gaggtgactg 12360
   atgaccacag tggaatgacc ctggtccaac tgagagctca ccagtgatgg gaagttgagg 12420
   aacattacag gctaagagtg acagggacaa gtcaaggaag gggagaaacc cacaaggcat 12480
   gtgaggatga ggatggggtc atcgagggcc taccaagatg tcggggtccc acaggcccag 12540
   ctgggcactg ggaccctaca cagccctctg cttcagcggc atctcctata acctggcagc 12600
   tctgagacct gctgcctccc tgccgtattg ttgcccagtc tcacgcctcc cttccctctg 12660
   cccacagtgc ctcaacctgg ccctcctgct ggcagatgtc tgcttgaact tcttgcccag 12720
   catctacctc atcttcatca tcattctgta cgaagggctc ctgggtgggg ccgcttacgt 12780
   gaataccttc cacaacattg ctctggaggt ctgtgccagt tggacaaggg ctgtgggtgg 12840
   ccttaggagc cttgtggatg gaagccctaa cccttgttct ttgctctccc agaccagtga 12900
   caagcaccga gagtttgcca tggaagctgc ctgtatctct gacaccttgg gaatctccct 12960
   gtcgggggtc ctggccctgc ctctgcatga cttcctctgt cacctccctt gacaggagtt 13020
   gctcgacaca cactgatctg caggcacatg agcagatcac acatcttcga gctctgccac 13080
   agcctttccc tgccccactg cagcaaggag cccctgatgt ttcccactcc tgagctggcc 13140
   tcagagtttt ctcctaccct ctgcccttct aataaatgct tattttaaca gtttcctttt 13200
   tgtgcagtct ctgaaacgga aagtttacat aagcccactg tgaaagatat atgtatttat 13260
   gaatgtataa agtatttcag acaaggtgtg gcacacacct gtaattccag cactcagcag 13320
   actggagaat ccaagttcac gtctgactgg gatatacggt gagaccctga ggactagggc 13380
   tatagttcag tggtagagta cttgcccagg atgtcctggg ctctcagttt tatccctgga 13440
   aaacaattca aaagcagcgt atgctaatat aagcctgtag tcccaggatt ggggaggtag 13500
   agacaggagg atccagagtt catggtcctc agctacatag gaaaccaagg tcagcctggc 13560
   ctacataaga tcttgtttta caataattac attgtgtatt tctggcttct ttataaaaaa 13620
   aaatcaaatg tccataggta tgtagattta tatctgggtc ttcgattcca ttgatcaact 13680
   tgtctgtttt tatgccaata ctgtgtgggt tttattacta taactctgta gtagtgcttg 13740
   aaataaggat ggtgatacct ccaggagttc tattactgta cagagttgtt ttagcaatcc 13800
   tgtgtttctt gtttttttca tatgaaattg agtattgttc tttcaaggtc tataaagaat 13860
   tgtgttggaa ttttgatgga caactgattt ctttcttcaa tgtcttaaag tttttatcat 13920
   ataactcttt gacttgcttg gttagattga ccctacaata ttcatagaag acgaagtggg 13980
   tagtagccct gaacgaatgt cacaggagac aacttcctga acagaacacc aatagttcag 14040
   gcactaaaat tgacaataaa ccagatctca tgaaactgaa aagcttttgt gaggcaaatg 14100
   atattgtcat ctggcagcct acagaataga aaaagatttc taccaactca atatctgata 14160
   gaaggctaat atccgtaata tataaagaac tcaaagccgg gcggtggtgg cgcacgcctt 14220
   taatcctagc actcaggagg cagaggcagg tggatttctg agttcaaggc cagcctggtc 14280
   tacaaagtga gttccaggac agccagagct acacagagaa accctgtctc gaaaaaaaaa 14340
   caaagaacaa aaaacacgag ggagagagaa agagaaagag aactcaagaa gctagacatc 14400
   aataaggcaa atagcccaaa taaaaaatgg ggtacagagc tgaacagaga cttctcaaca 14460
   ggaatctcaa atggctgaga aacacttaga acttcatcat ccttatccag cagggaaatg 14520
   caaatcaaaa ccactctgag attccatctt taacacctgt caggatggct aagatcaaaa 14580
   ctcaagtaac agctcatgct ggcgaggatg tggagaaagg ggagcacttg ggagcaaggg 14640
   gagcactctt cggttgttgg tgagaatgca aatttgtaca gccactttgg agatcaatat 14700
   ggtagtttct tagaaaattg gaacctccag acccagctat accacttctg gggatatacc 14760
   caaaggatgt cccatcctac cacagggaca cttgctcaat tatgttcaca gcagctttat 14820
   ttataataac cagaaactgg aaacaacctc gatgtccctt aactaaggaa tggataagaa 14880
   a 14881
<210> 3
   <211> 25
   <212> DNA
   <213> Artificial sequence
<220>
   <223> Synthetic oligonucleotide
<400> 3
   acacagaacc acacactcac cacac 25
<210> 4
   <211> 18
   <212> DNA
   <213> Artificial sequence
<220>
   <223> Synthetic oligonucleotide
<400> 4
   gagaagagat gaggagga 18
<210> 5
   <211> 18
   <212> DNA
   <213> Artificial sequence
<220>
   <223> Synthetic oligonucleotide
<400> 5
   ggctgagaag agatgagg 18
<210> 6
   <211> 18
   <212> DNA
   <213> Artificial sequence
<220>
   <223> Synthetic oligonucleotide
<400> 6
   gggctgagaa gagatgag 18
<210> 7
   <211> 18
   <212> DNA
   <213> Artificial sequence
<220>
   <223> Synthetic oligonucleotide
<400> 7
   cactgacgag cacccggg 18
<210> 8
   <211> 18
   <212> DNA
   <213> Artificial sequence
<220>
   <223> Synthetic oligonucleotide
<400> 8
   ccactgacga gcacccgg 18
<210> 9
   <211> 18
   <212> DNA
   <213> Artificial sequence
<220>
   <223> Synthetic oligonucleotide
<400> 9
   tccactgacg agcacccg 18
<210> 10
   <211> 18
   <212> DNA
   <213> Artificial sequence
<220>
   <223> Synthetic oligonucleotide
<400> 10
   aaactccact gacgagca 18
<210> 11
   <211> 18
   <212> DNA
   <213> Artificial sequence
<220>
   <223> Synthetic oligonucleotide
<400> 11
   gaacaaactc cactgacg 18
<210> 12
   <211> 18
   <212> DNA
   <213> Artificial sequence
<220>
   <223> Synthetic oligonucleotide
<400> 12
   agcagaacaa actccact 18
<210> 13
   <211> 18
   <212> DNA
   <213> Artificial sequence
<220>
   <223> Synthetic oligonucleotide
<400> 13
   tcccagcaga acaaactc 18
<210> 14
   <211> 18
   <212> DNA
   <213> Artificial sequence
<220>
   <223> Synthetic oligonucleotide
<400> 14
   aagctcccag cagaacaa 18
<210> 15
   <211> 18
   <212> DNA
   <213> Artificial sequence
<220>
   <223> Synthetic oligonucleotide
<400> 15
   aacaaagctc ccagcaga 18
<210> 16
   <211> 18
   <212> DNA
   <213> Artificial sequence
<220>
   <223> Synthetic oligonucleotide
<400> 16
   ccagaacaaa gctcccag 18
<210> 17
   <211> 18
   <212> DNA
   <213> Artificial sequence
<220>
   <223> Synthetic oligonucleotide
<400> 17
   gcaaccagaa caaagctc 18
<210> 18
   <211> 18
   <212> DNA
   <213> Artificial sequence
<220>
   <223> Synthetic oligonucleotide
<400> 18
   gaaggcaacc agaacaaa 18
<210> 19
   <211> 18
   <212> DNA
   <213> Artificial sequence
<220>
   <223> Synthetic oligonucleotide
<400> 19
   gagagaaggc aaccagaa 18
<210> 20
   <211> 18
   <212> DNA
   <213> Artificial sequence
<220>
   <223> Synthetic oligonucleotide
<400> 20
   gactgagaga aggcaacc 18
<210> 21
   <211> 18
   <212> DNA
   <213> Artificial sequence
<220>
   <223> Synthetic oligonucleotide
<400> 21
   cactgactga gagaaggc 18
<210> 22
   <211> 18
   <212> DNA
   <213> Artificial sequence
<220>
   <223> Synthetic oligonucleotide
<400> 22
   accccactga ctgagaga 18
<210> 23
   <211> 18
   <212> DNA
   <213> Artificial sequence
<220>
   <223> Synthetic oligonucleotide
<400> 23
   cttaacccca ctgactga 18
<210> 24
   <211> 18
   <212> DNA
   <213> Artificial sequence
<220>
   <223> Synthetic oligonucleotide
<400> 24
   caggcttaac cccactga 18
<210> 25
   <211> 18
   <212> DNA
   <213> Artificial sequence
<220>
   <223> Synthetic oligonucleotide
<400> 25
   cacacaggct taacccca 18
<210> 26
   <211> 18
   <212> DNA
   <213> Artificial sequence
<220>
   <223> Synthetic oligonucleotide
<400> 26
   tcaccacaca ggcttaac 18
<210> 27
   <211> 18
   <212> DNA
   <213> Artificial sequence
<220>
   <223> Synthetic oligonucleotide
<400> 27
   gtactcacca cacaggct 18
<210> 28
   <211> 18
   <212> DNA
   <213> Artificial sequence
<220>
   <223> Synthetic oligonucleotide
<400> 28
   cttcgtactc accacaca 18
<210> 29
   <211> 18
   <212> DNA
   <213> Artificial sequence
<220>
   <223> Synthetic oligonucleotide
<400> 29
   ccagcttcgt actcacca 18
<210> 30
   <211> 18
   <212> DNA
   <213> Artificial sequence
<220>
   <223> Synthetic oligonucleotide
<400> 30
   tctcccagct tcgtactc 18
<210> 31
   <211> 18
   <212> DNA
   <213> Artificial sequence
<220>
   <223> Synthetic oligonucleotide
<400> 31
   gggatggaat ggagaagc 18
<210> 32
   <211> 18
   <212> DNA
   <213> Artificial sequence
<220>
   <223> Synthetic oligonucleotide
<400> 32
   agctgggatg gaatggag 18
<210> 33
   <211> 18
   <212> DNA
   <213> Artificial sequence
<220>
   <223> Synthetic oligonucleotide
<400> 33
   aaatagctgg gatggaat 18
<210> 34
   <211> 18
   <212> DNA
   <213> Artificial sequence
<220>
   <223> Synthetic oligonucleotide
<400> 34
   caagaaatag ctgggatg 18
<210> 35
   <211> 18
   <212> DNA
   <213> Artificial sequence
<220>
   <223> Synthetic oligonucleotide
<400> 35
   gcaacaagaa atagctgg 18
<210> 36
   <211> 18
   <212> DNA
   <213> Artificial sequence
<220>
   <223> Synthetic oligonucleotide
<400> 36
   gtgagcaaca agaaatag 18
<210> 37
   <211> 18
   <212> DNA
   <213> Artificial sequence
<220>
   <223> Synthetic oligonucleotide
<400> 37
   agacgtgagc aacaagaa 18
<210> 38
   <211> 18
   <212> DNA
   <213> Artificial sequence
<220>
   <223> Synthetic oligonucleotide
<400> 38
   caggagacgt gagcaaca 18
<210> 39
   <211> 18
   <212> DNA
   <213> Artificial sequence
<220>
   <223> Synthetic oligonucleotide
<400> 39
   ggttcaggag acgtgagc 18
<210> 40
   <211> 18
   <212> DNA
   <213> Artificial sequence
<220>
   <223> Synthetic oligonucleotide
<400> 40
   gggttcagga gacgtgag 18
<210> 41
   <211> 18
   <212> DNA
   <213> Artificial sequence
<220>
   <223> Synthetic oligonucleotide
<400> 41
   cccctccagg gtccaggg 18
<210> 42
   <211> 18
   <212> DNA
   <213> Artificial sequence
<220>
   <223> Synthetic oligonucleotide
<400> 42
   tttcccctcc agggtcca 18
<210> 43
   <211> 18
   <212> DNA
   <213> Artificial sequence
<220>
   <223> Synthetic oligonucleotide
<400> 43
   tcgttttccc ctccaggg 18
<210> 44
   <211> 18
   <212> DNA
   <213> Artificial sequence
<220>
   <223> Synthetic oligonucleotide
<400> 44
   tgcctcgttt tcccctcc 18
<210> 45
   <211> 18
   <212> DNA
   <213> Artificial sequence
<220>
   <223> Synthetic oligonucleotide
<400> 45
   tctctgcctc gttttccc 18
<210> 46
   <211> 18
   <212> DNA
   <213> Artificial sequence
<220>
   <223> Synthetic oligonucleotide
<400> 46
   gcagtctctg cctcgttt 18
<210> 47
   <211> 18
   <212> DNA
   <213> Artificial sequence
<220>
   <223> Synthetic oligonucleotide
<400> 47
   ggcagcagtc tctgcctc 18
<210> 48
   <211> 18
   <212> DNA
   <213> Artificial sequence
<220>
   <223> Synthetic oligonucleotide
<400> 48
   gccgggcagc agtctctg 18
<210> 49
   <211> 18
   <212> DNA
   <213> Artificial sequence
<220>
   <223> Synthetic oligonucleotide
<400> 49
   ggctgccggg cagcagtc 18
<210> 50
   <211> 18
   <212> DNA
   <213> Artificial sequence
<220>
   <223> Synthetic oligonucleotide
<400> 50
   gagaggctgc cgggcagc 18
<210> 51
   <211> 18
   <212> DNA
   <213> Artificial sequence
<220>
   <223> Synthetic oligonucleotide
<400> 51
   ctatgagagg ctgccggg 18
<210> 52
   <211> 18
   <212> DNA
   <213> Artificial sequence
<220>
   <223> Synthetic oligonucleotide
<400> 52
   gtgcctatga gaggctgc 18
<210> 53
   <211> 18
   <212> DNA
   <213> Artificial sequence
<220>
   <223> Synthetic oligonucleotide
<400> 53
   ctcggtgcct atgagagg 18
<210> 54
   <211> 18
   <212> DNA
   <213> Artificial sequence
<220>
   <223> Synthetic oligonucleotide
<400> 54
   gggtctcggt gcctatga 18
<210> 55
   <211> 18
   <212> DNA
   <213> Artificial sequence
<220>
   <223> Synthetic oligonucleotide
<400> 55
   cctggctttg actctggg 18
<210> 56
   <211> 18
   <212> DNA
   <213> Artificial sequence
<220>
   <223> Synthetic oligonucleotide
<400> 56
   cctacctggc tttgactc 18
<210> 57
   <211> 18
   <212> DNA
   <213> Artificial sequence
<220>
   <223> Synthetic oligonucleotide
<400> 57
   gcatcctacc tggctttg 18
<210> 58
   <211> 18
   <212> DNA
   <213> Artificial sequence
<220>
   <223> Synthetic oligonucleotide
<400> 58
   ctttgcatcc tacctggc 18
<210> 59
   <211> 18
   <212> DNA
   <213> Artificial sequence
<220>
   <223> Synthetic oligonucleotide
<400> 59
   gggcctttgc atcctacc 18
<210> 60
   <211> 18
   <212> DNA
   <213> Artificial sequence
<220>
   <223> Synthetic oligonucleotide
<400> 60
   gggagggcct ttgcatcc 18
<210> 61
   <211> 18
   <212> DNA
   <213> Artificial sequence
<220>
   <223> Synthetic oligonucleotide
<400> 61
   agctgatcat attctacc 18
<210> 62
   <211> 25
   <212> DNA
   <213> Artificial sequence
<220>
   <223> Synthetic oligonucleotide
<400> 62
   agctgatcat attctacctg gtgct 25
<210> 63
   <211> 18
   <212> DNA
   <213> Artificial sequence
<220>
   <223> Synthetic oligonucleotide
<400> 63
   aaaggcaacc aggacgaa 18
<210> 64
   <211> 18
   <212> DNA
   <213> Artificial sequence
<220>
   <223> Synthetic oligonucleotide
<400> 64
   aaatgctctg atgtggtt 18
<210> 65
   <211> 18
   <212> DNA
   <213> Artificial sequence
<220>
   <223> Synthetic oligonucleotide
<400> 65
   acaaatccca ctgacgag 18
<210> 66
   <211> 18
   <212> DNA
   <213> Artificial sequence
<220>
   <223> Synthetic oligonucleotide
<400> 66
   acaggctggt ccccacag 18
<210> 67
   <211> 18
   <212> DNA
   <213> Artificial sequence
<220>
   <223> Synthetic oligonucleotide
<400> 67
   accacacagg ctggtccc 18
<210> 68
   <211> 18
   <212> DNA
   <213> Artificial sequence
<220>
   <223> Synthetic oligonucleotide
<400> 68
   actctcagca tctcagcc 18
<210> 69
   <211> 18
   <212> DNA
   <213> Artificial sequence
<220>
   <223> Synthetic oligonucleotide
<400> 69
   aggacgaagc ttccagca 18
<210> 70
   <211> 18
   <212> DNA
   <213> Artificial sequence
<220>
   <223> Synthetic oligonucleotide
<400> 70
   caaccaggac gaagcttc 18
<210> 71
   <211> 18
   <212> DNA
   <213> Artificial sequence
<220>
   <223> Synthetic oligonucleotide
<400> 71
   cactcaccac acaggctg 18
<210> 72
   <211> 18
   <212> DNA
   <213> Artificial sequence
<220>
   <223> Synthetic oligonucleotide
<400> 72
   cagaaccaca cactcacc 18
<210> 73
   <211> 18
   <212> DNA
   <213> Artificial sequence
<220>
   <223> Synthetic oligonucleotide
<400> 73
   cagaatgaga aaaggcaa 18
<210> 74
   <211> 18
   <212> DNA
   <213> Artificial sequence
<220>
   <223> Synthetic oligonucleotide
<400> 74
   ccacacactc accacaca 18
<210> 75
   <211> 18
   <212> DNA
   <213> Artificial sequence
<220>
   <223> Synthetic oligonucleotide
<400> 75
   ccatctgaca cagaacca 18
<210> 76
   <211> 18
   <212> DNA
   <213> Artificial sequence
<220>
   <223> Synthetic oligonucleotide
<400> 76
   cccacaaatg ctctgatg 18
<210> 77
   <211> 18
   <212> DNA
   <213> Artificial sequence
<220>
   <223> Synthetic oligonucleotide
<400> 77
   ccccacagaa tgagaaaa 18
<210> 78
   <211> 18
   <212> DNA
   <213> Artificial sequence
<220>
   <223> Synthetic oligonucleotide
<400> 78
   ctctgatgtg gttcctcg 18
<210> 79
   <211> 18
   <212> DNA
   <213> Artificial sequence
<220>
   <223> Synthetic oligonucleotide
<400> 79
   ctggtcccca cagaatga 18
<210> 80
   <211> 18
   <212> DNA
   <213> Artificial sequence
<220>
   <223> Synthetic oligonucleotide
<400> 80
   gaagcttcca gcagcaca 18
<210> 81
   <211> 18
   <212> DNA
   <213> Artificial sequence
<220>
   <223> Synthetic oligonucleotide
<400> 81
   gcagcacaaa tcccactg 18
<210> 82
   <211> 18
   <212> DNA
   <213> Artificial sequence
<220>
   <223> Synthetic oligonucleotide
<400> 82
   gctccccatc tgacacag 18
<210> 83
   <211> 18
   <212> DNA
   <213> Artificial sequence
<220>
   <223> Synthetic oligonucleotide
<400> 83
   ggaaggtcgg tctctact 18
<210> 84
   <211> 18
   <212> DNA
   <213> Artificial sequence
<220>
   <223> Synthetic oligonucleotide
<400> 84
   ggtgagaagg gaagggag 18
<210> 85
   <211> 18
   <212> DNA
   <213> Artificial sequence
<220>
   <223> Synthetic oligonucleotide
<400> 85
   gtcggtctct actctcag 18
<210> 86
   <211> 18
   <212> DNA
   <213> Artificial sequence
<220>
   <223> Synthetic oligonucleotide
<400> 86
   tcccactgac gagaaccc 18
<210> 87
   <211> 18
   <212> DNA
   <213> Artificial sequence
<220>
   <223> Synthetic oligonucleotide
<400> 87
   tctctactct cagcatct 18
<210> 88
   <211> 18
   <212> DNA
   <213> Artificial sequence
<220>
   <223> Synthetic oligonucleotide
<400> 88
   tgacacagaa ccacacac 18
<210> 89
   <211> 18
   <212> DNA
   <213> Artificial sequence
<220>
   <223> Synthetic oligonucleotide
<400> 89
   tgagaaaagg caaccagg 18
<210> 90
   <211> 18
   <212> DNA
   <213> Artificial sequence
<220>
   <223> Synthetic oligonucleotide
<400> 90
   ttccagcagc acaaatcc 18
<210> 91
   <211> 18
   <212> DNA
   <213> Artificial sequence
<220>
   <223> Synthetic oligonucleotide
<400> 91
   gggaaggtcc ccagggac 18
<210> 92
   <211> 18
   <212> DNA
   <213> Artificial sequence
<220>
   <223> Synthetic oligonucleotide
<400> 92
   tggctgggaa ggtcccca 18
<210> 93
   <211> 18
   <212> DNA
   <213> Artificial sequence
<220>
   <223> Synthetic oligonucleotide
<400> 93
   cctactggct gggaaggt 18
<210> 94
   <211> 18
   <212> DNA
   <213> Artificial sequence
<220>
   <223> Synthetic oligonucleotide
<400> 94
   aaaaccctac tggctggg 18
<210> 95
   <211> 18
   <212> DNA
   <213> Artificial sequence
<220>
   <223> Synthetic oligonucleotide
<400> 95
   gtcagaaaac cctactgg 18
<210> 96
   <211> 18
   <212> DNA
   <213> Artificial sequence
<220>
   <223> Synthetic oligonucleotide
<400> 96
   gcagggtcag aaaaccct 18
<210> 97
   <211> 18
   <212> DNA
   <213> Artificial sequence
<220>
   <223> Synthetic oligonucleotide
<400> 97
   tgaaggcagg gtcagaaa 18
<210> 98
   <211> 18
   <212> DNA
   <213> Artificial sequence
<220>
   <223> Synthetic oligonucleotide
<400> 98
   taggatgaag gcagggtc 18
<210> 99
   <211> 18
   <212> DNA
   <213> Artificial sequence
<220>
   <223> Synthetic oligonucleotide
<400> 99
   aggagtagga tgaaggca 18
<210> 100
   <211> 18
   <212> DNA
   <213> Artificial sequence
<220>
   <223> Synthetic oligonucleotide
<400> 100
   tagctaggag taggatga 18
<210> 101
   <211> 18
   <212> DNA
   <213> Artificial sequence
<220>
   <223> Synthetic oligonucleotide
<400> 101
   agaaatagct aggagtag 18
<210> 102
   <211> 18
   <212> DNA
   <213> Artificial sequence
<220>
   <223> Synthetic oligonucleotide
<400> 102
   caacaagaaa tagctagg 18
<210> 103
   <211> 18
   <212> DNA
   <213> Artificial sequence
<220>
   <223> Synthetic oligonucleotide
<400> 103
   gagatgtgag caacaaga 18
<210> 104
   <211> 18
   <212> DNA
   <213> Artificial sequence
<220>
   <223> Synthetic oligonucleotide
<400> 104
   ctcaggagat gtgagcaa 18
<210> 105
   <211> 18
   <212> DNA
   <213> Artificial sequence
<220>
   <223> Synthetic oligonucleotide
<400> 105
   tgggcctcag gagatgtg 18
<210> 106
   <211> 18
   <212> DNA
   <213> Artificial sequence
<220>
   <223> Synthetic oligonucleotide
<400> 106
   ggtcctgggc ctcaggag 18
<210> 107
   <211> 18
   <212> DNA
   <213> Artificial sequence
<220>
   <223> Synthetic oligonucleotide
<400> 107
   tccagggtcc tgggcctc 18
<210> 108
   <211> 18
   <212> DNA
   <213> Artificial sequence
<220>
   <223> Synthetic oligonucleotide
<400> 108
   tcccctccag ggtcctgg 18
<210> 109
   <211> 18
   <212> DNA
   <213> Artificial sequence
<220>
   <223> Synthetic oligonucleotide
<400> 109
   cttcttcccc tccagggt 18
<210> 110
   <211> 18
   <212> DNA
   <213> Artificial sequence
<220>
   <223> Synthetic oligonucleotide
<400> 110
   tgcttcttct tcccctcc 18
<210> 111
   <211> 18
   <212> DNA
   <213> Artificial sequence
<220>
   <223> Synthetic oligonucleotide
<400> 111
   ctctctgctt cttcttcc 18
<210> 112
   <211> 18
   <212> DNA
   <213> Artificial sequence
<220>
   <223> Synthetic oligonucleotide
<400> 112
   ctgcgctctc tgcttctt 18
<210> 113
   <211> 18
   <212> DNA
   <213> Artificial sequence
<220>
   <223> Synthetic oligonucleotide
<400> 113
   ccgggctgcg ctctctgc 18
<210> 114
   <211> 18
   <212> DNA
   <213> Artificial sequence
<220>
   <223> Synthetic oligonucleotide
<400> 114
   ggctgccggg ctgcgctc 18
<210> 115
   <211> 18
   <212> DNA
   <213> Artificial sequence
<220>
   <223> Synthetic oligonucleotide
<400> 115
   gggcctcggt tcttatga 18
<210> 116
   <211> 18
   <212> DNA
   <213> Artificial sequence
<220>
   <223> Synthetic oligonucleotide
<400> 116
   cctacctggc ttcgactc 18
<210> 117
   <211> 18
   <212> DNA
   <213> Artificial sequence
<220>
   <223> Synthetic oligonucleotide
<400> 117
   tgtctcctac ctggcttc 18
<210> 118
   <211> 18
   <212> DNA
   <213> Artificial sequence
<220>
   <223> Synthetic oligonucleotide
<400> 118
   gtctgtgtct cctacctg 18
<210> 119
   <211> 18
   <212> DNA
   <213> Artificial sequence
<220>
   <223> Synthetic oligonucleotide
<400> 119
   tgagggtctg tgtctcct 18
<210> 120
   <211> 18
   <212> DNA
   <213> Artificial sequence
<220>
   <223> Synthetic oligonucleotide
<400> 120
   ctctctgagg gtctgtgt 18
<210> 121
   <211> 18
   <212> DNA
   <213> Artificial sequence
<220>
   <223> Synthetic oligonucleotide
<400> 121
   gtgacctctc tgagggtc 18
<210> 122
   <211> 18
   <212> DNA
   <213> Artificial sequence
<220>
   <223> Synthetic oligonucleotide
<400> 122
   agaaagtgac ctctctga 18
<210> 123
   <211> 18
   <212> DNA
   <213> Artificial sequence
<220>
   <223> Synthetic oligonucleotide
<400> 123
   gagaaagaaa gtgacctc 18
<210> 124
   <211> 18
   <212> DNA
   <213> Artificial sequence
<220>
   <223> Synthetic oligonucleotide
<400> 124
   ccagagagaa agaaagtg 18
<210> 125
   <211> 18
   <212> DNA
   <213> Artificial sequence
<220>
   <223> Synthetic oligonucleotide
<400> 125
   caaacccaga gagaaaga 18
<210> 126
   <211> 18
   <212> DNA
   <213> Artificial sequence
<220>
   <223> Synthetic oligonucleotide
<400> 126
   aaggccaaac ccagagag 18
<210> 127
   <211> 18
   <212> DNA
   <213> Artificial sequence
<220>
   <223> Synthetic oligonucleotide
<400> 127
   aggaaaaggc caaaccca 18

## Description

### BACKGROUND

Juvenile neuronal ceroid lipofuscinosis (JNCL), also known as Batten Disease, is the most common of the NCL disorders, a group of childhood neurodegenerative diseases. JNCL occurs in approximately 1 in 25,000 births in the United States and Europe and has been reported in many other countries worldwide. Onset occurs between five and eight years of age and symptoms include progressive loss of motor function, seizures, vision loss, and loss of cognitive function, resulting in death before age 30. JNCL is an autosomal recessive disorder caused by mutations of the CLN3 gene. There are forty-nine known mutations of CLN3, but approximately 80% of JNCL cases result from a particular deletion of the CLN3 gene spanning exons 7 and 8 (*CLN3Δ78*). The *CLN3Δ78* deletion causes a frameshift that results in a premature stop codon in exon 9. The truncated protein product of *CLN3Δ78* is 33% of the length of the wild type. The function of the CLN3 protein is not well understood, but it is implicated in many important processes, for example, membrane trafficking, phospholipid distribution, and response to oxidative stress. Currently, there are no treatments for any of the NCL disorders, and patient options are limited to remedial management of symptoms (*see* Bennett and Rakheja, Dev. Disabil. Res. Rev. 2013, 17, 254-259).

Antisense compounds have been used to modulate target nucleic acids. Antisense compounds comprising a variety of chemical modifications and motifs have been reported. In certain instances, such compounds are useful as research tools, diagnostic reagents, and as therapeutic agents. In certain instances antisense compounds have been shown to modulate protein expression by binding to a target messenger RNA (mRNA) encoding the protein. In certain instances, such binding of an antisense compound to its target mRNA results in cleavage of the mRNA. Antisense compounds that modulate processing of a pre-mRNA have also been reported. Such antisense compounds alter splicing, interfere with polyadenlyation or prevent formation of the 5'-cap of a pre-mRNA.

Certain antisense compounds have been described previously. See for example United States Patent No. 7,399,845 and published International Patent Application No. WO 2008/049085.

WO 2013/066438 describes the evaluation and improvement of the nuclease cleavage specificity of engineered nucleases (e.g. zinc finger nucleases, transcriptional activator-like effector nucleases, etc.)

Svetlana N Rylova *et al.* (2002) describes the effects of blocking CLN3 protein expression on cancer cell growth, survival, ceramide production and apoptosis by using an adenovirus-bearing antisense CLN3 construct.

### SUMMARY

The invention provides a compound comprising a modified oligonucleotide consisting of 8 to 30 linked nucleosides and having a nucleobase sequence comprising a complementary region, wherein the complementary region comprises at least 8 contiguous nucleobases and is 100% complementary to an equal-length portion of a target region of a CLN3 transcript, wherein:
(i) the nucleobase sequence of the oligonucleotide is at least 90% complementary to an equal-length region of the CLN3 transcript, as measured over the entire length of the oligonucleotide; and
(ii) the modified oligonucleotide does not comprise more than 4 contiguous unmodified 2'deoxynucleosides; and
wherein the compound is able to induce skipping of one or more exons of the CLN3 transcript,
for use in the treatment of Batten Disease or for use in delaying or preventing the onset of Batten Disease.

The invention also provides a pharmaceutical composition comprising a compound comprising a modified oligonucleotide consisting of 8 to 30 linked nucleosides and having a nucleobase sequence comprising a complementary region, wherein the complementary region comprises at least 8 contiguous nucleobases and is 100% complementary to an equal-length portion of a target region of a CLN3 transcript, wherein:
(i) the nucleobase sequence of the oligonucleotide is at least 90% complementary to an equal-length region of the CLN3 transcript, as measured over the entire length of the oligonucleotide; and
(ii) the modified oligonucleotide does not comprise more than 4 contiguous unmodified 2'deoxynucleosides; and
wherein the compound is able to induce skipping of one or more exons of the CLN3 transcript, and a pharmaceutically acceptable carrier or diluent,
for use in the treatment of Batten Disease or for use in delaying or preventing the onset of Batten Disease.

Many JNCL cases result from a particular deletion of the CLN3 gene spanning exons 7 and 8 (*CLN3Δ78*). The *CLN3Δ78* deletion causes a frameshift that results in a premature stop codon in exon 9. The truncated protein product of *CLN3Δ78* is 33% of the length of the wild type. In certain embodiments, the truncated *CLN3Δ78* protein causes a variety of cellular defects, including lysosomal and transporter dysfunction. In certain embodiments, modified oligonucleotides targeted to CLN3 pre-mRNA are able to induce skipping of one or more exons and thereby prevent the frameshift that results in a premature stop codon in exon 9.

For example, in certain embodiments, modified oligonucleotides targeted to exon 6 CLN3 pre-mRNA may induce skipping on exon 6 and prevent recognition of the premature stop codon in exon 9, thereby producing a truncated CLN3 protein having restored functionality compared to the *CLN3Δ78* isoform. In certain embodiments, modified oligonucleotides targeted to exon 9 CLN3 pre-mRNA may induce skipping on exon 9 and prevent recognition of the premature stop codon in exon 9, thereby producing a truncated CLN3 protein having restored functionality compared to the *CLN3Δ78* isoform.

In certain embodiments, the present disclosure relates to compounds comprising oligonucleotides. In certain embodiments, such oligonucleotides are complementary to a ceroid-lipofuscinosis, neuronal 3 ("CLN3") transcript. In certain such embodiments, oligonucleotides are complementary to a target region of the CLN3 transcript comprising exon 6. In certain such embodiments, oligonucleotides are complementary to a target region of the CLN3 transcript comprising an intron adjacent to exon 6. In certain such embodiments, oligonucleotides are complementary to a target region of the CLN3 transcript comprising an intron adjacent to exon 6 and downstream of exon 6. In certain such embodiments, oligonucleotides are complementary to a target region of the CLN3 transcript comprising an intron adjacent to exon 6 and upstream of exon 6.

In certain such embodiments, oligonucleotides are complementary to a target region of the CLN3 transcript comprising exon 9. In certain such embodiments, oligonucleotides are complementary to a target region of the CLN3 transcript comprising an intron adjacent to exon 9. In certain such embodiments, oligonucleotides are complementary to a target region of the CLN3 transcript comprising an intron adjacent to exon 9 and downstream of exon 9. In certain such embodiments, oligonucleotides are complementary to a target region of the CLN3 transcript comprising an intron adjacent to exon 9 and upstream of exon 9.

In certain embodiments, oligonucleotides promote skipping of exon 6. In certain embodiments, oligonucleotides promote skipping of exon 6 of a *CLN3Δ78* transcript. In certain embodiments, oligonucleotides promote skipping of exon 9. In certain embodiments, oligonucleotides promote skipping of exon 9 of a *CLN3Δ78* transcript.

In certain embodiments, oligonucleotides promote skipping of exons 6, 7, and 8. In certain embodiments, oligonucleotides promote skipping of exons 7, 8, and 9. In certain embodiments, oligonucleotides promote skipping of exon 6. In certain embodiments, oligonucleotides promote skipping of exon 9.

In certain embodiments, the CLN3 transcript is in a human. In certain embodiments, the CLN3 transcript is in a mouse.

### BRIEF DESCRIPTION OF THE FIGURES

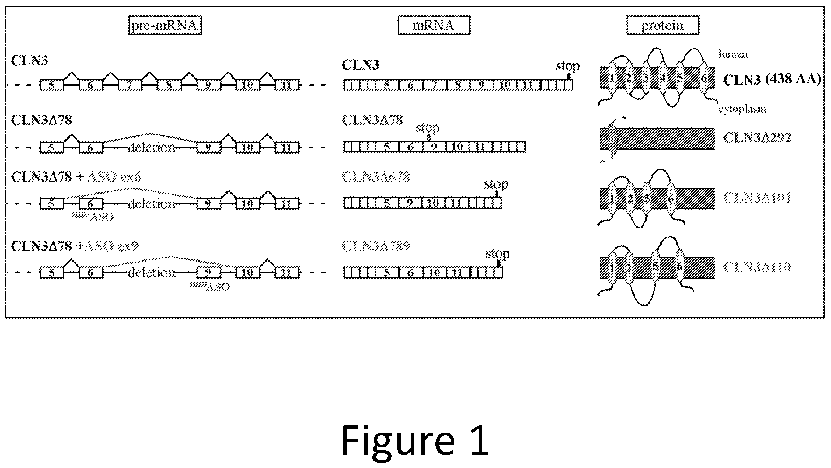

Figure 1 is a schematic of the human WT CLN3 and mutant CLN3Δ78 RNA and proteins. The splice products, CLN3Δ678 and CLN3Δ789, are depicted as well. Exons are depicted as boxes, introns are depicted as lines. In certain embodiments, ASOs are used to alter the splicing of CLN3Δ78 pre-mRNA.
Figure 2 shows splice products produced following treatment of WT, heterozygous, and homozygous mutant CLN3Δ78 fibroblasts with an ASO targeted to exon 6 of CLN3 and analysis by RT-PCR.
Figure 3 shows splice products produced following treatment of homozygous mutant CLN3A78 fibroblasts with varying concentrations of an ASO targeted to exon 6 of CLN3 and analysis by RT-PCR.
Figure 4 shows a western blot illustrating protein products produced following treatment of WT, heterozygous, and homozygous mutant CLN3Δ78 fibroblasts with an ASO targeted to exon 6 and a control ASO.
Figure 5 shows the RT-PCR results of a screen of ASOs targeted to exon 6 of mouse CLN3.
Figure 6 shows the RT-PCR results of a screen of ASOs targeted to exon 9 of mouse CLN3.
Figure 7 shows splice products produced following treatment of homozygous mutant CLN3Δ78 Batten mice with an ASO targeted to exon 6 of CLN3 (616709) or a control ASO and analysis by RT-PCR.
Figure 8 shows the RT-PCR results of a screen of ASOs targeted to exon 6 of human CLN3.
Figure 9 shows the RT-PCR results of a screen of ASOs targeted to exon 9 of human CLN3.
Figure 10 shows splice products produced following treatment of homozygous mutant CLN3Δ78 fibroblasts with ASOs targeted to exon 6 or exon 9 of human CLN3.

### DETAILED DESCRIPTION

Unless specific definitions are provided, the nomenclature used in connection with, and the procedures and techniques of, analytical chemistry, synthetic organic chemistry, and medicinal and pharmaceutical chemistry described herein are those well known and commonly used in the art. Standard techniques may be used for chemical synthesis, and chemical analysis. Certain such techniques and procedures may be found for example in "Carbohydrate Modifications in Antisense Research" Edited by Sangvi and Cook, American Chemical Society , Washington D.C., 1994; "Remington's Pharmaceutical Sciences," Mack Publishing Co., Easton, Pa., 21st edition, 2005; and "Antisense Drug Technology, Principles, Strategies, and Applications" Edited by Stanley T. Crooke, CRC Press, Boca Raton, Florida; and Sambrook et al., "Molecular Cloning, A laboratory Manual," 2nd Edition, Cold Spring Harbor Laboratory Press, 1989.

Unless otherwise indicated, the following terms have the following meanings:
As used herein, "nucleoside" means a compound comprising a nucleobase moiety and a sugar moiety. Nucleosides include, but are not limited to, naturally occurring nucleosides (as found in DNA and RNA) and modified nucleosides. Nucleosides may be linked to a phosphate moiety.

As used herein, "chemical modification" means a chemical difference in a compound when compared to a naturally occurring counterpart. In reference to an oligonucleotide, chemical modification does not include differences only in nucleobase sequence. Chemical modifications of oligonucleotides include nucleoside modifications (including sugar moiety modifications and nucleobase modifications) and internucleoside linkage modifications.

As used herein, "furanosyl" means a structure comprising a 5-membered ring comprising four carbon atoms and one oxygen atom.

As used herein, "naturally occurring sugar moiety" means a ribofuranosyl as found in naturally occurring RNA or a deoxyribofuranosyl as found in naturally occurring DNA.

As used herein, "sugar moiety" means a naturally occurring sugar moiety or a modified sugar moiety of a nucleoside.

As used herein, "modified sugar moiety" means a substituted sugar moiety, a bicyclic or tricyclic sugar moiety, or a sugar surrogate.

As used herein, "substituted sugar moiety" means a furanosyl comprising at least one substituent group that differs from that of a naturally occurring sugar moiety. Substituted sugar moieties include, but are not limited to furanosyls comprising substituents at the 2'-position, the 3'-position, the 5'-position and/or the 4'-position.

As used herein, "2'-substituted sugar moiety" means a furanosyl comprising a substituent at the 2'-position other than H or OH. Unless otherwise indicated, a 2'-substituted sugar moiety is not a bicyclic sugar moiety (i.e., the 2'-substituent of a 2'-substituted sugar moiety does not form a bridge to another atom of the furanosyl ring.

As used herein, "MOE" means -OCH₂CH₂OCH₃.

As used herein, "bicyclic sugar moiety" means a modified sugar moiety comprising a 4 to 7 membered ring (including but not limited to a furanosyl) comprising a bridge connecting two atoms of the 4 to 7 membered ring to form a second ring, resulting in a bicyclic structure. In certain embodiments, the 4 to 7 membered ring is a sugar ring. In certain embodiments the 4 to 7 membered ring is a furanosyl. In certain such embodiments, the bridge connects the 2'-carbon and the 4'-carbon of the furanosyl.

As used herein the term "sugar surrogate" means a structure that does not comprise a furanosyl and that is capable of replacing the naturally occurring sugar moiety of a nucleoside, such that the resulting nucleoside is capable of (1) incorporation into an oligonucleotide and (2) hybridization to a complementary nucleoside. Such structures include rings comprising a different number of atoms than furanosyl (e.g., 4, 6, or 7-membered rings); replacement of the oxygen of a furanosyl with a non-oxygen atom (e.g., carbon, sulfur, or nitrogen); or both a change in the number of atoms and a replacement of the oxygen. Such structures may also comprise substitutions corresponding to those described for substituted sugar moieties (e.g., 6-membered carbocyclic bicyclic sugar surrogates optionally comprising additional substituents). Sugar surrogates also include more complex sugar replacements (e.g., the non-ring systems of peptide nucleic acid). Sugar surrogates include without limitation morpholino, modified morpholinos, cyclohexenyls and cyclohexitols.

As used herein, "nucleotide" means a nucleoside further comprising a phosphate linking group. As used herein, "linked nucleosides" may or may not be linked by phosphate linkages and thus includes, but is not limited to "linked nucleotides." As used herein, "linked nucleosides" are nucleosides that are connected in a continuous sequence (i.e. no additional nucleosides are present between those that are linked).

As used herein, "nucleobase" means a group of atoms that can be linked to a sugar moiety to create a nucleoside that is capable of incorporation into an oligonucleotide, and wherein the group of atoms is capable of bonding with a complementary naturally occurring nucleobase of another oligonucleotide or nucleic acid. Nucleobases may be naturally occurring or may be modified.

As used herein, "heterocyclic base" or "heterocyclic nucleobase" means a nucleobase comprising a heterocyclic structure.

As used herein the terms, "unmodified nucleobase" or "naturally occurring nucleobase" means the naturally occurring heterocyclic nucleobases of RNA or DNA: the purine bases adenine (A) and guanine (G), and the pyrimidine bases thymine (T), cytosine (C) (including 5-methyl C), and uracil (U).

As used herein, "modified nucleobase" means any nucleobase that is not a naturally occurring nucleobase.

As used herein, "modified nucleoside" means a nucleoside comprising at least one chemical modification compared to naturally occurring RNA or DNA nucleosides. Modified nucleosides comprise a modified sugar moiety and/or a modified nucleobase.

As used herein, "bicyclic nucleoside" or "BNA" means a nucleoside comprising a bicyclic sugar moiety.

As used herein, "constrained ethyl nucleoside" or "cEt" means a nucleoside comprising a bicyclic sugar moiety comprising a 4'-CH(CH₃)-O-2'bridge.

As used herein, "locked nucleic acid nucleoside" or "LNA" means a nucleoside comprising a bicyclic sugar moiety comprising a 4'-CH₂-O-2'bridge.

As used herein, "2'-substituted nucleoside" means a nucleoside comprising a substituent at the 2'-position other than H or OH. Unless otherwise indicated, a 2'-substituted nucleoside is not a bicyclic nucleoside.

As used herein, "2'-deoxynucleoside" means a nucleoside comprising 2'-H furanosyl sugar moiety, as found in naturally occurring deoxyribonucleosides (DNA). In certain embodiments, a 2'-deoxynucleoside may comprise a modified nucleobase or may comprise an RNA nucleobase (e.g., uracil).

As used herein, "oligonucleotide" means a compound comprising a plurality of linked nucleosides. In certain embodiments, an oligonucleotide comprises one or more unmodified ribonucleosides (RNA) and/or unmodified deoxyribonucleosides (DNA) and/or one or more modified nucleosides.

As used herein "oligonucleoside" means an oligonucleotide in which none of the internucleoside linkages contains a phosphorus atom. As used herein, oligonucleotides include oligonucleosides.

As used herein, "modified oligonucleotide" means an oligonucleotide comprising at least one modified nucleoside and/or at least one modified internucleoside linkage.

As used herein "internucleoside linkage" means a covalent linkage between adjacent nucleosides in an oligonucleotide.

As used herein "naturally occurring internucleoside linkage" means a 3' to 5' phosphodiester linkage.

As used herein, "modified internucleoside linkage" means any internucleoside linkage other than a naturally occurring internucleoside linkage.

As used herein, "oligomeric compound" means a polymeric structure comprising two or more substructures. In certain embodiments, an oligomeric compound comprises an oligonucleotide. In certain embodiments, an oligomeric compound comprises one or more conjugate groups and/or terminal groups. In certain embodiments, an oligomeric compound consists of an oligonucleotide.

As used herein, "terminal group" means one or more atom attached to either, or both, the 3' end or the 5' end of an oligonucleotide. In certain embodiments a terminal group is a conjugate group. In certain embodiments, a terminal group comprises one or more terminal group nucleosides.

As used herein, "conjugate" means an atom or group of atoms bound to an oligonucleotide or oligomeric compound. In general, conjugate groups modify one or more properties of the compound to which they are attached, including, but not limited to pharmacodynamic, pharmacokinetic, binding, absorption, cellular distribution, cellular uptake, charge and/or clearance properties.

As used herein, "conjugate linking group" means any atom or group of atoms used to attach a conjugate to an oligonucleotide or oligomeric compound.

As used herein, "antisense compound" means a compound comprising or consisting of an oligonucleotide at least a portion of which is complementary to a target nucleic acid to which it is capable of hybridizing, resulting in at least one antisense activity.

As used herein, "antisense activity" means any detectable and/or measurable change attributable to the hybridization of an antisense compound to its target nucleic acid.

As used herein, "detecting" or "measuring" means that a test or assay for detecting or measuring is performed. Such detection and/or measuring may result in a value of zero. Thus, if a test for detection or measuring results in a finding of no activity (activity of zero), the step of detecting or measuring the activity has nevertheless been performed.

As used herein, "detectable and/or measureable activity" means a statistically significant activity that is not zero.

As used herein, "essentially unchanged" means little or no change in a particular parameter, particularly relative to another parameter which changes much more. In certain embodiments, a parameter is essentially unchanged when it changes less than 5%. In certain embodiments, a parameter is essentially unchanged if it changes less than two-fold while another parameter changes at least ten-fold. For example, in certain embodiments, an antisense activity is a change in the amount of a target nucleic acid. In certain such embodiments, the amount of a non-target nucleic acid is essentially unchanged if it changes much less than the target nucleic acid does, but the change need not be zero.

As used herein, "expression" means the process by which a gene ultimately results in a protein. Expression includes, but is not limited to, transcription, post-transcriptional modification (e.g., splicing, polyadenlyation, addition of 5'-cap), and translation.

As used herein, "target nucleic acid" means a nucleic acid molecule to which an antisense compound hybridizes.

As used herein, "mRNA" means an RNA molecule that encodes a protein.

As used herein, "pre-mRNA" means an RNA transcript that has not been fully processed into mRNA. Pre-RNA includes one or more intron.

As used herein, "transcript" means an RNA molecule transcribed from DNA. Transcripts include, but are not limitied to mRNA, pre-mRNA, and partially processed RNA.

As used herein, "CLN3" means ceroid-lipofuscinosis, neuronal 3.

As used herein, "CLN3 transcript" means a transcript transcribed from a CLN3 gene. In certain embodiments, a CLN3 transcript comprises SEQ ID NO: 1: the complement of GENBANK accession number NT_010393.16 truncated from nucleotides 28427600 to 28444620. In certain embodiments, a CLN3 transcript comprises SEQ ID NO: 2: the complement of GENBANK accession number NT_039433.8, truncated from nucleotides 44319075 to 44333955.

As used herein, "CLN3 gene" means a gene that encodes a ceroid-lipofuscinosis, neuronal 3 protein and any ceroid-lipofuscinosis, neuronal 3 protein isoforms. In certain embodiments, a CLN3 gene is represented by GENBANK accession number NT_010393.16 truncated from nucleotides 28427600 to 28444620, or a variant thereof. In certain embodiments, a CLN3 gene is at least 95% identical to GENBANK accession number NT_010393.16 truncated from nucleotides 28427600 to 28444620. In certain embodiments, a CLN3 gene is at least 90% identical to GENBANK accession number NT_010393.16 truncated from nucleotides 28427600 to 28444620. In certain embodiments, a CLN3 gene is represented by GENBANK accession number NT_039433.8, truncated from nucleotides 44319075 to 44333955, or a variant thereof. In certain embodiments, a CLN3 gene is at least 95% identical to GENBANK accession number NT_039433.8, truncated from nucleotides 44319075 to 44333955. In certain embodiments, a CLN3 gene is at least 90% identical to GENBANK accession number NT_039433.8, truncated from nucleotides 44319075 to 44333955. In certain embodiments, a CLN3 gene encodes a wild-type CLN3 protein. In certain embodiments, a CLN3 gene encodes a *CLN3Δ78* protein.

As used herein, *"CLN3Δ78"* means a CLN3 gene having a deletion spanning all or part of exons 7 and 8. In certain embodiments, the *CLN3Δ78* deletion causes a frameshift that result in a premature stop codon in exon 9. In certain embodiments, the truncated protein product of *CLN3Δ78* is 33% of the length of the wild type.

As used herein, "targeting" or "targeted to" means the association of an antisense compound to a particular target nucleic acid molecule or a particular region of a target nucleic acid molecule. An antisense compound targets a target nucleic acid if it is sufficiently complementary to the target nucleic acid to allow hybridization under physiological conditions.

As used herein, "nucleobase complementarity" or "complementarity" when in reference to nucleobases means a nucleobase that is capable of base pairing with another nucleobase. For example, in DNA, adenine (A) is complementary to thymine (T). For example, in RNA, adenine (A) is complementary to uracil (U). In certain embodiments, complementary nucleobase means a nucleobase of an antisense compound that is capable of base pairing with a nucleobase of its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be complementary at that nucleobase pair. Nucleobases comprising certain modifications may maintain the ability to pair with a counterpart nucleobase and thus, are still capable of nucleobase complementarity.

As used herein, "non-complementary" in reference to nucleobases means a pair of nucleobases that do not form hydrogen bonds with one another.

As used herein, "complementary" in reference to oligomeric compounds (e.g., linked nucleosides, oligonucleotides, or nucleic acids) means the capacity of such oligomeric compounds or regions thereof to hybridize to another oligomeric compound or region thereof through nucleobase complementarity under stringent conditions. Complementary oligomeric compounds need not have nucleobase complementarity at each nucleoside. Rather, some mismatches are tolerated. In certain embodiments, complementary oligomeric compounds or regions are complementary at 70% of the nucleobases (70% complementary). In certain embodiments, complementary oligomeric compounds or regions are 80% complementary. In certain embodiments, complementary oligomeric compounds or regions are 90% complementary. In certain embodiments, complementary oligomeric compounds or regions are 95% complementary. In certain embodiments, complementary oligomeric compounds or regions are 100% complementary.

As used herein, "hybridization" means the pairing of complementary oligomeric compounds (e.g., an antisense compound and its target nucleic acid). While not limited to a particular mechanism, the most common mechanism of pairing involves hydrogen bonding, which may be Watson-Crick, Hoogsteen or reversed Hoogsteen hydrogen bonding, between complementary nucleobases.

As used herein, "specifically hybridizes" means the ability of an oligomeric compound to hybridize to one nucleic acid site with greater affinity than it hybridizes to another nucleic acid site. In certain embodiments, an antisense oligonucleotide specifically hybridizes to more than one target site.

As used herein, "percent complementarity" means the percentage of nucleobases of an oligomeric compound that are complementary to an equal-length portion of a target nucleic acid. Percent complementarity is calculated by dividing the number of nucleobases of the oligomeric compound that are complementary to nucleobases at corresponding positions in the target nucleic acid by the total length of the oligomeric compound.

As used herein, "percent identity" means the number of nucleobases in a first nucleic acid that are the same type (independent of chemical modification) as nucleobases at corresponding positions in a second nucleic acid, divided by the total number of nucleobases in the first nucleic acid.

As used herein, "modulation" means a change of amount or quality of a molecule, function, or activity when compared to the amount or quality of a molecule, function, or activity prior to modulation. For example, modulation includes the change, either an increase (stimulation or induction) or a decrease (inhibition or reduction) in gene expression. As a further example, modulation of expression can include a change in splice site selection of pre-mRNA processing, resulting in a change in the absolute or relative amount of a particular splice-variant compared to the amount in the absence of modulation.

As used herein, "motif" means a pattern of chemical modifications in an oligomeric compound or a region thereof. Motifs may be defined by modifications at certain nucleosides and/or at certain linking groups of an oligomeric compound.

As used herein, "nucleoside motif" means a pattern of nucleoside modifications in an oligomeric compound or a region thereof. The linkages of such an oligomeric compound may be modified or unmodified. Unless otherwise indicated, motifs herein describing only nucleosides are intended to be nucleoside motifs. Thus, in such instances, the linkages are not limited.

As used herein, "sugar motif" means a pattern of sugar modifications in an oligomeric compound or a region thereof.

As used herein, "linkage motif" means a pattern of linkage modifications in an oligomeric compound or region thereof. The nucleosides of such an oligomeric compound may be modified or unmodified. Unless otherwise indicated, motifs herein describing only linkages are intended to be linkage motifs. Thus, in such instances, the nucleosides are not limited.

As used herein, "nucleobase modification motif" means a pattern of modifications to nucleobases along an oligonucleotide. Unless otherwise indicated, a nucleobase modification motif is independent of the nucleobase sequence.

As used herein, "sequence motif" means a pattern of nucleobases arranged along an oligonucleotide or portion thereof. Unless otherwise indicated, a sequence motif is independent of chemical modifications and thus may have any combination of chemical modifications, including no chemical modifications.

As used herein, "type of modification" in reference to a nucleoside or a nucleoside of a "type" means the chemical modification of a nucleoside and includes modified and unmodified nucleosides. Accordingly, unless otherwise indicated, a "nucleoside having a modification of a first type" may be an unmodified nucleoside.

As used herein, "differently modified" mean chemical modifications or chemical substituents that are different from one another, including absence of modifications. Thus, for example, a MOE nucleoside and an unmodified DNA nucleoside are "differently modified," even though the DNA nucleoside is unmodified. Likewise, DNA and RNA are "differently modified," even though both are naturally-occurring unmodified nucleosides. Nucleosides that are the same but for comprising different nucleobases are not differently modified. For example, a nucleoside comprising a 2'-OMe modified sugar and an unmodified adenine nucleobase and a nucleoside comprising a 2'-OMe modified sugar and an unmodified thymine nucleobase are not differently modified.

As used herein, "the same type of modifications" refers to modifications that are the same as one another, including absence of modifications. Thus, for example, two unmodified DNA nucleoside have "the same type of modification," even though the DNA nucleoside is unmodified. Such nucleosides having the same type modification may comprise different nucleobases.

As used herein, "pharmaceutically acceptable carrier or diluent" means any substance suitable for use in administering to an animal. In certain embodiments, a pharmaceutically acceptable carrier or diluent is sterile saline. In certain embodiments, such sterile saline is pharmaceutical grade saline.

As used herein, "substituent" and "substituent group," means an atom or group that replaces the atom or group of a named parent compound. For example a substituent of a modified nucleoside is any atom or group that differs from the atom or group found in a naturally occurring nucleoside (e.g., a modified 2'-substuent is any atom or group at the 2'-position of a nucleoside other than H or OH). Substituent groups can be protected or unprotected. In certain embodiments, compounds of use in the present invention have substituents at one or at more than one position of the parent compound. Substituents may also be further substituted with other substituent groups and may be attached directly or via a linking group such as an alkyl or hydrocarbyl group to a parent compound.

Likewise, as used herein, "substituent" in reference to a chemical functional group means an atom or group of atoms differs from the atom or a group of atoms normally present in the named functional group. In certain embodiments, a substituent replaces a hydrogen atom of the functional group (e.g., in certain embodiments, the substituent of a substituted methyl group is an atom or group other than hydrogen which replaces one of the hydrogen atoms of an unsubstituted methyl group). Unless otherwise indicated, groups amenable for use as substituents include without limitation, halogen, hydroxyl, alkyl, alkenyl, alkynyl, acyl (-C(O)Rₐₐ), carboxyl (-C(O)O-Rₐₐ), aliphatic groups, alicyclic groups, alkoxy, substituted oxy (-O-Rₐₐ), aryl, aralkyl, heterocyclic radical, heteroaryl, heteroarylalkyl, amino (-N(R_{bb})(R_{cc})), imino(=NR_{bb}), amido (-C(O)N(Rbb)(R_{cc}) or -N(Rbb)C(O)Rₐₐ), azido (-N₃), nitro (-NO₂), cyano (-CN), carbamido (-OC(O)N(R_{bb})(Rₑₑ) or -N(R_{bb})C(O)ORₐₐ), ureido (-N(R_{bb})C(O)N(R_{bb})(R_{cc})), thioureido (-N(R_{bb})C(S)N(R_{bb})-(Rcc)), guanidinyl (-N(R_{bb})C(=NR_{bb})N(R_{bb})(R_{cc})), amidinyl (-C(=NR_{bb})N(R_{bb})(R_{cc}) or -N(R_{bb})C(=NR_{bb})(Rₐₐ)), thiol (-SR_{bb}), sulfinyl (-S(O)R_{bb}), sulfonyl (-S(O)₂R_{bb}) and sulfonamidyl (-S(O)₂N(R_{bb})(R_{cc}) or -N(R_{bb})S-(O)₂R_{bb}). Wherein each Rₐₐ, R_{bb} and R_{cc} is, independently, H, an optionally linked chemical functional group or a further substituent group with a preferred list including without limitation, alkyl, alkenyl, alkynyl, aliphatic, alkoxy, acyl, aryl, aralkyl, heteroaryl, alicyclic, heterocyclic and heteroarylalkyl. Selected substituents within the compounds described herein are present to a recursive degree.

As used herein, "alkyl," as used herein, means a saturated straight or branched hydrocarbon radical containing up to twenty four carbon atoms. Examples of alkyl groups include without limitation, methyl, ethyl, propyl, butyl, isopropyl, n-hexyl, octyl, decyl, dodecyl and the like. Alkyl groups typically include from 1 to about 24 carbon atoms, more typically from 1 to about 12 carbon atoms (C₁-C₁₂ alkyl) with from 1 to about 6 carbon atoms being more preferred.

As used herein, "alkenyl," means a straight or branched hydrocarbon chain radical containing up to twenty four carbon atoms and having at least one carbon-carbon double bond. Examples of alkenyl groups include without limitation, ethenyl, propenyl, butenyl, 1-methyl-2-buten-1-yl, dienes such as 1,3-butadiene and the like. Alkenyl groups typically include from 2 to about 24 carbon atoms, more typically from 2 to about 12 carbon atoms with from 2 to about 6 carbon atoms being more preferred. Alkenyl groups as used herein may optionally include one or more further substituent groups.

As used herein, "alkynyl," means a straight or branched hydrocarbon radical containing up to twenty four carbon atoms and having at least one carbon-carbon triple bond. Examples of alkynyl groups include, without limitation, ethynyl, 1-propynyl, 1 -butynyl, and the like. Alkynyl groups typically include from 2 to about 24 carbon atoms, more typically from 2 to about 12 carbon atoms with from 2 to about 6 carbon atoms being more preferred. Alkynyl groups as used herein may optionally include one or more further substituent groups.

As used herein, "acyl," means a radical formed by removal of a hydroxyl group from an organic acid and has the general Formula -C(O)-X where X is typically aliphatic, alicyclic or aromatic. Examples include aliphatic carbonyls, aromatic carbonyls, aliphatic sulfonyls, aromatic sulfinyls, aliphatic sulfinyls, aromatic phosphates, aliphatic phosphates and the like. Acyl groups as used herein may optionally include further substituent groups.

As used herein, "alicyclic" means a cyclic ring system wherein the ring is aliphatic. The ring system can comprise one or more rings wherein at least one ring is aliphatic. Preferred alicyclics include rings having from about 5 to about 9 carbon atoms in the ring. Alicyclic as used herein may optionally include further substituent groups.

As used herein, "aliphatic" means a straight or branched hydrocarbon radical containing up to twenty four carbon atoms wherein the saturation between any two carbon atoms is a single, double or triple bond. An aliphatic group preferably contains from 1 to about 24 carbon atoms, more typically from 1 to about 12 carbon atoms with from 1 to about 6 carbon atoms being more preferred. The straight or branched chain of an aliphatic group may be interrupted with one or more heteroatoms that include nitrogen, oxygen, sulfur and phosphorus. Such aliphatic groups interrupted by heteroatoms include without limitation, polyalkoxys, such as polyalkylene glycols, polyamines, and polyimines. Aliphatic groups as used herein may optionally include further substituent groups.

As used herein, "alkoxy" means a radical formed between an alkyl group and an oxygen atom wherein the oxygen atom is used to attach the alkoxy group to a parent molecule. Examples of alkoxy groups include without limitation, methoxy, ethoxy, propoxy, isopropoxy, n-butoxy, sec-butoxy, *tert*-butoxy, n-pentoxy, neopentoxy, n-hexoxy and the like. Alkoxy groups as used herein may optionally include further substituent groups.

As used herein, "aminoalkyl" means an amino substituted C₁-C₁₂ alkyl radical. The alkyl portion of the radical forms a covalent bond with a parent molecule. The amino group can be located at any position and the aminoalkyl group can be substituted with a further substituent group at the alkyl and/or amino portions.

As used herein, "aralkyl" and "arylalkyl" mean an aromatic group that is covalently linked to a C₁-C₁₂ alkyl radical. The alkyl radical portion of the resulting aralkyl (or arylalkyl) group forms a covalent bond with a parent molecule. Examples include without limitation, benzyl, phenethyl and the like. Aralkyl groups as used herein may optionally include further substituent groups attached to the alkyl, the aryl or both groups that form the radical group.

As used herein, "aryl" and "aromatic" mean a mono- or polycyclic carbocyclic ring system radicals having one or more aromatic rings. Examples of aryl groups include without limitation, phenyl, naphthyl, tetrahydronaphthyl, indanyl, idenyl and the like. Preferred aryl ring systems have from about 5 to about 20 carbon atoms in one or more rings. Aryl groups as used herein may optionally include further substituent groups.

As used herein, "halo" and "halogen," mean an atom selected from fluorine, chlorine, bromine and iodine.

As used herein, "heteroaryl," and "heteroaromatic," mean a radical comprising a mono- or polycyclic aromatic ring, ring system or fused ring system wherein at least one of the rings is aromatic and includes one or more heteroatoms. Heteroaryl is also meant to include fused ring systems including systems where one or more of the fused rings contain no heteroatoms. Heteroaryl groups typically include one ring atom selected from sulfur, nitrogen or oxygen. Examples of heteroaryl groups include without limitation, pyridinyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, benzimidazolyl, benzooxazolyl, quinoxalinyl and the like. Heteroaryl radicals can be attached to a parent molecule directly or through a linking moiety such as an aliphatic group or hetero atom. Heteroaryl groups as used herein may optionally include further substituent groups.

### Oligomeric Compounds

The present invention relates to oligomeric compounds. In certain embodiments, such oligomeric compounds comprise oligonucleotides optionally comprising one or more conjugate and/or terminal groups. In certain embodiments, an oligomeric compound consists of an oligonucleotide. In certain embodiments, oligonucleotides comprise one or more chemical modifications. Such chemical modifications include modifications one or more nucleoside (including modifications to the sugar moiety and/or the nucleobase) and/or modifications to one or more internucleoside linkage.

### Certain Sugar Moieties

In certain embodiments, oligomeric compounds of use in the invention comprise one or more modifed nucleosides comprising a modifed sugar moiety. Such oligomeric compounds comprising one or more sugar-modified nucleosides may have desirable properties, such as enhanced nuclease stability or increased binding affinity with a target nucleic acid relative to oligomeric compounds comprising only nucleosides comprising naturally occurring sugar moieties. In certain embodiments, modified sugar moieties are substitued sugar moieties. In certain embodiments, modified sugar moieties are bicyclic or tricyclic sugar moieties. In certain embodiments, modified sugar moieties are sugar surrogates. Such sugar surogates may comprise one or more substitutions corresponding to those of substituted sugar moieties.

In certain embodiments, modified sugar moieties are substituted sugar moieties comprising one or more substituent, including but not limited to substituents at the 2' and/or 5' positions. Examples of sugar substituents suitable for the 2'-position, include, but are not limited to: 2'-F, 2'-OCH₃ ("OMe" or "O-methyl"), and 2'-O(CH₂)₂OCH₃ ("MOE"). In certain embodiments, sugar substituents at the 2' position is selected from allyl, amino, azido, thio, O-allyl, O-C₁-C₁₀ alkyl, O-C₁-C₁₀ substituted alkyl; O- C₁-C₁₀ alkoxy; O- C₁-C₁₀ substituted alkoxy, OCF₃, O(CH₂)₂SCH₃, O(CH₂)₂-O-N(Rm)(Rn), and O-CH₂-C(=O)-N(Rm)(Rn), where each Rm and Rn is, independently, H or substituted or unsubstituted C₁-C₁₀ alkyl. Examples of sugar substituents at the 5'-position, include, but are not limited to:, 5'-methyl (R or S); 5'-vinyl, and 5'-methoxy. In certain embodiments, substituted sugars comprise more than one non-bridging sugar substituent, for example, 2'-F-5'-methyl sugar moieties (*see,* e.g., PCT International Application WO 2008/101157, for additional 5', 2'-bis substituted sugar moieties and nucleosides).

Nucleosides comprising 2'-substituted sugar moieties are referred to as 2'-substituted nucleosides. In certain embodiments, a 2'- substituted nucleoside comprises a 2'-substituent group selected from halo, allyl, amino, azido, O- C₁-C₁₀ alkoxy; O- C₁-C₁₀ substituted alkoxy, SH, CN, OCN, CF₃, OCF₃, O-alkyl, S-alkyl, N(Rₘ)-alkyl; O- alkenyl, S- alkenyl, or N(Rₘ)-alkenyl; O- alkynyl, S- alkynyl, N(Rₘ)-alkynyl; O-alkylenyl-O-alkyl, alkynyl, alkaryl, aralkyl, O-alkaryl, O-aralkyl, O(CH₂)₂SCH₃, O-(CH₂)₂-O-N(Rₘ)(Rₙ) or O-CH₂-C(=O)-N(Rₘ)(Rₙ), where each Rₘ and Rₙ is, independently, H, an amino protecting group or substituted or unsubstituted C₁-C₁₀ alkyl. These 2'-substituent groups can be further substituted with one or more substituent groups independently selected from hydroxyl, amino, alkoxy, carboxy, benzyl, phenyl, nitro (NO₂), thiol, thioalkoxy (S-alkyl), halogen, alkyl, aryl, alkenyl and alkynyl.

In certain embodiments, a 2'- substituted nucleoside comprises a 2'-substituent group selected from F, NH₂, N₃, OCF₃, O-CH₃, O(CH₂)₃NH₂, CH₂-CH=CH₂, O-CH₂-CH=CH₂, OCH₂CH₂OCH₃, O(CH₂)₂SCH₃, O-(CH₂)₂-O-N(Rₘ)(Rₙ), O(CH₂)₂O(CH₂)₂N(CH₃)₂, and N-substituted acetamide (O-CH₂-C(=O)-N(Rₘ)(Rₙ) where each Rₘ and Rₙ is, independently, H, an amino protecting group or substituted or unsubstituted C₁-C₁₀ alkyl.

In certain embodiments, a 2'- substituted nucleoside comprises a sugar moiety comprising a 2'-substituent group selected from F, OCF₃, O-CH₃, OCH₂CH₂OCH₃, O(CH₂)₂SCH₃, O-(CH₂)₂-O-N(CH₃)₂, -O(CH₂)₂O(CH₂)₂N(CH₃)₂, and O-CH₂-C(=O)-N(H)CH₃.

In certain embodiments, a 2'- substituted nucleoside comprises a sugar moiety comprising a 2'-substituent group selected from F, O-CH₃, and OCH₂CH₂OCH₃.

Certain modifed sugar moieties comprise a bridging sugar substituent that forms a second ring resulting in a bicyclic sugar moiety. In certain such embodiments, the bicyclic sugar moiety comprises a bridge between the 4' and the 2' furanose ring atoms. Examples of such 4' to 2' sugar substituents, include, but are not limited to: -[C(Rₐ)(R_{b})]ₙ-, -[C(Rₐ)(R_{b})]ₙ-O-, -C(RₐR_{b})-N(R)-O- or, -C(RₐR_{b})-O-N(R)-; 4'-CH₂-2', 4'-(CH₂)₂-2', 4'-(CH₂)₃-2',. 4'-(CH₂)-O-2' (LNA); 4'-(CH₂)-S-2'; 4'-(CH₂)₂-O-2' (ENA); 4'-CH(CH₃)-O-2 ' (cEt) and 4'-CH(CH₂OCH₃)-O-2', and analogs thereof (*see, e.g.,* U.S. Patent 7,399,845, issued on July 15, 2008); 4'-C(CH₃)(CH₃)-O-2' and analogs thereof, (*see, e.g.,* WO2009/006478, published January 8, 2009); 4'-CH₂-N(OCH₃)-2' and analogs thereof (*see, e.g.,* WO2008/150729, published December 11, 2008); 4'-CH₂-O-N(CH₃)-2' (*see, e.g.,* US2004/0171570, published September 2, 2004); 4'-CH₂-O-N(R)-2', and 4'-CH₂-N(R)-O-2'-, wherein each R is, independently, H, a protecting group, or C₁-C₁₂ alkyl; 4'-CH₂-N(R)-O-2', wherein R is H, C₁-C₁₂ alkyl, or a protecting group (*see,* U.S. Patent 7,427,672, issued on September 23, 2008); 4'-CH₂-C(H)(CH₃)-2' (*see, e.g.,* Chattopadhyaya, et al., J. Org. Chem.,2009, 74, 118-134); and 4'-CH₂-C(=CH₂)-2' and analogs thereof (*see,* published PCT International Application WO 2008/154401, published on December 8, 2008).

In certain embodiments, such 4' to 2' bridges independently comprise from 1 to 4 linked groups independently selected from -[C(Rₐ)(R_{b})]ₙ-, -C(Rₐ)=C(R_{b})-, -C(Rₐ)=N-, -C(=NRₐ)-, -C(=O)-, -C(=S)-, -O-,-Si(Rₐ)₂-, -S(=O)ₓ-, and -N(Rₐ)-;
wherein:
x is 0, 1, or 2;
n is 1, 2, 3, or 4;
each Rₐ and R_{b} is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl, or a protecting group.

Nucleosides comprising bicyclic sugar moieties are referred to as bicyclic nucleosides or BNAs. Bicyclic nucleosides include, but are not limited to, (A) α-L-Methyleneoxy (4'-CH₂-O-2') BNA, (B) β-D-Methyleneoxy (4'-CH₂-O-2') BNA (also referred to as locked nucleic acid or LNA), (C) Ethyleneoxy (4'-(CH₂)₂-O-2') BNA, (D) Aminooxy (4'-CH₂-O-N(R)-2') BNA, (E) Oxyamino (4'-CH₂-N(R)-O-2') BNA, (F) Methyl(methyleneoxy) (4'-CH(CH₃)-O-2') BNA (also referred to as constrained ethyl or cEt), (G) methylene-thio (4'-CH₂-S-2') BNA, (H) methylene-amino (4'-CH2-N(R)-2') BNA, (I) methyl carbocyclic (4'-CH₂-CH(CH₃)-2') BNA, and (J) propylene carbocyclic (4'-(CH₂)₃-2') BNA as depicted below. 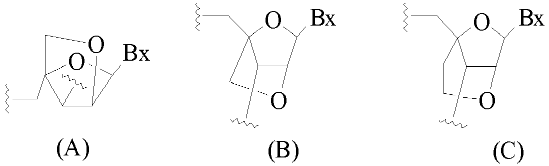 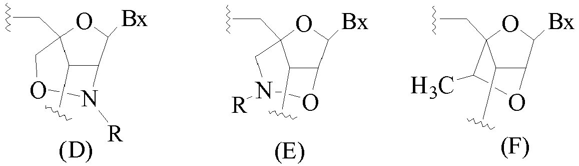 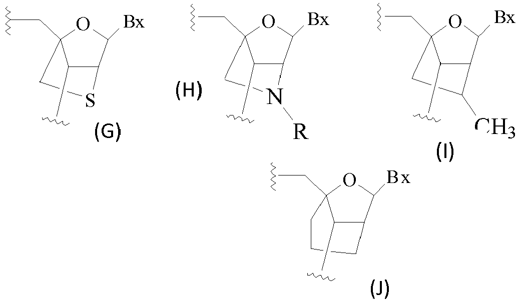 wherein Bx is a nucleobase moiety and R is, independently, H, a protecting group, or C₁-C₁₂ alkyl.

Additional bicyclic sugar moieties are known in the art, for example: Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; Singh et al., J. Org. Chem., 1998, 63, 10035-10039; Srivastava et al., J. Am. Chem. Soc., 129(26) 8362-8379 (Jul. 4, 2007); Elayadi et al., Curr. Opinion Invens. Drugs, 2001, 2, 558-561; Braasch et al., Chem. Biol., 2001, 8, 1-7; Orum et al., Curr. Opinion Mol. Ther., 2001, 3, 239-243; U.S. Patent Nos. 7,053,207, 6,268,490, 6,770,748, 6,794,499, 7,034,133, 6,525,191, 6,670,461, and 7,399,845; WO 2004/106356, WO 1994/14226, WO 2005/021570, and WO 2007/134181; U.S. Patent Publication Nos. US2004/0171570, US2007/0287831, and US2008/0039618; U.S. Patent Serial Nos. 12/129,154, 60/989,574, 61/026,995, 61/026,998, 61/056,564, 61/086,231, 61/097,787, and 61/099,844; and PCT International Applications Nos. PCT/US2008/064591, PCT/US2008/066154, and PCT/US2008/068922.

In certain embodiments, bicyclic sugar moieties and nucleosides incorporating such bicyclic sugar moieties are further defined by isomeric configuration. For example, a nucleoside comprising a 4'-2' methylene-oxy bridge, may be in the α-L configuration or in the β-D configuration. Previously, α-L-methyleneoxy (4'-CH₂-O-2') bicyclic nucleosides have been incorporated into antisense oligonucleotides that showed antisense activity (Frieden et al., Nucleic Acids Research, 2003, 21, 6365-6372).

In certain embodiments, substituted sugar moieties comprise one or more non-bridging sugar substituent and one or more bridging sugar substituent (e.g., 5'-substituted and 4'-2' bridged sugars), (*see,* PCT International Application WO 2007/134181, published on 11/22/07, wherein LNA is substituted with, for example, a 5'-methyl or a 5'-vinyl group).

In certain embodiments, modified sugar moieties are sugar surrogates. In certain such embodiments, the oxygen atom of the naturally occuring sugar is substituted, e.g., with a sulfur, carbon or nitrogen atom. In certain such embodiments, such modified sugar moiety also comprises bridging and/or non-bridging substituents as described above. For example, certain sugar surogates comprise a 4'-sulfur atom and a substitution at the 2'-position (*see,* e.g., published U.S. Patent Application US2005/0130923, published on June 16, 2005) and/or the 5' position. By way of additional example, carbocyclic bicyclic nucleosides having a 4'-2' bridge have been described (*see, e.g.,* Freier et al., Nucleic Acids Research, 1997, 25(22), 4429-4443 and Albaek et al., J. Org. Chem., 2006, 71, 7731-7740).

In certain embodiments, sugar surrogates comprise rings having other than 5-atoms. For example, in certain embodiments, a sugar surrogate comprises a six-membered tetrahydropyran. Such tetrahydropyrans may be further modified or substituted. Nucleosides comprising such modified tetrahydropyrans include, but are not limited to, hexitol nucleic acid (HNA), anitol nucleic acid (ANA), manitol nucleic acid (MNA) *(see* Leumann, CJ. Bioorg. & Med. Chem. (2002) 10:841-854), fluoro HNA (F-HNA), and those compounds having Formula VII: 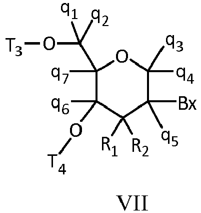 wherein independently for each of said at least one tetrahydropyran nucleoside analog of Formula VII:
Bx is a nucleobase moiety;
T₃ and T₄ are each, independently, an internucleoside linking group linking the tetrahydropyran nucleoside analog to the antisense compound or one of T₃ and T₄ is an internucleoside linking group linking the tetrahydropyran nucleoside analog to the antisense compound and the other of T₃ and T₄ is H, a hydroxyl protecting group, a linked conjugate group, or a 5' or 3'-terminal group; q₁, q₂, q₃, q₄, q₅, q₆ and q₇ are each, independently, H, C₁-C₆ alkyl, substituted C₁-C₆ alkyl, C₂-C₆ alkenyl, substituted C₂-C₆ alkenyl, C₂-C₆ alkynyl, or substituted C₂-C₆ alkynyl; and
each of R₁ and R₂ is independently selected from among: hydrogen, halogen, substituted or unsubstituted alkoxy, NJ₁J₂, SJ₁, N₃, OC(=X)J₁, OC(=X)NJ₁J₂, NJ₃C(=X)NJ₁J₂, and CN, wherein X is O, S or NJ₁, and each J₁, J₂, and J₃ is, independently, H or C₁-C₆ alkyl.

In certain embodiments, modified nucleosides are modified THP nucleosides of Formula VII wherein q₁, q₂, q₃, q₄, q₅, q₆ and q₇ are each H. In certain embodiments, at least one of q₁, q₂, q₃, q₄, q₅, q₆ and q₇ is other than H. In certain embodiments, at least one of q₁, q₂, q₃, q₄, q₅, q₆ and q₇ is methyl. In certain embodiments, modified nucleosides are THP nucleosides of Formula VII wherein one of R₁ and R₂ is F. In certain embodiments, R₁ is fluoro and R₂ is H, R₁ is methoxy and R₂ is H, and R₁ is methoxyethoxy and R₂ is H.

Many other bicyclic and tricyclic sugar and sugar surrogate ring systems are known in the art that can be used to modify nucleosides *(see, e.g.,* review article: Leumann, J. C, Bioorganic & Medicinal Chemistry, 2002, 10, 841-854).

In certain embodiments, sugar surrogates comprise rings having more than 5 atoms and more than one heteroatom. For example nucleosides comprising morpholino sugar moieties and their use in oligomeric compounds has been reported (see for example: Braasch et al., Biochemistry, 2002, 41, 4503-4510; and U.S. Patents 5,698,685; 5,166,315; 5,185,444; and 5,034,506). As used here, the term "morpholino" means a sugar surrogate having the following structure: 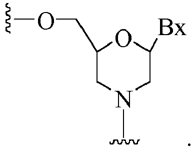 In certain embodiments, morpholinos may be modified, for example by adding or altering various substituent groups from the above morpholino structure. Such sugar surrogates are refered to herein as "modifed morpholinos."

Combinations of modifications are also disclosed without limitation, such as 2'-F-5'-methyl substituted nucleosides (see PCT International Application WO 2008/101157 Published on 8/21/08 for other disclosed 5', 2'-bis substituted nucleosides) and replacement of the ribosyl ring oxygen atom with S and further substitution at the 2'-position (see published U.S. Patent Application US2005-0130923, published on June 16, 2005) or alternatively 5'-substitution of a bicyclic nucleic acid (see PCT International Application WO 2007/134181, published on 11/22/07 wherein a 4'-CH₂-O-2' bicyclic nucleoside is further substituted at the 5' position with a 5'-methyl or a 5'-vinyl group). The synthesis and preparation of carbocyclic bicyclic nucleosides along with their oligomerization and biochemical studies have also been described *(see, e.g.,* Srivastava et al., J. Am. Chem. Soc. 2007, 129(26), 8362-8379).

### Certain Nucleobases

In certain embodiments, nucleosides of use in the present invention comprise one or more unmodified nucleobases. In certain embodiments, nucleosides of use in the present invention comprise one or more modifed nucleobases.

In certain embodiments, modified nucleobases are selected from: universal bases, hydrophobic bases, promiscuous bases, size-expanded bases, and fluorinated bases as defined herein. 5-substituted pyrimidines, 6-azapyrimidines and N-2, N-6 and O-6 substituted purines, including 2-aminopropyladenine, 5-propynyluracil; 5-propynylcytosine; 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine, 3-deazaguanine and 3-deazaadenine, universal bases, hydrophobic bases, promiscuous bases, size-expanded bases, and fluorinated bases as defined herein. Further modified nucleobases include tricyclic pyrimidines such as phenoxazine cytidine([5,4-b][1,4]benzoxazin-2(3H)-one), phenothiazine cytidine (1H-pyrimido[5,4-b][1,4]benzothiazin-2(3H)-one), G-clamps such as a substituted phenoxazine cytidine (e.g. 9-(2-aminoethoxy)-H-pyrimido[5,4-b][1,4]benzoxazin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in United States Patent No. 3,687,808, those disclosed in The Concise Encyclopedia Of Polymer Science And Engineering, Kroschwitz, J.I., Ed., John Wiley & Sons, 1990, 858-859; those disclosed by Englisch et al., Angewandte Chemie, International Edition, 1991, 30, 613; and those disclosed by Sanghvi, Y.S., Chapter 15, Antisense Research and Applications, Crooke, S.T. and Lebleu, B., Eds., CRC Press, 1993, 273-288.

Representative United States patents that teach the preparation of certain of the above noted modified nucleobases as well as other modified nucleobases include without limitation, U.S. 3,687,808; 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121; 5,596,091; 5,614,617; 5,645,985; 5,681,941; 5,750,692; 5,763,588; 5,830,653 and 6,005,096, certain of which are commonly owned with the instant application.

### Certain Internucleoside Linkages

The present invention relates to oligomeric compounds comprising linked nucleosides. In such embodiments, nucleosides may be linked together using any internucleoside linkage. The two main classes of internucleoside linking groups are defined by the presence or absence of a phosphorus atom. Representative phosphorus containing internucleoside linkages include, but are not limited to, phosphodiesters (P=O), phosphotriesters, methylphosphonates, phosphoramidate, and phosphorothioates (P=S). Representative non-phosphorus containing internucleoside linking groups include, but are not limited to, methylenemethylimino (-CH_{z}-N(CH₃)-O-CH₂-), thiodiester (-O-C(O)-S-), thionocarbamate (-O-C(O)(NH)-S-); siloxane (-O-Si(H)₂-O-); and N,N'-dimethylhydrazine (-CH₂-N(CH₃)-N(CH₃)-). Modified linkages, compared to natural phosphodiester linkages, can be used to alter, typically increase, nuclease resistance of the oligomeric compound. In certain embodiments, internucleoside linkages having a chiral atom can be prepared as a racemic mixture, or as separate enantiomers. Representative chiral linkages include, but are not limited to, alkylphosphonates and phosphorothioates. Methods of preparation of phosphorous-containing and non-phosphorous-containing internucleoside linkages are well known to those skilled in the art.

The oligonucleotides described herein contain one or more asymmetric centers and thus give rise to enantiomers, diastereomers, and other stereoisomeric configurations that may be defined, in terms of absolute stereochemistry, as (R) or (S), α or β such as for sugar anomers, or as (D) or (L) such as for amino acids etc. Included in the antisense compounds disclosed herein are all such possible isomers, as well as their racemic and optically pure forms.

Neutral internucleoside linkages include without limitation, phosphotriesters, methylphosphonates, MMI (3'-CH₂-N(CH₃)-O-5'), amide-3 (3'-CH₂-C(=O)-N(H)-5'), amide-4 (3'-CH₂-N(H)-C(=O)-5'), formacetal (3'-O-CH₂-O-5'), and thioformacetal (3'-S-CH₂-O-5'). Further neutral internucleoside linkages include nonionic linkages comprising siloxane (dialkylsiloxane), carboxylate ester, carboxamide, sulfide, sulfonate ester and amides (See for example: Carbohydrate Modifications in Antisense Research; Y.S. Sanghvi and P.D. Cook, Eds., ACS Symposium Series 580; Chapters 3 and 4, 40-65). Further neutral internucleoside linkages include nonionic linkages comprising mixed N, O, S and CH₂ component parts.

### Certain Motifs

The present invention relates to oligomeric compounds comprising oligonucleotides. In certain embodiments, such oligonucleotides comprise one or more chemical modification. In certain embodiments, chemically modified oligonucleotides comprise one or more modified nucleosides. In certain embodiments, chemically modified oligonucleotides comprise one or more modified nucleosides comprising modified sugars. In certain embodiments, chemically modified oligonucleotides comprise one or more modified nucleosides comprising one or more modified nucleobases. In certain embodiments, chemically modified oligonucleotides comprise one or more modified internucleoside linkages. In certain embodiments, the chemically modifications (sugar modifications, nucleobase modifications, and/or linkage modifications) define a pattern or motif. In certain embodiments, the patterns of chemical modifications of sugar moieties, internucleoside linkages, and nucleobases are each independent of one another. Thus, an oligonucleotide may be described by its sugar modification motif, internucleoside linkage motif and/or nucleobase modification motif (as used herein, nucleobase modification motif describes the chemical modifications to the nucleobases independent of the sequence of nucleobases).

### Certain sugar motifs

In certain embodiments, oligonucleotides comprise one or more type of modified sugar moieties and/or naturally occurring sugar moieties arranged along an oligonucleotide or region thereof in a defined pattern or sugar modification motif. Such motifs may include any of the sugar modifications discussed herein and/or other known sugar modifications.

In certain embodiments, the oligonucleotides comprise or consist of a region having a gapmer sugar modification motif, which comprises two external regions or "wings" and an internal region or "gap." The three regions of a gapmer motif (the 5'-wing, the gap, and the 3'-wing) form a contiguous sequence of nucleosides wherein at least some of the sugar moieties of the nucleosides of each of the wings differ from at least some of the sugar moieties of the nucleosides of the gap. Specifically, at least the sugar moieties of the nucleosides of each wing that are closest to the gap (the 3'-most nucleoside of the 5'-wing and the 5'-most nucleoside of the 3'-wing) differ from the sugar moiety of the neighboring gap nucleosides, thus defining the boundary between the wings and the gap. In certain embodiments, the sugar moieties within the gap are the same as one another. In certain embodiments, the gap includes one or more nucleoside having a sugar moiety that differs from the sugar moiety of one or more other nucleosides of the gap. In certain embodiments, the sugar modification motifs of the two wings are the same as one another (symmetric gapmer). In certain embodiments, the sugar modification motifs of the 5'-wing differs from the sugar modification motif of the 3'-wing (asymmetric gapmer). In certain embodiments, oligonucleotides comprise 2'-MOE modified nucleosides in the wings and 2'-F modified nucleosides in the gap.

In certain embodiments, oligonucleotides are fully modified. In certain such embodiments, oligonucleotides are uniformly modified. In certain embodiments, oligonucleotides are uniform 2'-MOE. In certain embodiments, oligonucleotides are uniform 2'-F. In certain embodiments, oligonucleotides are uniform morpholino. In certain embodiments, oligonucleotides are uniform BNA. In certain embodiments, oligonucleotides are uniform LNA. In certain embodiments, oligonucleotides are uniform cEt.

In certain embodiments, oligonucleotides comprise a uniformly modified region and additional nucleosides that are unmodified or differently modified. In certain embodiments, the uniformly modified region is at least 5, 10, 15, or 20 nucleosides in length. In certain embodiments, the uniform region is a 2'-MOE region. In certain embodiments, the uniform region is a 2'-F region. In certain embodiments, the uniform region is a morpholino region. In certain embodiments, the uniform region is a BNA region. In certain embodiments, the uniform region is a LNA region. In certain embodiments, the uniform region is a cEt region.

In certain embodiments, the oligonucleotide does not comprise more than 4 contiguous unmodified 2'-deoxynucleosides. In certain circumstances, antisesense oligonucleotides comprising more than 4 contiguous 2'-deoxynucleosides activate RNase H, resulting in cleavage of the target RNA. In certain embodiments, such cleavage is avoided by not having more than 4 contiguous 2'-deoxynucleosides, for example, where alteration of splicing and not cleavage of a target RNA is desired.

### Certain Internucleoside Linkage Motifs

In certain embodiments, oligonucleotides comprise modified internucleoside linkages arranged along the oligonucleotide or region thereof in a defined pattern or modified internucleoside linkage motif. In certain embodiments, internucleoside linkages are arranged in a gapped motif, as described above for sugar modification motif. In such embodiments, the internucleoside linkages in each of two wing regions are different from the internucleoside linkages in the gap region. In certain embodiments the internucleoside linkages in the wings are phosphodiester and the internucleoside linkages in the gap are phosphorothioate. The sugar modification motif is independently selected, so such oligonucleotides having a gapped internucleoside linkage motif may or may not have a gapped sugar modification motif and if it does have a gapped sugar motif, the wing and gap lengths may or may not be the same.

In certain embodiments, oligonucleotides comprise a region having an alternating internucleoside linkage motif. In certain embodiments, oligonucleotides of use in the present invention comprise a region of uniformly modified internucleoside linkages. In certain such embodiments, the oligonucleotide comprises a region that is uniformly linked by phosphorothioate internucleoside linkages. In certain embodiments, the oligonucleotide is uniformly linked by phosphorothioate. In certain embodiments, each internucleoside linkage of the oligonucleotide is selected from phosphodiester and phosphorothioate. In certain embodiments, each internucleoside linkage of the oligonucleotide is selected from phosphodiester and phosphorothioate and at least one internucleoside linkage is phosphorothioate.

In certain embodiments, the oligonucleotide comprises at least 6 phosphorothioate internucleoside linkages. In certain embodiments, the oligonucleotide comprises at least 8 phosphorothioate internucleoside linkages. In certain embodiments, the oligonucleotide comprises at least 10 phosphorothioate internucleoside linkages. In certain embodiments, the oligonucleotide comprises at least one block of at least 6 consecutive phosphorothioate internucleoside linkages. In certain embodiments, the oligonucleotide comprises at least one block of at least 8 consecutive phosphorothioate internucleoside linkages. In certain embodiments, the oligonucleotide comprises at least one block of at least 10 consecutive phosphorothioate internucleoside linkages. In certain embodiments, the oligonucleotide comprises at least block of at least one 12 consecutive phosphorothioate internucleoside linkages. In certain such embodiments, at least one such block is located at the 3' end of the oligonucleotide. In certain such embodiments, at least one such block is located within 3 nucleosides of the 3' end of the oligonucleotide.

### Certain Nucleobase Modification Motifs

In certain embodiments, oligonucleotides comprise chemical modifications to nucleobases arranged along the oligonucleotide or region thereof in a defined pattern or nucleobases modification motif. In certain such embodiments, nucleobase modifications are arranged in a gapped motif. In certain embodiments, nucleobase modifications are arranged in an alternating motif. In certain embodiments, each nucleobase is modified. In certain embodiments, none of the nucleobases is chemically modified.

In certain embodiments, oligonucleotides comprise a block of modified nucleobases. In certain such embodiments, the block is at the 3'-end of the oligonucleotide. In certain embodiments the block is within 3 nucleotides of the 3'-end of the oligonucleotide. In certain such embodiments, the block is at the 5'-end of the oligonucleotide. In certain embodiments the block is within 3 nucleotides of the 5'-end of the oligonucleotide.

In certain embodiments, nucleobase modifications are a function of the natural base at a particular position of an oligonucleotide. For example, in certain embodiments each purine or each pyrimidine in an oligonucleotide is modified. In certain embodiments, each adenine is modified. In certain embodiments, each guanine is modified. In certain embodiments, each thymine is modified. In certain embodiments, each cytosine is modified. In certain embodiments, each uracil is modified.

In certain embodiments, some, all, or none of the cytosine moieties in an oligonucleotide are 5-methyl cytosine moieties. Herein, 5-methyl cytosine is not a "modified nucleobase." Accordingly, unless otherwise indicated, unmodified nucleobases include both cytosine residues having a 5 -methyl and those lacking a 5 methyl. In certain embodiments, the methylation state of all or some cytosine nucleobases is specified.

### Certain Overall Lengths

The invention relates to modified oligonucleotides consisting of 8 to 30 linked nucleosides. In certain embodiments, the present disclosure relates to oligomeric compounds including oligonucleotides of any of a variety of ranges of lengths. In certain embodiments, the disclosure relates to oligomeric compounds or oligonucleotides consisting of X to Y linked nucleosides, where X represents the fewest number of nucleosides in the range and Y represents the largest number of nucleosides in the range. In certain such embodiments, X and Y are each independently selected from 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39,40, 41, 42, 43, 44, 45, 46, 47, 48, 49, and 50; provided that X≤Y. For example, in certain embodiments, the disclosure relates to oligomeric compounds which comprise oligonucleotides consisting of 8 to 9, 8 to 10, 8 to 11, 8 to 12, 8 to 13, 8 to 14, 8 to 15, 8 to 16, 8 to 17, 8 to 18, 8 to 19, 8 to 20, 8 to 21, 8 to 22, 8 to 23, 8 to 24, 8 to 25, 8 to 26, 8 to 27, 8 to 28, 8 to 29, 8 to 30, 9 to 10, 9 to 11, 9 to 12, 9 to 13, 9 to 14, 9 to 15, 9 to 16, 9 to 17, 9 to 18, 9 to 19, 9 to 20, 9 to 21, 9 to 22, 9 to 23, 9 to 24, 9 to 25, 9 to 26, 9 to 27, 9 to 28, 9 to 29, 9 to 30, 10 to 11, 10 to 12, 10 to 13, 10 to 14, 10 to 15, 10 to 16, 10 to 17, 10 to 18, 10 to 19, 10 to 20, 10 to 21, 10 to 22, 10 to 23, 10 to 24, 10 to 25, 10 to 26, 10 to 27, 10 to 28, 10 to 29, 10 to 30, 11 to 12, 11 to 13, 11 to 14, 11 to 15, 11 to 16, 11 to 17, 11 to 18, 11 to 19, 11 to 20, 11 to 21, 11 to 22, 11 to 23, 11 to 24, 11 to 25, 11 to 26, 11 to 27, 11 to 28, 11 to 29, 11 to 30, 12 to 13, 12 to 14, 12 to 15, 12 to 16, 12 to 17, 12 to 18, 12 to 19, 12 to 20, 12 to 21, 12 to 22, 12 to 23, 12 to 24, 12 to 25, 12 to 26, 12 to 27, 12 to 28, 12 to 29, 12 to 30, 13 to 14, 13 to 15, 13 to 16, 13 to 17, 13 to 18, 13 to 19, 13 to 20, 13 to 21, 13 to 22, 13 to 23, 13 to 24, 13 to 25, 13 to 26, 13 to 27, 13 to 28, 13 to 29, 13 to 30, 14 to 15, 14 to 16, 14 to 17, 14 to 18, 14 to 19, 14 to 20, 14 to 21, 14 to 22, 14 to 23, 14 to 24, 14 to 25, 14 to 26, 14 to 27, 14 to 28, 14 to 29, 14 to 30, 15 to 16, 15 to 17, 15 to 18, 15 to 19, 15 to 20, 15 to 21, 15 to 22, 15 to 23, 15 to 24, 15 to 25, 15 to 26, 15 to 27, 15 to 28, 15 to 29, 15 to 30, 16 to 17, 16 to 18, 16 to 19, 16 to 20, 16 to 21, 16 to 22, 16 to 23, 16 to 24, 16 to 25, 16 to 26, 16 to 27, 16 to 28, 16 to 29, 16 to 30, 17 to 18, 17 to 19, 17 to 20, 17 to 21, 17 to 22, 17 to 23, 17 to 24, 17 to 25, 17 to 26, 17 to 27, 17 to 28, 17 to 29, 17 to 30, 18 to 19, 18 to 20, 18 to 21, 18 to 22, 18 to 23, 18 to 24, 18 to 25, 18 to 26, 18 to 27, 18 to 28, 18 to 29, 18 to 30, 19 to 20, 19 to 21, 19 to 22, 19 to 23, 19 to 24, 19 to 25, 19 to 26, 19 to 29, 19 to 28, 19 to 29, 19 to 30, 20 to 21, 20 to 22, 20 to 23, 20 to 24, 20 to 25, 20 to 26, 20 to 27, 20 to 28, 20 to 29, 20 to 30, 21 to 22, 21 to 23, 21 to 24, 21 to 25, 21 to 26, 21 to 27, 21 to 28, 21 to 29, 21 to 30, 22 to 23, 22 to 24, 22 to 25, 22 to 26, 22 to 27, 22 to 28, 22 to 29, 22 to 30, 23 to 24, 23 to 25, 23 to 26, 23 to 27, 23 to 28, 23 to 29, 23 to 30, 24 to 25, 24 to 26, 24 to 27, 24 to 28, 24 to 29, 24 to 30, 25 to 26, 25 to 27, 25 to 28, 25 to 29, 25 to 30, 26 to 27, 26 to 28, 26 to 29, 26 to 30, 27 to 28, 27 to 29, 27 to 30, 28 to 29, 28 to 30, or 29 to 30 linked nucleosides. In embodiments where the number of nucleosides of an oligomeric compound or oligonucleotide is limited, whether to a range or to a specific number, the oligomeric compound or oligonucleotide may, nonetheless further comprise additional other substituents. For example, an oligonucleotide comprising 8-30 nucleosides excludes oligonucleotides having 31 nucleosides, but, unless otherwise indicated, such an oligonucleotide may further comprise, for example one or more conjugates, terminal groups, or other substituents. In certain embodiments, a gapmer oligonucleotide has any of the above lengths.

One of skill in the art will appreciate that certain lengths may not be possible for certain motifs. For example: a gapmer having a 5'-wing region consisting of four nucleotides, a gap consisting of at least six nucleotides, and a 3'-wing region consisting of three nucleotides cannot have an overall length less than 13 nucleotides. Thus, one would understand that the lower length limit is 13 and that the limit of 10 in "10-20" has no effect in that embodiment.

Further, where an oligonucleotide is described by an overall length range and by regions having specified lengths, and where the sum of specified lengths of the regions is less than the upper limit of the overall length range, the oligonucleotide may have additional nucleosides, beyond those of the specified regions, provided that the total number of nucleosides does not exceed the upper limit of the overall length range. For example, an oligonucleotide consisting of 20-25 linked nucleosides comprising a 5'-wing consisting of 5 linked nucleosides; a 3'-wing consisting of 5 linked nucleosides and a central gap consisting of 10 linked nucleosides (5+5+10=20) may have up to 5 nucleosides that are not part of the 5'-wing, the 3'-wing, or the gap (before reaching the overall length limitation of 25). Such additional nucleosides may be 5' of the 5'-wing and/or 3' of the 3' wing.

### Certain Oligonucleotides

In certain embodiments, oligonucleotides of use in the present invention are characterized by their sugar motif, internucleoside linkage motif, nucleobase modification motif and overall length. In certain embodiments, such parameters are each independent of one another. Thus, each internucleoside linkage of an oligonucleotide having a gapmer sugar motif may be modified or unmodified and may or may not follow the gapmer modification pattern of the sugar modifications. Thus, the internucleoside linkages within the wing regions of a sugar-gapmer may be the same or different from one another and may be the same or different from the internucleoside linkages of the gap region. Likewise, such sugar-gapmer oligonucleotides may comprise one or more modified nucleobase independent of the gapmer pattern of the sugar modifications. Herein if a description of an oligonucleotide or oligomeric compound is silent with respect to one or more parameter, such parameter is not limited. Thus, an oligomeric compound described only as having a gapmer sugar motif without further description may have any length, internucleoside linkage motif, and nucleobase modification motif. Unless otherwise indicated, all chemical modifications are independent of nucleobase sequence.

### Certain Conjugate Groups

In certain embodiments, oligomeric compounds are modified by attachment of one or more conjugate groups. In general, conjugate groups modify one or more properties of the attached oligomeric compound including but not limited to pharmacodynamics, pharmacokinetics, stability, binding, absorption, cellular distribution, cellular uptake, charge and clearance. Conjugate groups are routinely used in the chemical arts and are linked directly or via an optional conjugate linking moiety or conjugate linking group to a parent compound such as an oligomeric compound, such as an oligonucleotide. Conjugate groups includes without limitation, intercalators, reporter molecules, polyamines, polyamides, polyethylene glycols, thioethers, polyethers, cholesterols, thiocholesterols, cholic acid moieties, folate, lipids, phospholipids, biotin, phenazine, phenanthridine, anthraquinone, adamantane, acridine, fluoresceins, rhodamines, coumarins and dyes. Certain conjugate groups have been described previously, for example: cholesterol moiety (Letsinger et al., Proc. Natl. Acad. Sci. USA, 1989, 86, 6553-6556), cholic acid (Manoharan et al., Bioorg. Med. Chem. Let., 1994, 4, 1053-1060), a thioether, e.g., hexyl-S-tritylthiol (Manoharan et al., Ann. N.Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, e.g., do-decan-diol or undecyl residues (Saison-Behmoaras et al., EMBO J., 1991, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethyl-ammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 3651-3654), a palmityl moiety (Mishra et al., Biochim. Biophys. Acta, 1995, 1264, 229-237), or an octadecylamine or hexylamino-carbonyl-oxycholesterol moiety (Crooke et al., J. Pharmacol. Exp. Ther., 1996, 277, 923-937).

In certain embodiments, a conjugate group comprises an active drug substance, for example, aspirin, warfarin, phenylbutazone, ibuprofen, suprofen, fen-bufen, ketoprofen, (S)-(+)-pranoprofen, carprofen, dansylsarcosine, 2,3,5-triiodobenzoic acid, flufenamic acid, folinic acid, a benzothiadiazide, chlorothiazide, a diazepine, indo-methicin, a barbiturate, a cephalosporin, a sulfa drug, an antidiabetic, an antibacterial or an antibiotic.

In certain embodiments, conjugate groups are directly attached to oligonucleotides in oligomeric compounds. In certain embodiments, conjugate groups are attached to oligonucleotides by a conjugate linking group. In certain such embodiments, conjugate linking groups, including, but not limited to, bifunctional linking moieties such as those known in the art are amenable to the compounds disclosed herein. Conjugate linking groups are useful for attachment of conjugate groups, such as chemical stabilizing groups, functional groups, reporter groups and other groups to selective sites in a parent compound such as for example an oligomeric compound. In general a bifunctional linking moiety comprises a hydrocarbyl moiety having two functional groups. One of the functional groups is selected to bind to a parent molecule or compound of interest and the other is selected to bind essentially any selected group such as chemical functional group or a conjugate group. In some embodiments, the conjugate linker comprises a chain structure or an oligomer of repeating units such as ethylene glycol or amino acid units. Examples of functional groups that are routinely used in a bifunctional linking moiety include, but are not limited to, electrophiles for reacting with nucleophilic groups and nucleophiles for reacting with electrophilic groups. In some embodiments, bifunctional linking moieties include amino, hydroxyl, carboxylic acid, thiol, unsaturations (e.g., double or triple bonds), and the like.

Some nonlimiting examples of conjugate linking moieties include pyrrolidine, 8-amino-3,6-dioxaoctanoic acid (ADO), succinimidyl 4-(N-maleimidomethyl) cyclohexane-1-carboxylate (SMCC) and 6-aminohexanoic acid (AHEX or AHA). Other linking groups include, but are not limited to, substituted C₁-C₁₀ alkyl, substituted or unsubstituted C₂-C₁₀ alkenyl or substituted or unsubstituted C₂-C₁₀ alkynyl, wherein a nonlimiting list of preferred substituent groups includes hydroxyl, amino, alkoxy, carboxy, benzyl, phenyl, nitro, thiol, thioalkoxy, halogen, alkyl, aryl, alkenyl and alkynyl.

Conjugate groups may be attached to either or both ends of an oligonucleotide (terminal conjugate groups) and/or at any internal position.

In certain embodiments, conjugate groups are at the 3'-end of an oligonucleotide of an oligomeric compound. In certain embodiments, conjugate groups are near the 3'-end. In certain embodiments, conjugates are attached at the 3'end of an oligomeric compound, but before one or more terminal group nucleosides. In certain embodiments, conjugate groups are placed within a terminal group.
In certain embodiments, the present invention relates to oligomeric compounds. In certain embodiments, oligomeric compounds comprise an oligonucleotide. In certain embodiments, an oligomeric compound comprises an oligonucleotide and one or more conjugate and/or terminal groups. Such conjugate and/or terminal groups may be added to oligonucleotides having any of the chemical motifs discussed above. Thus, for example, an oligomeric compound comprising an oligonucleotide having region of alternating nucleosides may comprise a terminal group.

### Antisense Compounds

In certain embodiments, oligomeric compounds of the present disclosure are antisense compounds. Such antisense compounds are capable of hybridizing to a target nucleic acid, resulting in at least one antisense activity. In certain embodiments, antisense compounds specifically hybridize to one or more target nucleic acid. In certain embodiments, a specifically hybridizing antisense compound has a nucleobase sequence comprising a region having sufficient complementarity to a target nucleic acid to allow hybridization and result in antisense activity and insufficient complementarity to any non-target so as to avoid non-specific hybridization to any non-target nucleic acid sequences under conditions in which specific hybridization is desired (e.g., under physiological conditions for *in vivo* or therapeutic uses, and under conditions in which assays are performed in the case of *in vitro* assays).

The oligonucleotides of use in the invention are at least 90% complementary to the target nucleic acid. In certain embodiments, the present invention relates to antisense compounds comprising oligonucleotides that are fully complementary to the target nucleic acid over the entire length of the oligonucleotide. In certain embodiments of the invention, oligonucleotides are 99% complementary to the target nucleic acid. In certain embodiments of the invention, oligonucleotides are 95% complementary to the target nucleic acid.

In certain embodiments of the disclosure, such oligonucleotides are 85% complementary to the target nucleic acid. In certain embodiments of the disclosure, such oligonucleotides are 80% complementary to the target nucleic acid. In certain embodiments of the disclosure, an antisense compound comprises a region that is fully complementary to a target nucleic acid and is at least 80% complementary to the target nucleic acid over the entire length of the oligonucleotide. In certain such embodiments, the region of full complementarity is from 6 to 14 nucleobases in length.

In certain embodiments antisense compounds and antisense oligonucleotides comprise single-strand compounds. In certain embodiments antisense compounds and antisense oligonucleotides comprise double-strand compounds.

### Certain Pathways and Mechanisms Associated With Neuronal ceroid lipofuscinoses

Neuronal ceroid lipofuscinoses (NCL) is the general name for a family of neurodegenerative disorders that result from excessive accumulation of lipopigments, e.g. lipofuscin, in the body's tissues. Juvenile neuronal ceroid lipofuscinosis (JNCL), also known as Batten Disease, is the most common of the NCL disorders. In certain embodiments, JNCL onset occurs between five and eight years of age and symptoms include progressive loss of motor function, seizures, vision loss, and loss of cognitive function, resulting in death before age 30.

JNCL is an autosomal recessive disorder caused by mutations of the CLN3 gene. Approximately 80% of JNCL cases result from a particular deletion of the CLN3 gene spanning exons 7 and 8 (*CLN3Δ78*). In certain embodiments, the *CLN3Δ78* deletion causes a frameshift that results in a premature stop codon in exon 9. In certain embodiments, the truncated protein product of *CLN3Δ78* is 33% of the length of the wild type CLN3 protein. In certain embodiments, the truncated protein product of *CLN3Δ78* is non-functional. In certain embodiments, the truncated protein product of *CLN3Δ78* is partially functional.

In certain embodiments, antisense oligonucleotides complementary to the mutant *CLN3Δ78* transcript eliminate the frameshift that results in a premature stop codon in exon 9. In certain embodiments, for example, antisense oligonucleotides complementary to the mutant *CLN3Δ78* transcript produce *CLN3Δ78* mRNA having exons downstream of exon 9. For example, in certain embodiments, antisense oligonucleotides complementary to the mutant *CLN3Δ78* transcript produce *CLN3Δ78* mRNA having exons 10 and/or 11. In certain embodiments, for example, antisense oligonucleotides complementary to the mutant *CLN3Δ78* transcript produce a partially deleted , but still functional *CLN3Δ78* protein.

In certain embodiments, antisense oligonucleotides complementary to exon 6 of a *CLN3Δ78* transcript induces skipping of exon 6 and eliminates the frameshift in *CLN3Δ78* pre-mRNA that results in a premature stop codon in exon 9. In such embodiments, oligonucleotides complementary to exon 6 of a *CLN3Δ78* transcript produce *CLN3Δ78* mRNA without exon 6, but having the remaining exons (e.g. exons 1, 2, 3, 4, 5, 9, 10, 11...). In certain embodiments, *CLN3Δ78* mRNA having the remaining exons is translated into a *CLN3Δ78* protein that is partially functional. In certain embodiments, *CLN3Δ78* mRNA having the remaining exons is translated into a *CLN3Δ78* protein that functions in a similar manner as wild type CLN3 protein. In certain embodiments, *CLN3Δ78* mRNA having the remaining exons is translated into a *CLN3Δ78* protein that functions better than the truncated protein product of *CLN3Δ78* containing the premature stop codon. In certain embodiments, *CLN3Δ78* mRNA having the remaining exons is translated into a *CLN3Δ78* protein that does not produce the excessive accumulation of lipopigments in the body tissue.

In certain embodiments, antisense oligonucleotides complementary to exon 9 of a *CLN3Δ78* transcript induce skipping of exon 9 and eliminate the frameshift in *CLN3Δ78* pre-mRNA that results in a premature stop codon in exon 9. In such embodiments, oligonucleotides complementary to exon 9 of a *CLN3Δ78* transcript produce *CLN3Δ78* mRNA without exon 9, but having the remaining exons (e.g. exons 1, 2, 3, 4, 5, 6, 10, 11...). In certain embodiments, *CLN3Δ78* mRNA having the remaining exons, e.g. exons 1, 2, 3, 4, 5, 6, 10, 11..., is translated into a *CLN3Δ78* protein that is partially functional. In certain embodiments, *CLN3Δ78* mRNA missing exons 7, 8, and 9 is translated into a *CLN3Δ78* protein that functions in a similar manner as wild type CLN3 protein. In certain embodiments, *CLN3Δ78* mRNA missing exons 7, 8, and 9 is translated into a *CLN3Δ78* protein that functions better than the truncated protein product of *CLNΔ78* containing the premature stop codon. In certain embodiments, *CLN3Δ78* mRNA missing exons 7, 8, and 9 is translated into a *CLN3Δ78* protein that does not produce the excessive accumulation of lipopigments in the body tissue.

### Certain Target Nucleic Acids and Mechanisms

In certain embodiments, antisense compounds comprise or consist of an oligonucleotide comprising a region that is complementary to a target nucleic acid. In certain embodiments, the target nucleic acid is an endogenous RNA molecule. In certain embodiments, the target nucleic acid is a pre-mRNA. In certain embodiments, the target nucleic acid is a CLN3 transcript. In certain embodiments, the target RNA is a CLN3 pre-mRNA.

In certain embodiments, an antisense compound is complementary to a region of CLN3 pre-mRNA. In certain embodiments, an antisense compound is complementary to a region of CLN3 pre-mRNA comprising an intron-exon splice junction. In certain embodiments, an antisense compound is complementary to a region of CLN3 pre-mRNA comprising the intron-exon splice junction adjacent to exon 6. In certain embodiments, an antisense compound is complementary within a region of CLN3 pre-mRNA consisting of exon 6. In certain embodiments, an antisense compound is complementary within a region of CLN3 pre-mRNA comprising an exonic splicing silencer within an exon 6. In certain embodiments, an antisense compound is complementary to a region of CLN3 pre-mRNA comprising the intron-exon splice junction adjacent to exon 9. In certain embodiments, an antisense compound is complementary within a region of CLN3 pre-mRNA consisting of exon 9. In certain embodiments, an antisense compound is complementary within a region of CLN3 pre-mRNA comprising an exonic splicing silencer within an exon 9.

In certain embodiments, an antisense compound comprises a modified oligonucleotide consisting of 8 to 30 linked nucleosides and having a nucleobase sequence comprising a complementary region comprising at least 8 contiguous nucleobases complementary to a target region of equal length of a CLN3 transcript. In certain embodiments, the target region is within nucleobase 5082 and nucleobase 5119 of SEQ ID NO.: 2. In certain embodiments, the target region is within nucleobase 5082 and nucleobase 5099 of SEQ ID NO.: 2. In certain embodiments, the target region is within nucleobase 5053 and nucleobase 5070 of SEQ ID NO.: 2. In certain embodiments, the target region is within nucleobase 5086 and nucleobase 5103 of SEQ ID NO.: 2. In certain embodiments, the target region is within nucleobase 5090 and nucleobase 5107 of SEQ ID NO.: 2. In certain embodiments, the target region is within nucleobase 5094 and nucleobase 5111 of SEQ ID NO.: 2. In certain embodiments, the target region is within nucleobase 5098 and nucleobase 5115 of SEQ ID NO.: 2. In certain embodiments, the target region is within nucleobase 5102 and nucleobase 5119 of SEQ ID NO.: 2. In certain embodiments, the target region is within nucleobase 5126 and nucleobase 5143 of SEQ ID NO.: 2. In certain embodiments, the target region is within nucleobase 5134 and nucleobase 5155 of SEQ ID NO.: 2. In certain embodiments, the target region is within nucleobase 5134 and nucleobase 5151 of SEQ ID NO.: 2. In certain embodiments, the target region is within nucleobase 5138 and nucleobase 5155 of SEQ ID NO.: 2.

In certain embodiments, an antisense compound comprises a modified oligonucleotide consisting of 8 to 30 linked nucleosides and having a nucleobase sequence comprising a complementary region comprising at least 8 contiguous nucleobases complementary to a target region of equal length of a CLN3 transcript. In certain embodiments, the target region is within nucleobase 7366 and nucleobase 7411 of SEQ ID NO.: 2. In certain embodiments, the target region is within nucleobase 7366 and nucleobase 7383 of SEQ ID NO.: 2. In certain embodiments, the target region is within nucleobase 7370 and nucleobase 7387 of SEQ ID NO.: 2. In certain embodiments, the target region is within nucleobase 7371 and nucleobase 7388 of SEQ ID NO.: 2. In certain embodiments, the target region is within nucleobase 7387 and nucleobase 7404 of SEQ ID NO.: 2. In certain embodiments, the target region is within nucleobase 7394 and nucleobase 7411 of SEQ ID NO.: 2. In certain embodiments, the target region is within nucleobase 7454 and nucleobase 7471 of SEQ ID NO.: 2. In certain embodiments, the target region is within nucleobase 7462 and nucleobase 7483 of SEQ ID NO.: 2. In certain embodiments, the target region is within nucleobase 7462 and nucleobase 7479 of SEQ ID NO.: 2. In certain embodiments, the target region is within nucleobase 7466 and nucleobase 7483 of SEQ ID NO.: 2.

In certain embodiments, an antisense compound has a nucleobase sequence comprising SEQ ID NO. 3. In certain embodiments, an antisense compound has a nucleobase sequence comprising SEQ ID NO. 4. In certain embodiments, an antisense compound has a nucleobase sequence comprising SEQ ID NO. 5. In certain embodiments, an antisense compound has a nucleobase sequence comprising SEQ ID NO. 6. In certain embodiments, an antisense compound has a nucleobase sequence comprising SEQ ID NO. 7. In certain embodiments, an antisense compound has a nucleobase sequence comprising SEQ ID NO. 8. In certain embodiments, an antisense compound has a nucleobase sequence comprising SEQ ID NO. 9. In certain embodiments, an antisense compound has a nucleobase sequence comprising SEQ ID NO. 10. In certain embodiments, an antisense compound has a nucleobase sequence comprising SEQ ID NO. 11. In certain embodiments, an antisense compound has a nucleobase sequence comprising SEQ ID NO. 12. In certain embodiments, an antisense compound has a nucleobase sequence comprising SEQ ID NO. 13. In certain embodiments, an antisense compound has a nucleobase sequence comprising SEQ ID NO. 14. In certain embodiments, an antisense compound has a nucleobase sequence comprising SEQ ID NO. 15. In certain embodiments, an antisense compound has a nucleobase sequence comprising SEQ ID NO. 16. In certain embodiments, an antisense compound has a nucleobase sequence comprising SEQ ID NO. 17.

In certain embodiments, an antisense compound has a nucleobase sequence comprising SEQ ID NO. 18. In certain embodiments, an antisense compound has a nucleobase sequence comprising SEQ ID NO. 19. In certain embodiments, an antisense compound has a nucleobase sequence comprising SEQ ID NO. 20. In certain embodiments, an antisense compound has a nucleobase sequence comprising SEQ ID NO. 21. In certain embodiments, an antisense compound has a nucleobase sequence comprising SEQ ID NO. 22. In certain embodiments, an antisense compound has a nucleobase sequence comprising SEQ ID NO. 23. In certain embodiments, an antisense compound has a nucleobase sequence comprising SEQ ID NO. 24. In certain embodiments, an antisense compound has a nucleobase sequence comprising SEQ ID NO. 25. In certain embodiments, an antisense compound has a nucleobase sequence comprising SEQ ID NO. 26. In certain embodiments, an antisense compound has a nucleobase sequence comprising SEQ ID NO. 27. In certain embodiments, an antisense compound has a nucleobase sequence comprising SEQ ID NO. 28. In certain embodiments, an antisense compound has a nucleobase sequence comprising SEQ ID NO. 29. In certain embodiments, an antisense compound has a nucleobase sequence comprising SEQ ID NO. 30. In certain embodiments, an antisense compound has a nucleobase sequence comprising SEQ ID NO. 31. In certain embodiments, an antisense compound has a nucleobase sequence comprising SEQ ID NO. 32.

In certain embodiments, an antisense compound has a nucleobase sequence comprising SEQ ID NO. 33. In certain embodiments, an antisense compound has a nucleobase sequence comprising SEQ ID NO.3 4. In certain embodiments, an antisense compound has a nucleobase sequence comprising SEQ ID NO. 35. In certain embodiments, an antisense compound has a nucleobase sequence comprising SEQ ID NO. 36. In certain embodiments, an antisense compound has a nucleobase sequence comprising SEQ ID NO. 37. In certain embodiments, an antisense compound has a nucleobase sequence comprising SEQ ID NO. 38. In certain embodiments, an antisense compound has a nucleobase sequence comprising SEQ ID NO. 39. In certain embodiments, an antisense compound has a nucleobase sequence comprising SEQ ID NO. 40. In certain embodiments, an antisense compound has a nucleobase sequence comprising SEQ ID NO. 41. In certain embodiments, an antisense compound has a nucleobase sequence comprising SEQ ID NO. 42. In certain embodiments, an antisense compound has a nucleobase sequence comprising SEQ ID NO. 43. In certain embodiments, an antisense compound has a nucleobase sequence comprising SEQ ID NO. 44. In certain embodiments, an antisense compound has a nucleobase sequence comprising SEQ ID NO. 45. In certain embodiments, an antisense compound has a nucleobase sequence comprising SEQ ID NO. 46. In certain embodiments, an antisense compound has a nucleobase sequence comprising SEQ ID NO. 47.

In certain embodiments, an antisense compound has a nucleobase sequence comprising SEQ ID NO. 48. In certain embodiments, an antisense compound has a nucleobase sequence comprising SEQ ID NO. 49. In certain embodiments, an antisense compound has a nucleobase sequence comprising SEQ ID NO. 50. In certain embodiments, an antisense compound has a nucleobase sequence comprising SEQ ID NO. 51. In certain embodiments, an antisense compound has a nucleobase sequence comprising SEQ ID NO. 52. In certain embodiments, an antisense compound has a nucleobase sequence comprising SEQ ID NO. 53. In certain embodiments, an antisense compound has a nucleobase sequence comprising SEQ ID NO. 54. In certain embodiments, an antisense compound has a nucleobase sequence comprising SEQ ID NO. 55. In certain embodiments, an antisense compound has a nucleobase sequence comprising SEQ ID NO. 56. In certain embodiments, an antisense compound has a nucleobase sequence comprising SEQ ID NO. 57. In certain embodiments, an antisense compound has a nucleobase sequence comprising SEQ ID NO. 58. In certain embodiments, an antisense compound has a nucleobase sequence comprising SEQ ID NO. 59. In certain embodiments, an antisense compound has a nucleobase sequence comprising SEQ ID NO. 60.

In certain embodiments, an antisense compound has a nucleobase sequence consisting of SEQ ID NO. 3. In certain embodiments, an antisense compound has a nucleobase sequence consisting of SEQ ID NO. 4. In certain embodiments, an antisense compound has a nucleobase sequence consisting of SEQ ID NO. 5. In certain embodiments, an antisense compound has a nucleobase sequence consisting of SEQ ID NO. 6. In certain embodiments, an antisense compound has a nucleobase sequence consisting of SEQ ID NO. 7. In certain embodiments, an antisense compound has a nucleobase sequence consisting of SEQ ID NO. 8. In certain embodiments, an antisense compound has a nucleobase sequence consisting of SEQ ID NO. 9. In certain embodiments, an antisense compound has a nucleobase sequence consisting of SEQ ID NO. 10. In certain embodiments, an antisense compound has a nucleobase sequence consisting of SEQ ID NO. 11. In certain embodiments, an antisense compound has a nucleobase sequence consisting of SEQ ID NO. 12. In certain embodiments, an antisense compound has a nucleobase sequence consisting of SEQ ID NO. 13. In certain embodiments, an antisense compound has a nucleobase sequence consisting of SEQ ID NO. 14. In certain embodiments, an antisense compound has a nucleobase sequence consisting of SEQ ID NO. 15. In certain embodiments, an antisense compound has a nucleobase sequence consisting of SEQ ID NO. 16. In certain embodiments, an antisense compound has a nucleobase sequence consisting of SEQ ID NO. 17.

In certain embodiments, an antisense compound has a nucleobase sequence consisting of SEQ ID NO. 18. In certain embodiments, an antisense compound has a nucleobase sequence consisting of SEQ ID NO. 19. In certain embodiments, an antisense compound has a nucleobase sequence consisting of SEQ ID NO. 20. In certain embodiments, an antisense compound has a nucleobase sequence consisting of SEQ ID NO. 21. In certain embodiments, an antisense compound has a nucleobase sequence consisting of SEQ ID NO. 22. In certain embodiments, an antisense compound has a nucleobase sequence consisting of SEQ ID NO. 23. In certain embodiments, an antisense compound has a nucleobase sequence consisting of SEQ ID NO. 24. In certain embodiments, an antisense compound has a nucleobase sequence consisting of SEQ ID NO. 25. In certain embodiments, an antisense compound has a nucleobase sequence consisting of SEQ ID NO. 26. In certain embodiments, an antisense compound has a nucleobase sequence consisting of SEQ ID NO. 27. In certain embodiments, an antisense compound has a nucleobase sequence consisting of SEQ ID NO. 28. In certain embodiments, an antisense compound has a nucleobase sequence consisting of SEQ ID NO. 29. In certain embodiments, an antisense compound has a nucleobase sequence consisting of SEQ ID NO. 30. In certain embodiments, an antisense compound has a nucleobase sequence consisting of SEQ ID NO. 31. In certain embodiments, an antisense compound has a nucleobase sequence consisting of SEQ ID NO. 32.

In certain embodiments, an antisense compound has a nucleobase sequence consisting of SEQ ID NO. 33. In certain embodiments, an antisense compound has a nucleobase sequence consisting of SEQ ID NO.34. In certain embodiments, an antisense compound has a nucleobase sequence consisting of SEQ ID NO. 35. In certain embodiments, an antisense compound has a nucleobase sequence consisting of SEQ ID NO. 36. In certain embodiments, an antisense compound has a nucleobase sequence consisting of SEQ ID NO. 37. In certain embodiments, an antisense compound has a nucleobase sequence consisting of SEQ ID NO. 38. In certain embodiments, an antisense compound has a nucleobase sequence consisting of SEQ ID NO. 39. In certain embodiments, an antisense compound has a nucleobase sequence consisting of SEQ ID NO. 40. In certain embodiments, an antisense compound has a nucleobase sequence consisting of SEQ ID NO. 41. In certain embodiments, an antisense compound has a nucleobase sequence consisting of SEQ ID NO. 42. In certain embodiments, an antisense compound has a nucleobase sequence consisting of SEQ ID NO. 43. In certain embodiments, an antisense compound has a nucleobase sequence consisting of SEQ ID NO. 44. In certain embodiments, an antisense compound has a nucleobase sequence consisting of SEQ ID NO. 45. In certain embodiments, an antisense compound has a nucleobase sequence consisting of SEQ ID NO. 46. In certain embodiments, an antisense compound has a nucleobase sequence consisting of SEQ ID NO. 47.

In certain embodiments, an antisense compound has a nucleobase sequence consisting of SEQ ID NO. 48. In certain embodiments, an antisense compound has a nucleobase sequence consisting of SEQ ID NO. 49. In certain embodiments, an antisense compound has a nucleobase sequence consisting of SEQ ID NO. 50. In certain embodiments, an antisense compound has a nucleobase sequence consisting of SEQ ID NO. 51. In certain embodiments, an antisense compound has a nucleobase sequence consisting of SEQ ID NO. 52. In certain embodiments, an antisense compound has a nucleobase sequence consisting of SEQ ID NO. 53. In certain embodiments, an antisense compound has a nucleobase sequence consisting of SEQ ID NO. 54. In certain embodiments, an antisense compound has a nucleobase sequence consisting of SEQ ID NO. 55. In certain embodiments, an antisense compound has a nucleobase sequence consisting of SEQ ID NO. 56. In certain embodiments, an antisense compound has a nucleobase sequence consisting of SEQ ID NO. 57. In certain embodiments, an antisense compound has a nucleobase sequence consisting of SEQ ID NO. 58. In certain embodiments, an antisense compound has a nucleobase sequence consisting of SEQ ID NO. 59. In certain embodiments, an antisense compound has a nucleobase sequence consisting of SEQ ID NO. 60.

In certain embodiments, an antisense oligonucleotide modulates splicing of a pre-mRNA. In certain embodiments, an antisense oligonucleotide modulates splicing a CLN3 pre-mRNA. In certain embodiments, an antisense oligonucleotide increases the amount of CLN3 mRNA. In certain embodiments, an antisense oligonucleotide increases the exclusion of exon 6 in CLN3 mRNA. In certain embodiments, an antisense oligonucleotide decreases the inclusion of exon 6 in CLN3 mRNA. In certain embodiments, an antisense oligonucleotide increases the exclusion of exon 9 in CLN3 mRNA. In certain embodiments, an antisense oligonucleotide decreases the inclusion of exon 9 in CLN3 mRNA.

In certain embodiments, an antisense oligonucleotide comprises ISIS NO. 616709 (SEQ ID NO: 20). In certain embodiments, an antisense oligonucleotide consists of ISIS NO. 616709.

In certain embodiments, an antisense oligonucleotide promotes skipping of exon 6. In certain embodiments, an antisense oligonucleotide promotes skipping of exon 6 of a *CLN3Δ78* transcript. In certain embodiments, an antisense oligonucleotide promotes skipping of exon 9. In certain embodiments, an antisense oligonucleotide promotes skipping of exon 9 of a *CLN3Δ78* transcript.

In certain embodiments, an antisense oligonucleotide comprises the nucleobase sequence and modification motif of ISIS NO. 688555. In certain embodiments, an antisense oligonucleotide consists of the nucleobase sequence and modification motif of ISIS NO. 688555. In certain embodiments, an antisense oligonucleotide comprises the nucleobase sequence and modification motif of ISIS NO. 688559. In certain embodiments, an antisense oligonucleotide consists of the nucleobase sequence and modification motif of ISIS NO. 688559. In certain embodiments, an antisense oligonucleotide comprises the nucleobase sequence and modification motif of ISIS NO. 688595. In certain embodiments, an antisense oligonucleotide consists of the nucleobase sequence and modification motif of ISIS NO. 688595.

In certain embodiments, an antisense compound has a nucleobase sequence consisting of SEQ ID NO. 63. In certain embodiments, an antisense compound has a nucleobase sequence consisting of SEQ ID NO. 64. In certain embodiments, an antisense compound has a nucleobase sequence consisting of SEQ ID NO. 65. In certain embodiments, an antisense compound has a nucleobase sequence consisting of SEQ ID NO. 66. In certain embodiments, an antisense compound has a nucleobase sequence consisting of SEQ ID NO. 67. In certain embodiments, an antisense compound has a nucleobase sequence consisting of SEQ ID NO. 68. In certain embodiments, an antisense compound has a nucleobase sequence consisting of SEQ ID NO. 69. In certain embodiments, an antisense compound has a nucleobase sequence consisting of SEQ ID NO. 70. In certain embodiments, an antisense compound has a nucleobase sequence consisting of SEQ ID NO. 71. In certain embodiments, an antisense compound has a nucleobase sequence consisting of SEQ ID NO. 72. In certain embodiments, an antisense compound has a nucleobase sequence consisting of SEQ ID NO. 73. In certain embodiments, an antisense compound has a nucleobase sequence consisting of SEQ ID NO. 74. In certain embodiments, an antisense compound has a nucleobase sequence consisting of SEQ ID NO. 75. In certain embodiments, an antisense compound has a nucleobase sequence consisting of SEQ ID NO. 76. In certain embodiments, an antisense compound has a nucleobase sequence consisting of SEQ ID NO. 77. In certain embodiments, an antisense compound has a nucleobase sequence consisting of SEQ ID NO. 78. In certain embodiments, an antisense compound has a nucleobase sequence consisting of SEQ ID NO. 79. In certain embodiments, an antisense compound has a nucleobase sequence consisting of SEQ ID NO. 80. In certain embodiments, an antisense compound has a nucleobase sequence consisting of SEQ ID NO. 81. In certain embodiments, an antisense compound has a nucleobase sequence consisting of SEQ ID NO. 82. In certain embodiments, an antisense compound has a nucleobase sequence consisting of SEQ ID NO. 83 In certain embodiments, an antisense compound has a nucleobase sequence consisting of SEQ ID NO. 84. In certain embodiments, an antisense compound has a nucleobase sequence consisting of SEQ ID NO. 85. In certain embodiments, an antisense compound has a nucleobase sequence consisting of SEQ ID NO. 86. In certain embodiments, an antisense compound has a nucleobase sequence consisting of SEQ ID NO. 87. In certain embodiments, an antisense compound has a nucleobase sequence consisting of SEQ ID NO. 88. In certain embodiments, an antisense compound has a nucleobase sequence consisting of SEQ ID NO. 89. In certain embodiments, an antisense compound has a nucleobase sequence consisting of SEQ ID NO. 90.

In certain embodiments, an antisense compound has a nucleobase sequence consisting of SEQ ID NO. 91. In certain embodiments, an antisense compound has a nucleobase sequence consisting of SEQ ID NO. 92. In certain embodiments, an antisense compound has a nucleobase sequence consisting of SEQ ID NO. 93. In certain embodiments, an antisense compound has a nucleobase sequence consisting of SEQ ID NO. 94. In certain embodiments, an antisense compound has a nucleobase sequence consisting of SEQ ID NO. 95. In certain embodiments, an antisense compound has a nucleobase sequence consisting of SEQ ID NO. 96. In certain embodiments, an antisense compound has a nucleobase sequence consisting of SEQ ID NO. 97. In certain embodiments, an antisense compound has a nucleobase sequence consisting of SEQ ID NO. 98. In certain embodiments, an antisense compound has a nucleobase sequence consisting of SEQ ID NO. 99. In certain embodiments, an antisense compound has a nucleobase sequence consisting of SEQ ID NO. 100. In certain embodiments, an antisense compound has a nucleobase sequence consisting of SEQ ID NO. 101.

In certain embodiments, an antisense compound has a nucleobase sequence consisting of SEQ ID NO. 102. In certain embodiments, an antisense compound has a nucleobase sequence consisting of SEQ ID NO. 103. In certain embodiments, an antisense compound has a nucleobase sequence consisting of SEQ ID NO. 104. In certain embodiments, an antisense compound has a nucleobase sequence consisting of SEQ ID NO. 105. In certain embodiments, an antisense compound has a nucleobase sequence consisting of SEQ ID NO. 106. In certain embodiments, an antisense compound has a nucleobase sequence consisting of SEQ ID NO. 107. In certain embodiments, an antisense compound has a nucleobase sequence consisting of SEQ ID NO. 108. In certain embodiments, an antisense compound has a nucleobase sequence consisting of SEQ ID NO. 109. In certain embodiments, an antisense compound has a nucleobase sequence consisting of SEQ ID NO. 110.

In certain embodiments, an antisense compound has a nucleobase sequence consisting of SEQ ID NO. 111. In certain embodiments, an antisense compound has a nucleobase sequence consisting of SEQ ID NO. 112. In certain embodiments, an antisense compound has a nucleobase sequence consisting of SEQ ID NO. 113. In certain embodiments, an antisense compound has a nucleobase sequence consisting of SEQ ID NO. 114. In certain embodiments, an antisense compound has a nucleobase sequence consisting of SEQ ID NO. 115. In certain embodiments, an antisense compound has a nucleobase sequence consisting of SEQ ID NO. 116. In certain embodiments, an antisense compound has a nucleobase sequence consisting of SEQ ID NO. 117. In certain embodiments, an antisense compound has a nucleobase sequence consisting of SEQ ID NO. 118. In certain embodiments, an antisense compound has a nucleobase sequence consisting of SEQ ID NO. 119. In certain embodiments, an antisense compound has a nucleobase sequence consisting of SEQ ID NO. 120. In certain embodiments, an antisense compound has a nucleobase sequence consisting of SEQ ID NO. 121. In certain embodiments, an antisense compound has a nucleobase sequence consisting of SEQ ID NO. 122. In certain embodiments, an antisense compound has a nucleobase sequence consisting of SEQ ID NO. 123. In certain embodiments, an antisense compound has a nucleobase sequence consisting of SEQ ID NO. 124. In certain embodiments, an antisense compound has a nucleobase sequence consisting of SEQ ID NO. 125. In certain embodiments, an antisense compound has a nucleobase sequence consisting of SEQ ID NO. 126. In certain embodiments, an antisense compound has a nucleobase sequence consisting of SEQ ID NO. 127.

### Certain Pharmaceutical Compositions

The present invention relates to pharmaceutical compositions comprising one or more antisense compound. In certain embodiments, such pharmaceutical composition comprises a suitable pharmaceutically acceptable diluent or carrier. In certain embodiments, a pharmaceutical composition comprises a sterile saline solution and one or more antisense compound. In certain embodiments, such pharmaceutical composition consists of a sterile saline solution and one or more antisense compound. In certain embodiments, the sterile saline is pharmaceutical grade saline. In certain embodiments, a pharmaceutical composition comprises one or more antisense compound and sterile water. In certain embodiments, a pharmaceutical composition consists of one or more antisense compound and sterile water. In certain embodiments, the sterile saline is pharmaceutical grade water. In certain embodiments, a pharmaceutical composition comprises one or more antisense compound and phosphate-buffered saline (PBS). In certain embodiments, a pharmaceutical composition consists of one or more antisense compound and sterile phosphate-buffered saline (PBS). In certain embodiments, the sterile saline is pharmaceutical grade PBS.

In certain embodiments, antisense compounds may be admixed with pharmaceutically acceptable active and/or inert substances for the preparation of pharmaceutical compositions or formulations. Compositions and methods for the formulation of pharmaceutical compositions depend on a number of criteria, including, but not limited to, route of administration, extent of disease, or dose to be administered.

Pharmaceutical compositions comprising antisense compounds encompass any pharmaceutically acceptable salts, esters, or salts of such esters. In certain embodiments, pharmaceutical compositions comprising antisense compounds comprise one or more oligonucleotide which, upon administration to an animal, including a human, is capable of providing (directly or indirectly) the biologically active metabolite or residue thereof. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts.

A prodrug can include the incorporation of additional nucleosides at one or both ends of an oligomeric compound which are cleaved by endogenous nucleases within the body, to form the active antisense oligomeric compound.

Lipid moieties have been used in nucleic acid therapies in a variety of methods. In certain such methods, the nucleic acid is introduced into preformed liposomes or lipoplexes made of mixtures of cationic lipids and neutral lipids. In certain methods, DNA complexes with mono- or poly-cationic lipids are formed without the presence of a neutral lipid. In certain embodiments, a lipid moiety is selected to increase distribution of a pharmaceutical agent to a particular cell or tissue. In certain embodiments, a lipid moiety is selected to increase distribution of a pharmaceutical agent to fat tissue. In certain embodiments, a lipid moiety is selected to increase distribution of a pharmaceutical agent to muscle tissue.

In certain embodiments, pharmaceutical compositions disclosed herein comprise one or more modified oligonucleotides and one or more excipients. In certain such embodiments, excipients are selected from water, salt solutions, alcohol, polyethylene glycols, gelatin, lactose, amylase, magnesium stearate, talc, silicic acid, viscous paraffin, hydroxymethylcellulose and polyvinylpyrrolidone.

In certain embodiments, a pharmaceutical composition disclosed herein comprises a delivery system. Examples of delivery systems include, but are not limited to, liposomes and emulsions. Certain delivery systems are useful for preparing certain pharmaceutical compositions including those comprising hydrophobic compounds. In certain embodiments, certain organic solvents such as dimethylsulfoxide are used.

In certain embodiments, a pharmaceutical composition disclosed herein comprises one or more tissue-specific delivery molecules designed to deliver the one or more pharmaceutical agents of use in the present invention to specific tissues or cell types. For example, in certain embodiments, pharmaceutical compositions include liposomes coated with a tissue-specific antibody.

In certain embodiments, a pharmaceutical composition disclosed herein comprises a co-solvent system. Certain of such co-solvent systems comprise, for example, benzyl alcohol, a nonpolar surfactant, a water-miscible organic polymer, and an aqueous phase. In certain embodiments, such co-solvent systems are used for hydrophobic compounds. A non-limiting example of such a co-solvent system is the VPD co-solvent system, which is a solution of absolute ethanol comprising 3% w/v benzyl alcohol, 8% w/v of the nonpolar surfactant Polysorbate 80™ and 65% w/v polyethylene glycol 300. The proportions of such co-solvent systems may be varied considerably without significantly altering their solubility and toxicity characteristics. Furthermore, the identity of co-solvent components may be varied: for example, other surfactants may be used instead of Polysorbate 80™; the fraction size of polyethylene glycol may be varied; other biocompatible polymers may replace polyethylene glycol, e.g., polyvinyl pyrrolidone; and other sugars or polysaccharides may substitute for dextrose.

In certain embodiments, a pharmaceutical composition disclosed herein is prepared for oral administration. In certain embodiments, pharmaceutical compositions are prepared for buccal administration.

In certain embodiments, a pharmaceutical composition is prepared for administration by injection (e.g., intravenous, subcutaneous, intramuscular, etc.). In certain of such embodiments, a pharmaceutical composition comprises a carrier and is formulated in aqueous solution, such as water or physiologically compatible buffers such as Hanks's solution, Ringer's solution, or physiological saline buffer. In certain embodiments, other ingredients are included (e.g., ingredients that aid in solubility or serve as preservatives). In certain embodiments, injectable suspensions are prepared using appropriate liquid carriers, suspending agents and the like. Certain pharmaceutical compositions for injection are presented in unit dosage form, e.g., in ampoules or in multi-dose containers. Certain pharmaceutical compositions for injection are suspensions, solutions or emulsions in oily or aqueous vehicles, and may contain formulatory agents such as suspending, stabilizing and/or dispersing agents. Certain solvents suitable for use in pharmaceutical compositions for injection include, but are not limited to, lipophilic solvents and fatty oils, such as sesame oil, synthetic fatty acid esters, such as ethyl oleate or triglycerides, and liposomes. Aqueous injection suspensions may contain substances that increase the viscosity of the suspension, such as sodium carboxymethyl cellulose, sorbitol, or dextran. Optionally, such suspensions may also contain suitable stabilizers or agents that increase the solubility of the pharmaceutical agents to allow for the preparation of highly concentrated solutions.

In certain embodiments, a pharmaceutical composition is prepared for transmucosal administration. In certain of such embodiments penetrants appropriate to the barrier to be permeated are used in the formulation. Such penetrants are generally known in the art.

In certain embodiments, a pharmaceutical composition disclosed herein comprises an oligonucleotide in a therapeutically effective amount. In certain embodiments, the therapeutically effective amount is sufficient to prevent, alleviate or ameliorate symptoms of a disease or to prolong the survival of the subject being treated. Determination of a therapeutically effective amount is well within the capability of those skilled in the art.

In certain embodiments, one or more modified oligonucleotide disclosed herein is formulated as a prodrug. In certain embodiments, upon *in vivo* administration, a prodrug is chemically converted to the biologically, pharmaceutically or therapeutically more active form of an oligonucleotide. In certain embodiments, prodrugs are useful because they are easier to administer than the corresponding active form. For example, in certain instances, a prodrug may be more bioavailable (e.g., through oral administration) than is the corresponding active form. In certain instances, a prodrug may have improved solubility compared to the corresponding active form. In certain embodiments, prodrugs are less water soluble than the corresponding active form. In certain instances, such prodrugs possess superior transmittal across cell membranes, where water solubility is detrimental to mobility. In certain embodiments, a prodrug is an ester. In certain such embodiments, the ester is metabolically hydrolyzed to carboxylic acid upon administration. In certain instances the carboxylic acid containing compound is the corresponding active form. In certain embodiments, a prodrug comprises a short peptide (polyaminoacid) bound to an acid group. In certain of such embodiments, the peptide is cleaved upon administration to form the corresponding active form.

In certain embodiments, the present disclosure relates to compositions and methods for reducing the amount or activity of a target nucleic acid in a cell. In certain embodiments, the cell is in an animal. In certain embodiments, the animal is a mammal. In certain embodiments, the animal is a rodent. In certain embodiments, the animal is a primate. In certain embodiments, the animal is a non-human primate. In certain embodiments, the animal is a human.

In certain embodiments, the present disclosure relates to methods of administering a pharmaceutical composition comprising an oligomeric compound as disclosed herein to an animal. Suitable administration routes include, but are not limited to, oral, rectal, transmucosal, intestinal, enteral, topical, suppository, through inhalation, intrathecal, intracerebroventricular, intraperitoneal, intranasal, intratumoral, and parenteral (e.g., intravenous, intramuscular, intramedullary, and subcutaneous). In certain embodiments, pharmaceutical intrathecals are administered to achieve local rather than systemic exposures. For example, pharmaceutical compositions may be injected directly in the area of desired effect (e.g., into the ears).

In certain embodiments, a pharmaceutical composition is administered to an animal having at least one symptom associated with Batten Disease. In certain embodiments, such administration results in amelioration of at least one symptom. In certain embodiments, administration of a pharmaceutical composition to an animal results in an increase in functional CLN3 protein in a cell. In certain embodiments, the administration of certain antisense oligonucleotides delays the onset of Batten Disease. In certain embodiments, the administration of certain antisense oligonucleotides prevents the onset of Batten Disease. In certain embodiments, the administration of certain antisense oligonucleotides rescues cellular phenotype.

### Nonlimiting disclosure

While certain compounds, compositions and methods described herein have been described with specificity in accordance with certain embodiments, the following examples serve only to illustrate the compounds described herein and are not intended to limit the same. Although the sequence listing accompanying this filing identifies each sequence as either "RNA" or "DNA" as required, in reality, those sequences may be modified with any combination of chemical modifications. One of skill in the art will readily appreciate that such designation as "RNA" or "DNA" to describe modified oligonucleotides is, in certain instances, arbitrary. For example, an oligonucleotide comprising a nucleoside comprising a 2'-OH sugar moiety and a thymine base could be described as a DNA having a modified sugar (2'-OH for the natural 2'-H of DNA) or as an RNA having a modified base (thymine (methylated uracil) for natural uracil of RNA).

Accordingly, nucleic acid sequences herein, including, but not limited to those in the sequence listing, are intended to encompass nucleic acids containing any combination of natural or modified RNA and/or DNA, including, but not limited to such nucleic acids having modified nucleobases. By way of further example and without limitation, an oligomeric compound having the nucleobase sequence "ATCGATCG" encompasses any oligomeric compounds having such nucleobase sequence, whether modified or unmodified, including, but not limited to, such compounds comprising RNA bases, such as those having sequence "AUCGAUCG" and those having some DNA bases and some RNA bases such as "AUCGATCG" and oligomeric compounds having other modified or naturally occurring bases, such as "AT^{me}CGAUCG," wherein ^{me}C indicates a cytosine base comprising a methyl group at the 5-position.

### Examples

The following examples illustrate certain embodiments of the present invention and are not limiting. Moreover, where specific embodiments are provided, the inventors have contemplated generic application of those specific embodiments. For example, disclosure of an oligonucleotide having a particular motif provides reasonable support for additional oligonucleotides having the same or similar motif. And, for example, where a particular high-affinity modification appears at a particular position, other high-affinity modifications at the same position are considered suitable, unless otherwise indicated.

### Example 1: Antisense Oligonucleotide Synthesis

The preparation of nucleoside phosphoramidites is performed following procedures that are extensively illustrated in the art such as but not limited to US Patent 6,426,220 and published PCT application WO 02/36743.

### Oligonucleotide and oligonucleoside synthesis

The oligomeric compounds used in accordance with this invention may be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives.

Oligonucleotides: Unsubstituted and substituted phosphodiester (P=O) oligonucleotides are synthesized on an automated DNA synthesizer (Applied Biosystems model 394) using standard phosphoramidite chemistry with oxidation by iodine.

Phosphorothioates (P=S) are synthesized similar to phosphodiester oligonucleotides with the following exceptions: thiation was effected by utilizing a 10% w/v solution of 3,H-1,2-benzodithiole-3-one 1,1-dioxide in acetonitrile for the oxidation of the phosphite linkages. The thiation reaction step time was increased to 180 sec and preceded by the normal capping step. After cleavage from the CPG column and deblocking in concentrated ammonium hydroxide at 55°C (12-16 hr), the oligonucleotides were recovered by precipitating with >3 volumes of ethanol from a 1 M NH₄OAc solution. Phosphinate oligonucleotides are prepared as described in U.S. Patent 5,508,270.

Alkyl phosphonate oligonucleotides are prepared as described in U.S. Patent 4,469,863.

3'-Deoxy-3'-methylene phosphonate oligonucleotides are prepared as described in U.S. Patents 5,610,289 or 5,625,050.

Phosphoramidite oligonucleotides are prepared as described in U.S. Patent, 5,256,775 or U.S. Patent 5,366,878.

Alkylphosphonothioate oligonucleotides are prepared as described in published PCT applications PCT/US94/00902 and PCT/US93/06976 (published as WO 94/17093 and WO 94/02499, respectively).

3'-Deoxy-3'-amino phosphoramidate oligonucleotides are prepared as described in U.S. Patent 5,476,925.

Phosphotriester oligonucleotides are prepared as described in U.S. Patent 5,023,243.

Borano phosphate oligonucleotides are prepared as described in U.S. Patents 5,130,302 and 5,177,198.

Oligonucleosides: Methylenemethylimino linked oligonucleosides, also identified as MMI linked oligonucleosides, methylenedimethylhydrazo linked oligonucleosides, also identified as MDH linked oligonucleosides, and methylenecarbonylamino linked oligonucleosides, also identified as amide-3 linked oligonucleosides, and methyleneaminocarbonyl linked oligonucleosides, also identified as amide-4 linked oligonucleosides, as well as mixed backbone oligomeric compounds having, for instance, alternating MMI and P=O or P=S linkages are prepared as described in U.S. Patents 5,378,825, 5,386,023, 5,489,677, 5,602,240 and 5,610,289.

Formacetal and thioformacetal linked oligonucleosides are prepared as described in U.S. Patents 5,264,562 and 5,264,564.

Ethylene oxide linked oligonucleosides are prepared as described in U.S. Patent 5,223,618.

### Oligonucleotide Isolation

After cleavage from the controlled pore glass solid support and deblocking in concentrated ammonium hydroxide at 55°C for 12-16 hours, the oligonucleotides or oligonucleosides are recovered by precipitation out of 1 M NH₄OAc with >3 volumes of ethanol. Synthesized oligonucleotides were analyzed by electrospray mass spectroscopy (molecular weight determination) and by capillary gel electrophoresis and judged to be at least 70% full length material. The relative amounts of phosphorothioate and phosphodiester linkages obtained in the synthesis was determined by the ratio of correct molecular weight relative to the -16 amu product (+/-32 +/-48). For some studies oligonucleotides were purified by HPLC, as described by Chiang et al., J. Biol. Chem. 1991, 266, 18162-18171. Results obtained with HPLC-purified material were similar to those obtained with non-HPLC purified material.

### Oligonucleotide Synthesis - 96 Well Plate Format

Oligonucleotides can be synthesized via solid phase P(III) phosphoramidite chemistry on an automated synthesizer capable of assembling 96 sequences simultaneously in a 96-well format. Phosphodiester internucleotide linkages are afforded by oxidation with aqueous iodine. Phosphorothioate internucleotide linkages are generated by sulfurization utilizing 3,H-1,2 benzodithiole-3-one 1,1 dioxide (Beaucage Reagent) in anhydrous acetonitrile. Standard base-protected beta-cyanoethyl-diiso-propyl phosphoramidites are purchased from commercial vendors (e.g. PE-Applied Biosystems, Foster City, CA, or Pharmacia, Piscataway, NJ). Non-standard nucleosides are synthesized as per standard or patented methods. They are utilized as base protected beta-cyanoethyldiisopropyl phosphoramidites.

Oligonucleotides are cleaved from support and deprotected with concentrated NH₄OH at elevated temperature (55-60°C) for 12-16 hours and the released product then dried *in vacuo.* The dried product is then re-suspended in sterile water to afford a master plate from which all analytical and test plate samples are then diluted utilizing robotic pipettors.

### Oligonucleotide Analysis using 96-Well Plate Format

The concentration of oligonucleotide in each well is assessed by dilution of samples and UV absorption spectroscopy. The full-length integrity of the individual products is evaluated by capillary electrophoresis (CE) in either the 96-well format (Beckman P/ACE™ MDQ) or, for individually prepared samples, on a commercial CE apparatus (e.g., Beckman P/ACE™ 5000, ABI 270). Base and backbone composition is confirmed by mass analysis of the oligomeric compounds utilizing electrospray-mass spectroscopy. A11 assay test plates are diluted from the master plate using single and multi-channel robotic pipettors. Plates are judged to be acceptable if at least 85% of the oligomeric compounds on the plate are at least 85% full length.

### Example 2: Expression of CLN3Δ678 and CLN3Δ78 in vitro

Plasmids comprising a C-terminal FLAG-tagged WT or mutant CLN3 gene were prepared using standard molecular biology techniques. The mutant CLN3 genes contained a deletion of exons 6, 7, and 8 (CLN3Δ678) or deletion of exons 7, 8, and 9 (CLN3Δ789). The WT and mutant CLN3 genes were under control of the CMV promoter. HeLa cells were transfected with plasmids containing WT CLN3, CLN3Δ78, CLN3Δ678, or empty plasmids (mock) using standard methods. 48 hours after transfection, the cells were fixed and stained with anti-FLAG M2 FITC antibody (F4049, Sigma), mounted with Prolong antifade with DAPI (Invitrogen) and visualized under an Olympus FV10i confocal microscope.

While none of the mock or *CLN3Δ78* transfected cells exhibited FITC fluorescence, many of the WT CLN3 and CLN3Δ678 transfected cells were fluorescent in the FITC channel. These results indicate that the *CLN3Δ78* protein was not visibly expressed, but the wild type CLN3 and CLN3Δ678 proteins were visibly expressed.

### Example 3: Antisense Modulation of CLN3 Transcript Splicing

An antisense oligonucleotide targeted to the exon 6/intron 6 junction of human CLN3 pre-mRNA (MO-ASO ACACAGAACCACACACTCACCACAC, SEQ ID NO: 3) was synthesized by Gene Tools, LLC, and tested for its ability to modulate splicing of CLN3. The ASO targets the exon 6/intron 6 junction of the complement of GENBANK accession number NT_010393.16 truncated from nucleotides 28427600 to 28444620 (SEQ ID NO: 1). The ASO is a uniform morpholino ASO.

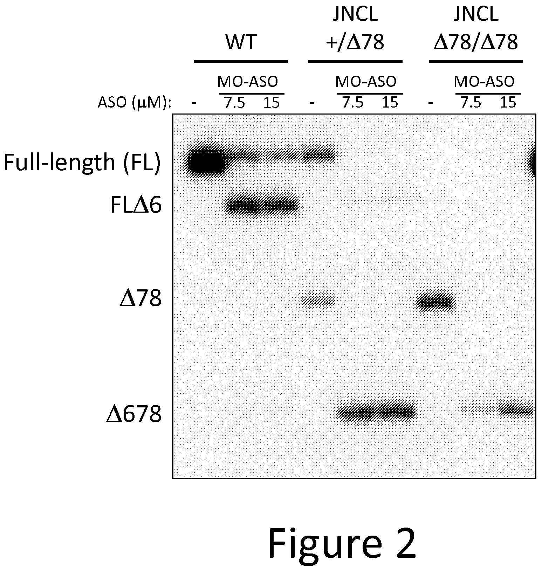

0 µM, 7.5 µM, 15 µM of the morpholino ASO targeted to the exon 6/intron 6 junction (MO-ASO) and Endo-porter transfection reagent (Gene Tools, LLC) were added to fibroblast cells of a JNCL patient homozygous for the *CLN3Δ78* mutation, a heterozygous carrier of the *CLN3Δ78* mutation, and a family member of the JNCL patient who is homozygous for WT CLN3. After 48 hours, total RNA was then isolated from the cells and CLN3 mRNA was detected via radioactive RT-PCR and PAGE. The gel is shown in Figure 2. Bands were quantitated by densitometry analysis using Image J software, and the results are shown in Table 1 below. In Table 1, 'Ht' means heterozygote, and 'Hm' means homozygote.

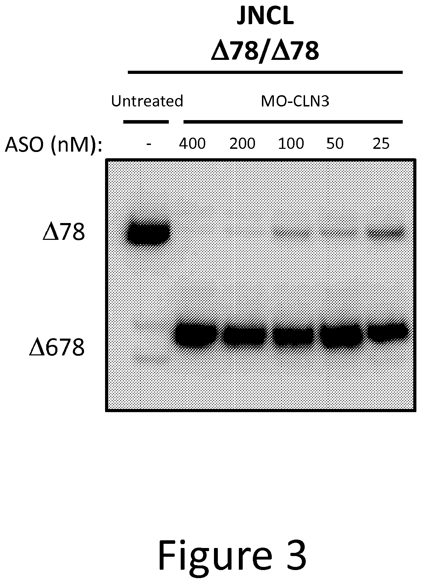

In a similar experiment, increasing concentrations of MO-ASO were incubated with homozygous CLN3Δ78/Δ78 patient fibroblasts. The lowest dose of 25 nM induced % exon 6 skipping, and the highest doses tested induced % exon 6 skipping. The results are shown in Table 2 below, and the gel is shown in Figure 3.

**Table 1: Modulation of Splicing of CLN3 pre-mRNA by MO-ASO as analyzed by RT-PCR**

| Genotype | MO-ASO Concentration | % of all Isoforms Lacking Exon 6 |
|---|---|---|
| WT | 0 µM (untreated control) | 0.0 |
| WT | 7.5 µM | 67.9 |
| WT | 15 µM | 73.7 |
| Ht | 0 µM (untreated control) | 0.0 |
| Ht | 7.5 µM | 92.8 |
| Ht | 15 µM | 93.4 |
| Hm | 0 µM (untreated control) | 0.0 |
| Hm | 7.5 µM | 74.6 |
| Hm | 15 µM | 93.3 |

**Table 2: MO-ASO Dose Response Analysis of CLN3 pre-mRNA Splicing in CLN3Δ78/Δ78 cells**

| MO-ASO concentration | % of all Isoforms Lacking Exon 6 |
|---|---|
| 0 nM (untreated control) | 4.0 |
| 25 nM | 82.9 |
| 50 nM | 94.5 |
| 100 nM | 89.7 |
| 200 nM | 98.4 |
| 400 nM | 99.4 |

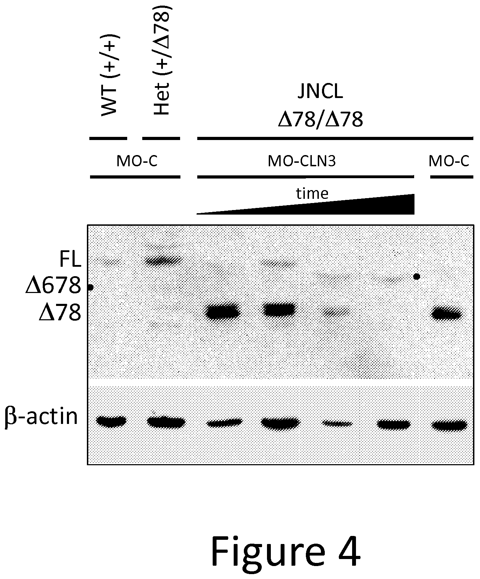

CLN3 protein levels were measured in a similar experiment in which a single concentration (7.5µM) of the MO-ASO or a control morpholino oligonucleotide (MO-C, AGCTGATCATATTCTACCTGGTGCT SEQ ID NO: 62) was used and protein levels in homozygous mutant *CLN3Δ78* cells were analyzed at the following 0, 56, 144, or 192 hours following incubation. Protein levels were analyzed by western blot using a rabbit poly-clonal anti-CLN3 antibody (Proteintech) and anti-β-actin as a loading control. The blot is shown in Figure 4.

### Example 4: Design of Antisense Oligonucleotides for Induction of CLN3 Exon 6 Skipping

Antisense oligonucleotides complementary to exon 6 and/or the introns flanking exon 6 of the mouse CLN3 pre-mRNA transcript were synthesized and tested for their ability to modulate splicing of CLN3 by skipping exon 6. The oligonucleotides in Table 3 below are uniformly 2'-MOE modified, and all internucleoside linkages are phosphorothioate linkages. The cytosine bases are 5-methylcytosine. The ASOs target the complement of GENBANK accession number NT_039433.8, truncated from nucleotides 44319075 to 44333955 (SEQ ID NO: 2), and the ASO sequences are listed in Table 3 below. The start and stop sites associated with each oligonucleotide are the 5'- and 3'-positions, respectively, of the portion of SEQ ID NO: 2 that is complementary to the ASO.

**Table 3: Antisense Oligonucleotides for Induction of CLN3 Exon 6 Skipping**

| ISIS No. | Start Site | Stop Site | Sequence (5' to 3') | CLN3 Target Region | SEQ ID NO |
|---|---|---|---|---|---|
| 616693 | 5030 | 5047 | GAGAAGAGATGAGGAGGA | Intron 5 | 4 |
| 616694 | 5034 | 5051 | GGCTGAGAAGAGATGAGG | Intron 5/Exon 6 | 5 |
| 616695 | 5035 | 5052 | GGGCTGAGAAGAGATGAG | Intron 5/Exon 6 | 6 |
| 616696 | 5052 | 5069 | CACTGACGAGCACCCGGG | Exon 6 | 7 |
| 616697 | 5053 | 5070 | CCACTGACGAGCACCCGG | Exon 6 | 8 |
| 616698 | 5054 | 5071 | TCCACTGACGAGCACCCG | Exon 6 | 9 |
| 616699 | 5058 | 5075 | AAACTCCACTGACGAGCA | Exon 6 | 10 |
| 616700 | 5062 | 5079 | GAACAAACTCCACTGACG | Exon 6 | 11 |
| 616701 | 5066 | 5083 | AGCAGAACAAACTCCACT | Exon 6 | 12 |
| 616702 | 5070 | 5087 | TCCCAGCAGAACAAACTC | Exon 6 | 13 |
| 616703 | 5074 | 5091 | AAGCTCCCAGCAGAACAA | Exon 6 | 14 |
| 616704 | 5078 | 5095 | AACAAAGCTCCCAGCAGA | Exon 6 | 15 |
| 616705 | 5082 | 5099 | CCAGAACAAAGCTCCCAG | Exon 6 | 16 |
| 616706 | 5086 | 5103 | GCAACCAGAACAAAGCTC | Exon 6 | 17 |
| 616707 | 5090 | 5107 | GAAGGCAACCAGAACAAA | Exon 6 | 18 |
| 616708 | 5094 | 5111 | GAGAGAAGGCAACCAGAA | Exon 6 | 19 |
| 616709 | 5098 | 5115 | GACTGAGAGAAGGCAACC | Exon 6 | 20 |
| 616710 | 5102 | 5119 | CACTGACTGAGAGAAGGC | Exon 6 | 21 |
| 616711 | 5106 | 5123 | ACCCCACTGACTGAGAGA | Exon 6 | 22 |
| 616712 | 5110 | 5127 | CTTAACCCCACTGACTGA | Exon 6 | 23 |
| 616713 | 5114 | 5131 | CAGGCTTAACCCCACTGA | Exon 6 | 24 |
| 616714 | 5118 | 5135 | CACACAGGCTTAACCCCA | Exon 6 | 25 |
| 616715 | 5122 | 5139 | TCACCACACAGGCTTAAC | Exon 6/Intron 6 | 26 |
| 616716 | 5126 | 5143 | GTACTCACCACACAGGCT | Exon 6/Intron 6 | 27 |
| 616717 | 5130 | 5147 | CTTCGTACTCACCACACA | Exon 6/Intron 6 | 28 |
| 616718 | 5134 | 5151 | CCAGCTTCGTACTCACCA | Exon 6/Intron 6 | 29 |
| 616719 | 5138 | 5155 | TCTCCCAGCTTCGTACTC | Intron 6 | 30 |

### Example 5: Antisense Oligonucleotide Induction of CLN3 Exon 6 Skipping in vitro

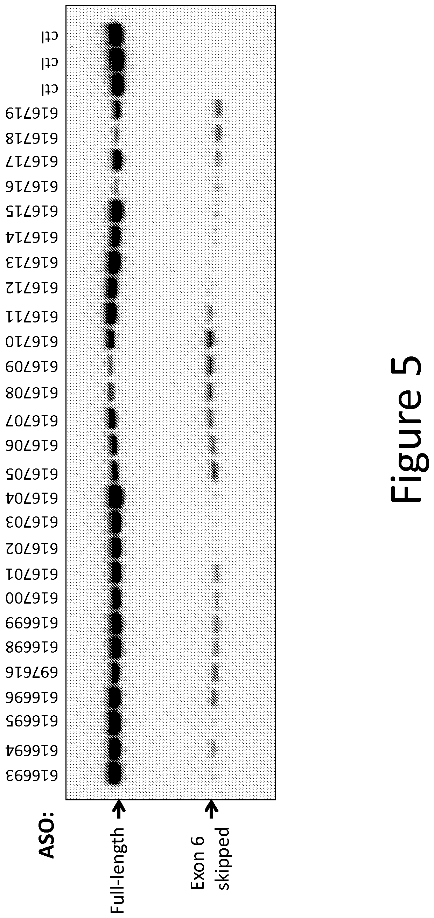

To test the ability of the antisense oligonucleotides to modulate splicing by skipping exon 6, mouse cell line 208ee, derived from C57/B16 mouse kidney, was transfected with 50 nM of the ASOs listed in Table 3 using Lipofectamine 2000 (Invitrogen). Untreated control cells received neither ASO nor Lipofectamine, while mock transfected cells received only Lipofectamine. After 48 hours, total RNA was collected from the cells and RT-PCR was used to identify CLN3 full-length and CLN3Δ6 transcripts. The PCR products were analyzed by PAGE and quantitated by phosphorimager analysis (Typhoon 9400, GE Healthcare). The gel is shown in Figure 5, and the results are shown in Table 4 below as the percentage of exon 6 skipped (Δ6) transcript out of the total full-length (FL) plus exon 6 skipped transcripts. Band intensities are reported in relative fluorescence units (RFU). This example illustrates that many ASOs induce CLN3 exon 6 skipping.

**Table 4: Induction of CLN3 Exon 6 Skipping in vitro**

| ISIS NO | Full-length CLN3 (RFU) | CLN3Δ6 (RFU) | Δ6/(FL + Δ6) (%) |
|---|---|---|---|
| untreated control | 6,823,783 | 762 | 0.01 |
| mock | 5,188,149 | 2,110 | 0.04 |
| 616693 | 6,723,467 | 253,542 | 3.63 |
| 616694 | 5,083,972 | 703,577 | 12.16 |
| 616695 | 6,278,156 | 225,133 | 3.46 |
| 616696 | 5,488,331 | 941,697 | 14.65 |
| 616697 | 3,262,413 | 1,067,086 | 24.65 |
| 616698 | 5,125,340 | 688,277 | 11.84 |
| 616699 | 4,999,204 | 727,472 | 12.70 |
| 616700 | 3,286,457 | 523332 | 13.74 |
| 616701 | 4,054,613 | 653,463 | 13.88 |
| 616702 | 4,047,520 | 73,846 | 1.79 |
| 616703 | 4,452,892 | 96,627 | 2.12 |
| 616704 | 7,603,794 | 152,903 | 1.97 |
| 616705 | 2,177,616 | 1,254,116 | 36.54 |
| 616706 | 2,222,239 | 903,504 | 28.91 |
| 616707 | 2,432,817 | 1,009,358 | 29.32 |
| 616708 | 1,182,124 | 1,120,453 | 48.66 |
| 616709 | 894,622 | 1,501,534 | 62.66 |
| 616710 | 2,315,933 | 1,597,682 | 40.82 |
| 616711 | 4,134,353 | 640,071 | 13.41 |
| 616712 | 3,928,470 | 229,565 | 5.52 |
| 616713 | 5,499,952 | 56,355 | 1.01 |
| 616714 | 3,618,665 | 180,643 | 4.75 |
| 616715 | 5,176,194 | 235,685 | 4.35 |
| 616716 | 692,245 | 335,115 | 32.62 |
| 616717 | 2,855,386 | 512,449 | 15.22 |
| 616718 | 730,706 | 832,631 | 53.26 |
| 616719 | 1,738,032 | 856,673 | 33.02 |

### Example 6: Design of Antisense Oligonucleotides for Induction of CLN3 Exon 9 Skipping

Antisense oligonucleotides complementary to exon 9 and/or the introns flanking exon 9 of the mouse CLN3 pre-mRNA transcript were synthesized and tested for their ability to modulate splicing of CLN3 by skipping exon 9. The oligonucleotides were uniformly modified to contain 2'-MOE, and all internucleoside linkages are phosphorothioate linkages. The cytosine bases are 5-methylcytosine. The ASOs target the complement of GENBANK accession number NT_039433.8 truncated from nucleotides 44319075 to 44333955 (SEQ ID NO: 2), and the ASO sequences are listed in Table 5 below. The start and stop sites associated with each oligonucleotide are the 5'- and 3'-positions, respectively, of the portion of SEQ ID NO: 2 that is complementary to the ASO.

**Table 5: Antisense Oligonucleotides for Induction of CLN3 Exon 9 Skipping**

| 3 | Start Site | Stop Site | Sequence (5'to 3') | CLN3 Target Region | SEQ ID NO |
|---|---|---|---|---|---|
| 616720 | 7338 | 7355 | GGGATGGAATGGAGAAGC | Intron 8 | 31 |
| 616721 | 7342 | 7359 | AGCTGGGATGGAATGGAG | Intron 8/Exon 9 | 32 |
| 616722 | 7346 | 7363 | AAATAGCTGGGATGGAAT | Intron 8/Exon 9 | 33 |
| 616723 | 7350 | 7367 | CAAGAAATAGCTGGGATG | Intron 8/Exon 9 | 34 |
| 616724 | 7354 | 7371 | GCAACAAGAAATAGCTGG | Intron 8/Exon 9 | 35 |
| 616725 | 7358 | 7375 | GTGAGCAACAAGAAATAG | Exon 9 | 36 |
| 616726 | 7362 | 7379 | AGACGTGAGCAACAAGAA | Exon 9 | 37 |
| 616727 | 7366 | 7383 | CAGGAGACGTGAGCAACA | Exon 9 | 38 |
| 616728 | 7370 | 7387 | GGTTCAGGAGACGTGAGC | Exon 9 | 39 |
| 616729 | 7371 | 7388 | GGGTTCAGGAGACGTGAG | Exon 9 | 40 |
| 616730 | 7387 | 7404 | CCCCTCCAGGGTCCAGGG | Exon 9 | 41 |
| 616731 | 7390 | 7407 | TTTCCCCTCCAGGGTCCA | Exon 9 | 42 |
| 616732 | 7394 | 7411 | TCGTTTTCCCCTCCAGGG | Exon 9 | 43 |
| 616733 | 7398 | 7415 | TGCCTCGTTTTCCCCTCC | Exon 9 | 44 |
| 616734 | 7402 | 7419 | TCTCTGCCTCGTTTTCCC | Exon 9 | 45 |
| 616735 | 7406 | 7423 | GCAGTCTCTGCCTCGTTT | Exon 9 | 46 |
| 616736 | 7410 | 7427 | GGCAGCAGTCTCTGCCTC | Exon 9 | 47 |
| 616737 | 7414 | 7431 | GCCGGGCAGCAGTCTCTG | Exon 9 | 48 |
| 616738 | 7418 | 7435 | GGCTGCCGGGCAGCAGTC | Exon 9 | 49 |
| 616739 | 7422 | 7439 | GAGAGGCTGCCGGGCAGC | Exon 9 | 50 |
| 616740 | 7426 | 7443 | CTATGAGAGGCTGCCGGG | Exon 9 | 51 |
| 616741 | 7430 | 7447 | GTGCCTATGAGAGGCTGC | Exon 9 | 52 |
| 616742 | 7434 | 7451 | CTCGGTGCCTATGAGAGG | Exon 9 | 53 |
| 616743 | 7438 | 7455 | GGGTCTCGGTGCCTATGA | Exon 9 | 54 |
| 616744 | 7454 | 7471 | CCTGGCTTTGACTCTGGG | Exon 9/Intron 9 | 55 |
| 616745 | 7458 | 7475 | CCTACCTGGCTTTGACTC | Exon 9/Intron 9 | 56 |
| 616746 | 7462 | 7479 | GCATCCTACCTGGCTTTG | Exon 9/Intron 9 | 57 |
| 616747 | 7466 | 7483 | CTTTGCATCCTACCTGGC | Exon 9/Intron 9 | 58 |
| 616748 | 7470 | 7487 | GGGCCTTTGCATCCTACC | Exon 9/Intron 9 | 59 |
| 616749 | 7474 | 7491 | GGGAGGGCCTTTGCATCC | Intron 9 | 60 |

### Example 7: Antisense Oligonucleotide Induction of CLN3 Exon 9 Skipping in vitro

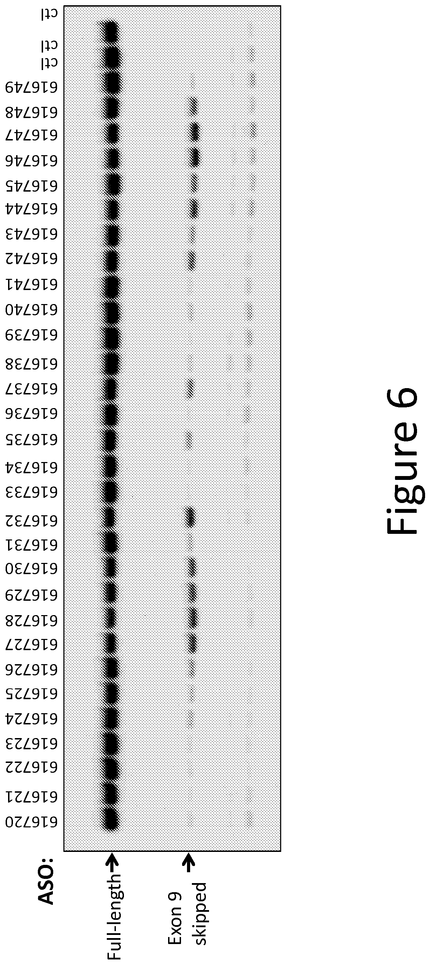

To test the ability of the antisense oligonucleotides to modulate splicing by skipping exon 9, mouse cell line 208ee, derived from C57/B16 mouse kidney, was transfected with 50 nM of the ASOs listed in Table 5 using Lipofectamine 2000 (Invitrogen). Untreated controls cells received neither ASO nor Lipofectamine, while mock transfected cells received only Lipofectamine. After 48 hours, total RNA was collected from the cells and RT-PCR was used to identify CLN3 full-length and CLN3Δ9 transcripts. The PCR products were analyzed by PAGE and quantitated by phosphorimager analysis (Typhoon 9400, GE Healthcare). The gel is shown in Figure 6, and the results are shown in Table 6 as the percentage of exon 9 skipped (Δ9) transcript out of the total full-length (FL) plus exon 9 skipped transcripts. Band intensities are reported in relative fluorescence units (RFU). This example illustrates that many ASOs induce CLN3 exon 9 skipping.

**Table 6: Induction of CLN3 Exon 9 Skipping in vitro**

| ISIS NO | Full-length CLN3 (RFU) | CLN3Δ9 (RFU) | Δ9/(FL + Δ9) (%) |
|---|---|---|---|
| untreated control | 9,181,862 | 123,203 | 1.32 |
| mock | 11,512,749 | 112,061 | 0.96 |
| 616720 | 8,594,979 | 360,150 | 4.02 |
| 616721 | 8,065,276 | 236,557 | 2.85 |
| 616722 | 9,180,522 | 336,620 | 3.54 |
| 616723 | 9,434,304 | 341,373 | 3.49 |
| 616724 | 8,253,838 | 790,632 | 8.74 |
| 616725 | 7,304,964 | 704,209 | 8.79 |
| 616726 | 7,557,368 | 1,175,984 | 13.47 |
| 616727 | 5,415,612 | 3,045,762 | 36.00 |
| 616728 | 4,294,429 | 3,391,413 | 44.13 |
| 616729 | 5,530,907 | 2,760,074 | 33.29 |
| 616730 | 5,419,114 | 2,431,785 | 30.97 |
| 616731 | 8,003,964 | 792,062 | 9.00 |
| 616732 | 5,661,965 | 3,374,571 | 37.34 |
| 616733 | 8,423,448 | 221,903 | 2.57 |
| 616734 | 7,865,161 | 226,765 | 2.80 |
| 616735 | 7,533,174 | 1,036,100 | 12.09 |
| 616736 | 8,566,723 | 261,101 | 2.96 |
| 616737 | 7,177,269 | 1,539,887 | 17.67 |
| 616738 | 9,536,684 | 433,505 | 4.35 |
| 616739 | 10,062,225 | 213,983 | 2.08 |
| 616740 | 9,458,260 | 522,205 | 5.23 |
| 616741 | 9,741,862 | 294,485 | 2.93 |
| 616742 | 6,988,392 | 1,994,800 | 22.21 |
| 616743 | 7,293,927 | 1,125,023 | 13.36 |
| 616744 | 6,063,867 | 2,719,387 | 30.96 |
| 616745 | 7,885,435 | 1,812,122 | 18.69 |
| 616746 | 6,220,132 | 3,850,348 | 38.23 |
| 616747 | 6,181,617 | 3,699,891 | 37.44 |
| 616748 | 7,778,346 | 2,210,461 | 22.13 |
| 616749 | 10,815,904 | 467,192 | 4.14 |

### Example 8: Antisense Oligonucleotide Induction of CLN3 Exon 6 Skipping in Batten Mice in vivo

A mouse model of Batten Disease, in which the *CLN3Δ78* deletion is knocked-in to C57/BL6J mice, has been described (Cotman et al., Hum. Molec. Genet. (2002) 11, 2709-2721). The CLN3Δ78/Δ78 homozygous mice exhibit gliosis in the CNS, abnormal gait traces, clasping behavior indicating neurodegeneration, and early PT-10.

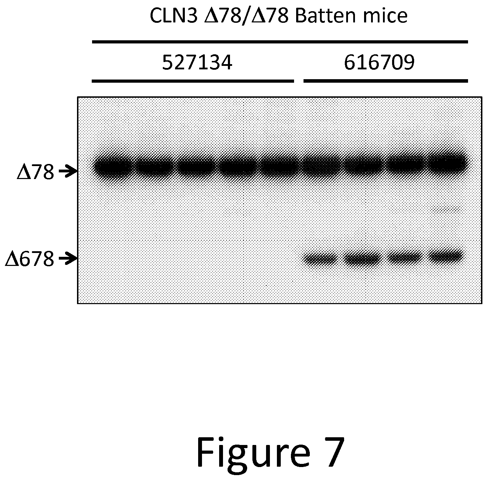

20 µg of Isis No. 616709 (see Table 3) or a control ASO, Isis No. 527134 (AGCTGATCATATTCTACC SEQ ID NO: 61) that is uniform 2'-MOE and contains uniform phosphorothioate internucleoside linkages, was administered to groups of six CLN3Δ78/Δ78 mice to test the ability of Isis No. 616709 to induce exon 6 skipping *in vivo.* The ASOs were administered by intracerebroventricular injection (ICV injection) on either post-natal day one or two. Four months after the single ICV injection, the mice were euthanized and RNA was isolated from brain tissue. *CLN3Δ78* and CLN3Δ678 transcripts were analyzed by radiolabeled RT-PCR. The PCR products were separated by PAGE and quantitated by phosphorimager analysis (Typhoon 9400, GE Healthcare). A gel is shown in Figure 7, and the results for each group are presented in Table 7 below. As indicated by the results in Table 7 below, ISIS NO. 616709 induced skipping of CLN3 exon 6 in a mouse model of Batten Disease *in vivo.*

**Table 7: Induction of CLN3 Exon 6 Skipping in CLN3Δ78/Δ78 mice in vivo**

| ISIS NO. | Δ678/(Δ678 + Δ78) (%) | Std Dev |
|---|---|---|
| 527134 | 3.70 | 5.30 |
| 616709 | 33.72 | 7.18 |

### Example 9: Antisense Oligonucleotide Rescue of Motor Coordination in CLN3Δ78/Δ78 Mice

In order to test whether ASOs that induce exon 6 skipping relieve symptoms of Batten Disease, homozygous CLN3Δ78/Δ78 and heterozygous CLN3+/Δ78 mice were divided into mixed gender and male only groups and treated with 20 µg of Isis NO. 616709 (SEQ ID NO. 20; *see* Table 3), control ASO, Isis No. 527134 (SEQ ID NO: 61; *see* Example 8), or no ASO (untreated). The ASOs were administered by intracerebroventricular injection on post-natal day one or two. Two or three months after the single injection, motor coordination of each mouse was measured based on the time each mouse spent on an accelerating rotarod. The results are presented in Tables 8-12 below.

**Table 8: Time Spent on an Accelerating Rotarod by Mixed Gender CLN3Δ78/Δ78 Mice, Age 2 Months**

| Isis No. | Genotype | No. of Mice in Group | Average Time Spent on Rotarod (seconds) | Std Dev. |
|---|---|---|---|---|
| Untreated | CLN3Δ78/Δ78 | 28 | 82.7 | 25.8 |
| 527134 | CLN3Δ78/Δ78 | 21 | 81.5 | 20.8 |
| 616709 | CLN3Δ78/Δ78 | 21 | 84.7 | 14.5 |
| Untreated | CLN3+/Δ78 | 22 | 91.6 | 19.7 |
| 527134 | CLN3+/Δ78 | 17 | 98.2 | 39.2 |
| 616709 | CLN3+/Δ78 | 31 | 99.8 | 22.9 |

**Table 9: Time Spent on an Accelerating Rotarod by Mixed Gender CLN3Δ78/Δ78 Mice, Age 3 Months**

| Isis No. | Genotype | No. of Mice in Group | Average Time Spent on Rotarod (seconds) | Std Dev. |
|---|---|---|---|---|
| Untreated | CLN3Δ78/Δ78 | 9 | 51.8 | 17.6 |
| 527134 | CLN3Δ78/Δ78 | 8 | 66.1 | 15.3 |
| 616709 | CLN3Δ78/Δ78 | 9 | 81.2 | 12.8 |
| Untreated | CLN3+/Δ78 | 8 | 70.3 | 14.6 |
| 527134 | CLN3+/Δ78 | 7 | 68.8 | 13.4 |
| 616709 | CLN3+/Δ78 | 13 | 96.0 | 29.3 |

**Table 10: Time Spent on an Accelerating Rotarod by Male CLN3Δ78/Δ78 Mice, Age 2 Months**

| Isis No. | Genotype | No. of Mice in Group | Average Time Spent on Rotarod (seconds) | Std Dev. |
|---|---|---|---|---|
| Untreated | CLN3Δ78/Δ78 | 13 | 68.1 | 19.3 |
| 527134 | CLN3Δ78/Δ78 | 5 | 74.3 | 15.2 |
| 616709 | CLN3Δ78/Δ78 | 12 | 84.1 | 14.3 |
| Untreated | CLN3+/Δ78 | 12 | 94.0 | 19.7 |
| 527134 | CLN3+/Δ78 | 8 | 108.2 | 47.5 |
| 616709 | CLN3+/Δ78 | 14 | 103.2 | 23.7 |

**Table 11: Time Spent on an Accelerating Rotarod by Male CLN3Δ78/Δ78 Mice, Age 3 Months**

| Isis No. | Genotype | No. of Mice in Group | Average Time Spent on Rotarod (seconds) | Std Dev. |
|---|---|---|---|---|
| Untreated | CLN3Δ78/Δ78 | 4 | 39.3 | 9.5 |
| 527134 | CLN3Δ78/Δ78 | 1 | 36.4 | n/a |
| 616709 | CLN3Δ78/Δ78 | 4 | 75.3 | 9.9 |
| Untreated | CLN3+/Δ78 | 8 | 70.3 | 14.6 |
| 527134 | CLN3+/Δ78 | 3 | 75.4 | 11.8 |
| 616709 | CLN3+/Δ78 | 7 | 117.4 | 15.8 |

The results in Tables 8-11 above indicate that the motor coordination deficit in the CLN3Δ78/Δ78 Batten mice worsens significantly between two and three months of age and is more severe in males than females. Furthermore, treatment with Isis No. 616709, which induces exon 6 skipping, increases the amount of time CLN3Δ78/Δ78 Batten mice are able to spend on an accelerating rotarod, thereby improving a motor coordination deficit that is symptomatic of Batten Disease.

### Example 10: Antisense Oligonucleotide Induction of Human CLN3 Exon 6 Skipping in vitro

Antisense oligonucleotides complementary to exon 6 and/or the introns flanking exon 6 of the human CLN3 pre-mRNA transcript were synthesized and tested for their ability to modulate splicing of CLN3 by skipping exon 6. The oligonucleotides in Table 12 below are uniformly 2'-MOE modified, and all internucleoside linkages are phosphorothioate linkages. The cytosine bases are 5-methylcytosine. The ASOs target the complement of GENBANK accession number NT_010393.16, truncated from nucleotides 28427600 to 28444620 (SEQ ID NO: 1), and the ASO sequences are listed in Table 12 below. The start and stop sites associated with each oligonucleotide are the 5'- and 3'-positions, respectively, of the portion of SEQ ID NO: 1 that is complementary to the ASO.

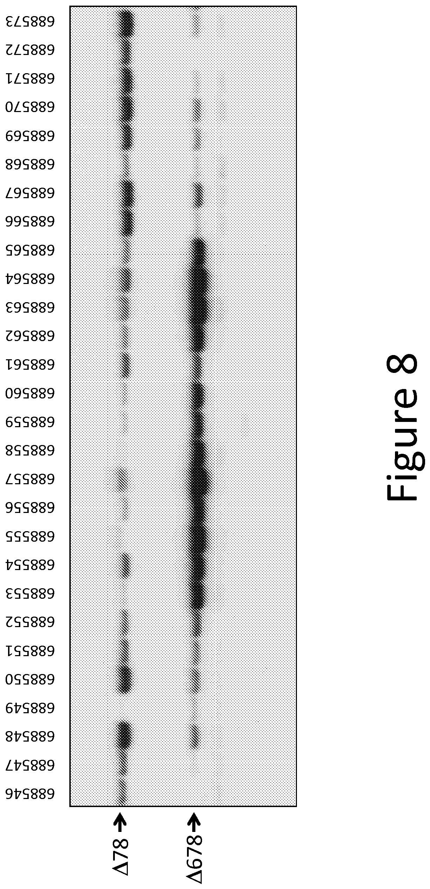

To test the ability of the antisense oligonucleotides to modulate splicing by skipping exon 6, human JNCL patient fibroblasts that are homozygous for the CLN3Δ78 deletion were transfected with 50 nM of an ASO using Cytofectin transfection reagent. After 48 hours, total RNA was collected from the cells and RT-PCR was used to identify the CLN3Δ78 and CLN3Δ678 mRNA transcripts. The PCR products were analyzed by PAGE and quantitated by phosphorimager analysis (Typhoon 9400, GE Healthcare). The gel is shown in Figure 8, and the results are shown in Table 12 below as the ratios of exon 6, 7, and 8 skipped (Δ678) transcript to exon 7 and 8 skipped (Δ78) transcript. The results below illustrate that many ASOs induced CLN3 exon 6 skipping.

**Table 12: CLN3 Exon 6 Skipping in patient fibroblasts**

| ISIS No. | Start Site | Stop Site | Sequence (5' to 3') | CLN3 Target Region | CLN3Δ678/CLN3Δ78 | SEQ ID NO |
|---|---|---|---|---|---|---|
| 688546 | 5699 | 5716 | ACTCTCAGCATCTCAGCC | Intron 5 | 0.00 | 63 |
| 688547 | 5704 | 5721 | TCTCTACTCTCAGCATCT | Intron 5 | 0.00 | 64 |
| 688548 | 5709 | 5726 | GTCGGTCTCTACTCTCAG | Intron 5 | 0.25 | 65 |
| 688549 | 5714 | 5731 | GGAAGGTCGGTCTCTACT | Intron 5 | 1.38 | 66 |
| 688550 | 5734 | 5751 | GGTGAGAAGGGAAGGGAG | Intron 5 | 0.37 | 67 |
| 688551 | 5764 | 5781 | TCCCACTGACGAGAACCC | Exon 6 | 0.74 | 68 |
| 688552 | 5769 | 5786 | ACAAATCCCACTGACGAG | Exon 6 | 1.58 | 69 |
| 688553 | 5774 | 5791 | GCAGCACAAATCCCACTG | Exon 6 | 14.77 | 70 |
| 688554 | 5779 | 5796 | TTCCAGCAGCACAAATCC | Exon 6 | 3.68 | 71 |
| 688555 | 5784 | 5801 | GAAGCTTCCAGCAGCACA | Exon 6 | 31.07 | 72 |
| 688556 | 5789 | 5806 | AGGACGAAGCTTCCAGCA | Exon 6 | 9.28 | 73 |
| 688557 | 5794 | 5811 | CAACCAGGACGAAGCTTC | Exon 6 | 13.03 | 74 |
| 688558 | 5799 | 5816 | AAAGGCAACCAGGACGAA | Exon 6 | 24.37 | 75 |
| 688559 | 5804 | 5821 | TGAGAAAAGGCAACCAGG | Exon 6 | 10.29 | 76 |
| 688560 | 5809 | 5826 | CAGAATGAGAAAAGGCAA | Exon 6 | 8.44 | 77 |
| 688561 | 5814 | 5831 | CCCCACAGAATGAGAAAA | Exon 6 | 1.28 | 78 |
| 688562 | 5819 | 5836 | CTGGTCCCCACAGAATGA | Exon 6 | 7.32 | 79 |
| 688563 | 5824 | 5841 | ACAGGCTGGTCCCCACAG | Exon 6 | 8.32 | 80 |
| 688564 | 5829 | 5846 | ACCACACAGGCTGGTCCC | Exon 6/Intron 6 | 6.52 | 81 |
| 688565 | 5834 | 5851 | CACTCACCACACAGGCTG | Exon 6/Intron 6 | 4.56 | 82 |
| 688566 | 5839 | 5856 | CCACACACTCACCACACA | Exon 6/Intron 6 | 0.16 | 83 |
| 688567 | 5844 | 5861 | CAGAACCACACACTCACC | Intron 6 | 0.41 | 84 |
| 688568 | 5849 | 5866 | TGACACAGAACCACACAC | Intron 6 | 0.46 | 85 |
| 688569 | 5854 | 5871 | CCATCTGACACAGAACCA | Intron 6 | 0.33 | 86 |
| 688570 | 5859 | 5876 | GCTCCCCATCTGACACAG | Intron 6 | 0.27 | 87 |
| 688571 | 5879 | 5896 | CTCTGATGTGGTTCCTCG | Intron 6 | 0.11 | 88 |
| 688572 | 5884 | 5901 | AAATGCTCTGATGTGGTT | Intron 6 | 0.10 | 89 |
| 688573 | 5889 | 5906 | CCCACAAATGCTCTGATG | Intron 6 | 0.12 | 90 |

### Example 11: Antisense Oligonucleotide Induction of Human CLN3 Exon 9 Skipping in vitro

Antisense oligonucleotides complementary to exon 9 and/or the introns flanking exon 9 of the human CLN3 pre-mRNA transcript were synthesized and tested for their ability to modulate splicing of CLN3 by skipping exon 9. The oligonucleotides were uniformly modified to contain 2'-MOE, and all internucleoside linkages are phosphorothioate linkages. The cytosine bases are 5-methylcytosine. The ASOs target the complement of GENBANK accession number NT_010393.16, truncated from nucleotides 28427600 to 28444620 (SEQ ID NO: 1), and the ASO sequences are listed in Table 13 below. The start and stop sites associated with each oligonucleotide are the 5'- and 3'-positions, respectively, of the portion of SEQ ID NO: 1 that is complementary to the ASO.

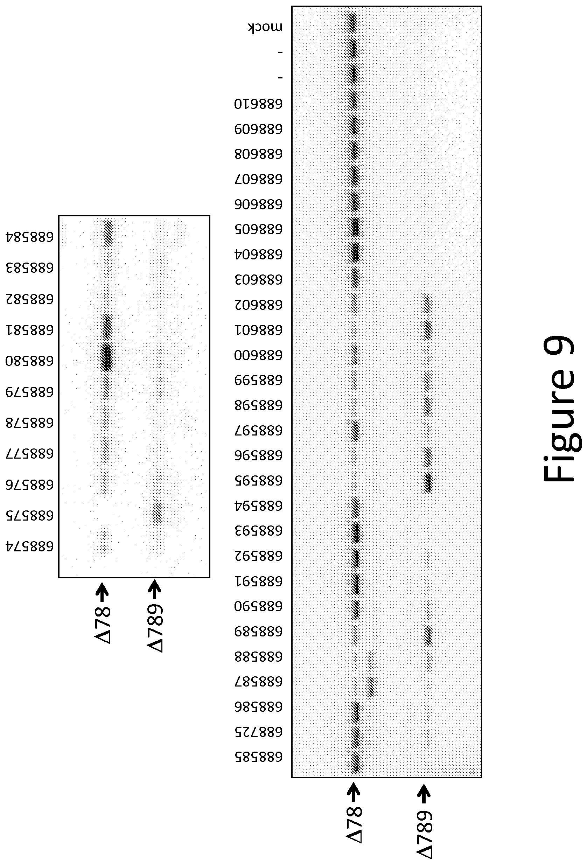

To test the ability of the antisense oligonucleotides to modulate splicing by skipping exon 9, human JNCL patient fibroblasts that are homozygous for the CLN3Δ78 deletion were transfected with 50 nM of an ASO using Cytofectin transfection reagent or were mock transfected. After 48 hours, total RNA was collected from the cells and RT-PCR was used to identify the CLN3Δ78 and CLN3Δ789 mRNA transcripts. The PCR products were analyzed by PAGE and quantitated by phosphorimager analysis (Typhoon 9400, GE Healthcare). The gel is shown in Figure 9, and the results are shown in Table 13 below as the ratios of exon 7, 8, and 9 skipped (Δ789) transcript to exon 7 and 8 skipped (Δ78) transcript. The results below illustrate that many ASOs induced CLN3 exon 9 skipping to a greater extent than the exon 9 skipping observed in mock transfected cells.

**Table 13: CLN3 Exon 9 Skipping in patient fibroblasts**

| ISIS No. | Start Site | Stop Site | Sequence (5' to 3') | CLN3 Target Region | CLN3Δ789/CLN3Δ78 | SEQ ID NO |
|---|---|---|---|---|---|---|
| mock | n/a | n/a | n/a | n/a | 0.05 | n/a |
| 688574 | 9122 | 9139 | GGGAAGGTCCCCAGGGAC | Intron 8 | 1.14 | 91 |
| 688575 | 9127 | 9144 | TGGCTGGGAAGGTCCCCA | Intron 8 | 48.56 | 92 |
| 688576 | 9132 | 9149 | CCTACTGGCTGGGAAGGT | Intron 8 | 1.59 | 93 |
| 688577 | 9137 | 9154 | AAAACCCTACTGGCTGGG | Intron 8 | 0.46 | 94 |
| 688578 | 9142 | 9159 | GTCAGAAAACCCTACTGG | Intron 8 | 0.46 | 95 |
| 688579 | 9147 | 9164 | GCAGGGTCAGAAAACCCT | Intron 8 | 0.89 | 96 |
| 688580 | 9152 | 9169 | TGAAGGCAGGGTCAGAAA | Intron 8 | 0.20 | 97 |
| 688581 | 9157 | 9174 | TAGGATGAAGGCAGGGTC | Intron 8 | 0.08 | 98 |
| 688582 | 9162 | 9179 | AGGAGTAGGATGAAGGCA | Intron 8 | 0.79 | 99 |
| 688583 | 9167 | 9184 | TAGCTAGGAGTAGGATGA | Intron 8/Exon 9 | 1.05 | 100 |
| 688584 | 9172 | 9189 | AGAAATAGCTAGGAGTAG | Intron 8/Exon 9 | 0.22 | 101 |
| 688585 | 9177 | 9194 | CAACAAGAAATAGCTAGG | Intron 8/Exon 9 | 0.08 | 102 |
| 616725 | 9182 | 9199 | GTGAGCAACAAGAAATAG | Exon 9 | 0.29 | 36 |
| 688586 | 9187 | 9204 | GAGATGTGAGCAACAAGA | Exon 9 | 0.24 | 103 |
| 688587 | 9192 | 9209 | CTCAGGAGATGTGAGCAA | Exon 9 | 0.92 | 104 |
| 688588 | 9197 | 9214 | TGGGCCTCAGGAGATGTG | Exon 9 | 2.73 | 105 |
| 688589 | 9202 | 9219 | GGTCCTGGGCCTCAGGAG | Exon 9 | 2.73 | 106 |
| 688590 | 9207 | 9224 | TCCAGGGTCCTGGGCCTC | Exon 9 | 0.57 | 107 |
| 688591 | 9212 | 9229 | TCCCCTCCAGGGTCCTGG | Exon 9 | 0.14 | 108 |
| 688592 | 9217 | 9234 | CTTCTTCCCCTCCAGGGT | Exon 9 | 0.23 | 109 |
| 688593 | 9222 | 9239 | TGCTTCTTCTTCCCCTCC | Exon 9 | 0.06 | 110 |
| 688594 | 9227 | 9244 | CTCTCTGCTTCTTCTTCC | Exon 9 | 0.07 | 111 |
| 688595 | 9232 | 9249 | CTGCGCTCTCTGCTTCTT | Exon 9 | 8.80 | 112 |
| 688596 | 9237 | 9254 | CCGGGCTGCGCTCTCTGC | Exon 9 | 4.76 | 113 |
| 688597 | 9242 | 9259 | GGCTGCCGGGCTGCGCTC | Exon 9 | 0.51 | 114 |
| 688598 | 9262 | 9279 | GGGCCTCGGTTCTTATGA | Exon 9 | 3.74 | 115 |
| 688599 | 9282 | 9299 | CCTACCTGGCTTCGACTC | Exon 9/Intron 9 | 2.30 | 116 |
| 688600 | 9287 | 9304 | TGTCTCCTACCTGGCTTC | Exon 9/Intron 9 | 0.79 | 117 |
| 688601 | 9292 | 9309 | GTCTGTGTCTCCTACCTG | Exon 9/Intron 9 | 5.10 | 118 |
| 688602 | 9297 | 9314 | TGAGGGTCTGTGTCTCCT | Intron 9 | 1.75 | 119 |
| 688603 | 9302 | 9319 | CTCTCTGAGGGTCTGTGT | Intron 9 | 0.08 | 120 |
| 688604 | 9307 | 9324 | GTGACCTCTCTGAGGGTC | Intron 9 | 0.03 | 121 |
| 688605 | 9312 | 9329 | AGAAAGTGACCTCTCTGA | Intron 9 | 0.05 | 122 |
| 688606 | 9317 | 9334 | GAGAAAGAAAGTGACCTC | Intron 9 | 0.07 | 123 |
| 688607 | 9322 | 9339 | CCAGAGAGAAAGAAAGTG | Intron 9 | 0.05 | 124 |
| 688608 | 9327 | 9344 | CAAACCCAGAGAGAAAGA | Intron 9 | 0.08 | 125 |
| 688609 | 9332 | 9349 | AAGGCCAAACCCAGAGAG | Intron 9 | 0.02 | 126 |
| 688610 | 9337 | 9354 | AGGAAAAGGCCAAACCCA | Intron 9 | 0.01 | 127 |

### Example 12: Dose Response of Antisense Oligonucleotide Induction of Human CLN3 Exon Skipping

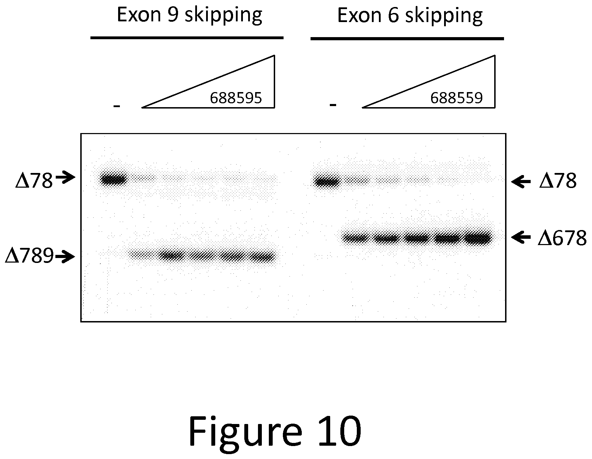

JNCL patient fibroblasts were transfected with Isis No. 688555, 688559 (see Table 12), or Isis No. 688595 (see Table 13) at a concentration listed in Table 14 below using Endo-porter transfection reagent (Gene Tools, LLC). After 48 hours, total RNA was isolated from the cells and CLN3 mRNA was detected as described in Examples 10 and 11. The gel is shown in Figure 10. The results are shown in Table 14 below and show that all three ASOs tested induced exon skipping in a dose dependent manner. "n/a" indicates that the ASO was not tested at the indicated concentration.

**Table 14: Dose Responses of an Exon 6 Skipping ASO and an Exon 9 Skipping ASO in Patient Cells**

| Concentration (nM) | CLN3Δ678/CLN3Δ78 | | CLN3Δ789/CLN3Δ78 Isis No. 688595 |
|---|---|---|---|
| | Isis No. 688555 | Isis No. 688559 | |
| 0 | 0.00 | 0.01 | 0.04 |
| 0.39 | 0.37 | n/a | n/a |
| 0.78 | 1.13 | n/a | n/a |
| 1.56 | 4.31 | n/a | n/a |
| 3.125 | 15.24 | n/a | n/a |
| 12.5 | n/a | 3.46 | 3.84 |
| 25 | n/a | 7.36 | 19.34 |
| 50 | n/a | 11.88 | 23.16 |
| 100 | n/a | 23.66 | 21.31 |
| 200 | n/a | 70.91 | 27.9 |

### Example 13: Performance in a Water Maze by CLN3Δ78/Δ78 Mice Following ASO Treatment

In order to test whether an ASO that induces exon 6 skipping relieves symptoms of Batten Disease, homozygous CLN3Δ78/Δ78, heterozygous CLN3+/Δ78, and wild type mice were treated with 20 µg of Isis No. 616709 (see Table 3), Isis No. 527134 as a control (see Example 8), or no ASO (untreated). The ASOs were administered by intracerebroventricular injection on post-natal day one or two. Two or three months after the single injection, performance in the Morris water maze was measured based on the time each mouse took to swim to a hidden platform. Mice were placed individually in a circular pool, 48 inches in diameter, filled to a depth of 26 inches with 23 °C water. The pool walls were made opaque with white paint and placed in a room with prominent extra-maze cues at elast 16 inches from the pool edge. Four unique, proximal cues were affixed to the 8 cm high interior pool wall above water level at 0, 90, 180, and 270 degrees. Mice were placed in one of four starting quadrants facing the pool wall and allowed to swim until coming to rest atop a 4 inch square plexiglass platform submerged in 0.5 cm of water, or until a maximum of 60 seconds. Upon finding a platform, mice were left there for 20 seconds before reentry at the next start point or removal from the cage. Mice that did not find the platform within 60 seconds were guided to it by the experimenter. Trials were performed once at each starting quadrant point per session. Mice were tested for four consecutive days with two sessions of four trials each per day, and the time to reach a hidden platform was recorded. Each treatment group consisted of 13-35 mice. The results for each treatment group are shown in Table 15 below. The results show that CLN3Δ78/Δ78 homozygous mice that received an antisense oligonucleotide that induces exon 6 skipping performed similarly to WT or heterozygous mice and performed significantly better than CLN3Δ78/Δ78 homozygous mice that were untreated or received the control ASO. In particular, the differences between the control CLN3Δ78/Δ78 homozygous mice and the ASO treated CLN3Δ78/Δ78 homozygous mice and WT and heterozygous controls were significant, with p values ≤0.01 on days 2 and 4.

**Table 15: Time to Reach a Hidden Platform in a Morris Water Maze**

| Isis No. | Genotype | Number of mice in group | Trial day | Time to Platform(s) | Std. Dev. |
|---|---|---|---|---|---|
| Untreated | WT or CLN3+/Δ78 | 21 | 1 | 44.8 | 10.5 |
| | | | 2 | 24.9 | 12.0 |
| | | | 3 | 17.2 | 8.3 |
| | | | 4 | 13.5 | 7.6 |
| Untreated or 527134 | CLN3A78/A78 | 35 | 1 | 48.9 | 10.0 |
| | | | 2 | 35.9 | 14.5 |
| | | | 3 | 27.4 | 12.7 |
| | | | 4 | 26.0 | 14.2 |
| 616709 | CLN3A78/A78 | 13 | 1 | 43.0 | 7.6 |
| | | | 2 | 19.3 | 7.5 |
| | | | 3 | 24.1 | 12.9 |
| | | | 4 | 16.8 | 8.4 |

## Claims

1. A compound comprising a modified oligonucleotide consisting of 8 to 30 linked nucleosides and having a nucleobase sequence comprising a complementary region, wherein the complementary region comprises at least 8 contiguous nucleobases and is 100% complementary to an equal-length portion of a target region of a CLN3 transcript, wherein:
(i) the nucleobase sequence of the oligonucleotide is at least 90% complementary to an equal-length region of the CLN3 transcript, as measured over the entire length of the oligonucleotide; and
(ii) the modified oligonucleotide does not comprise more than 4 contiguous unmodified 2'deoxynucleosides; and
wherein the compound is able to induce skipping of one or more exons of the CLN3 transcript,
for use in the treatment of Batten Disease or for use in delaying or preventing the onset of Batten Disease.

2. The compound for use according to claim 1, wherein the target region of the CLN3 transcript comprises at least a portion of exon 6, exon 9, intron 5, intron 6, intron 9, or intron 10 of the CLN3 transcript, and wherein optionally the compound is able to prevent the frameshift that results in a premature stop codon in exon 9 of the CLN3 transcript.

3. The compound for use according to claim 1 or claim 2, wherein the complementary region of the modified oligonucleotide comprises at least 10, at least 12, at least 14, at least 15, at least 16, at least 17, at least 18, at least 19, or at least 20 contiguous nucleobases.

4. The compound for use according to any of claims 1-3, wherein the nucleobase sequence of the oligonucleotide is 100% complementary to an equal-length region of the CLN3 transcript, as measured over the entire length of the oligonucleotide.

5. The compound for use according to any of claims 1-4, wherein the modified oligonucleotide comprises at least one modified nucleoside.

6. The compound for use according to claim 5, wherein at least one modified nucleoside comprises a modified sugar moiety.

7. The compound for use according to claim 6, wherein at least one modified sugar moiety is a 2'-substituted sugar moiety.

8. The compound for use according to claim 7, wherein the 2'-substitutent of at least one 2'-substituted sugar moiety is selected from among: 2'-OMe, 2'-F, and 2'-MOE.

9. The compound for use according to any of claims 1-8, wherein the modified oligonucleotide comprises at least 5 modified nucleosides, at least 10 modified nucleosides, or at least 15 modified nucleosides, each independently comprising a modified sugar moiety.

10. The compound for use according to claim 9, wherein each nucleoside of the modified oligonucleotide is a modified nucleoside, each independently comprising a modified sugar moiety.

11. The compound for use according to claim 9 or claim 10, wherein each modified sugar moiety is a 2'-MOE.

12. A pharmaceutical composition comprising a compound comprising a modified oligonucleotide consisting of 8 to 30 linked nucleosides and having a nucleobase sequence comprising a complementary region, wherein the complementary region comprises at least 8 contiguous nucleobases and is 100% complementary to an equal-length portion of a target region of a CLN3 transcript, wherein:
(i) the nucleobase sequence of the oligonucleotide is at least 90% complementary to an equal-length region of the CLN3 transcript, as measured over the entire length of the oligonucleotide; and
(ii) the modified oligonucleotide does not comprise more than 4 contiguous unmodified 2'deoxynucleosides; and
wherein the compound is able to induce skipping of one or more exons of the CLN3 transcript, and a pharmaceutically acceptable carrier or diluent,
for use in the treatment of Batten Disease or for use in delaying or preventing the onset of Batten Disease.

13. The pharmaceutical composition for use according to claim 12, wherein the composition comprises a pharmaceutically acceptable salt of the compound.

14. The pharmaceutical composition for use according to claim 13, wherein the pharmaceutically acceptable salt is a sodium or potassium salt.

## Patentansprüche

1. Verbindung, umfassend ein modifiziertes Oligonukleotid, das aus 8 bis 30 verknüpften Nukleosiden besteht und eine Nukleobasensequenz aufweist, umfassend eine komplementäre Region, wobei die komplementäre Region mindestens 8 zusammenhängende Nukleobasen umfasst und zu einem gleich langen Teil einer Zielregion eines CLN3-Transkripts zu 100% komplementär ist, wobei:
(i) die Nukleobasensequenz des Oligonukleotids zu einer gleich langen Region des CLN3-Transkripts zu mindestens 90% komplementär ist, gemessen über die gesamte Länge des Oligonukleotids hinweg; und
(ii) das modifizierte Oligonukleotid nicht mehr als 4 zusammenhängende unmodifizierte 2'-Desoxynukleoside umfasst; und
wobei die Verbindung in der Lage ist, das Überspringen eines oder mehrerer Exons des CLN3-Transkripts zu induzieren,
zur Verwendung bei der Behandlung von Morbus Batten oder zur Verwendung bei der Verzögerung oder Verhinderung des Ausbruchs von Morbus Batten.

2. Verbindung zur Verwendung nach Anspruch 1, wobei die Zielregion des CLN3-Transkripts mindestens einen Teil von Exon 6, Exon 9, Intron 5, Intron 6, Intron 9 oder Intron 10 des CLN3-Transkripts umfasst und wobei optional die Verbindung in der Lage ist, den Leserasterschub zu verhindern, der zu einem vorzeitigen Stoppcodon im Exon 9 des CLN3-Transkripts führt.

3. Verbindung zur Verwendung nach Anspruch 1 oder Anspruch 2, wobei die komplementäre Region des modifizierten Oligonukleotids mindestens 10, mindestens 12, mindestens 14, mindestens 15, mindestens 16, mindestens 17, mindestens 18, mindestens 19 oder mindestens 20 zusammenhängende Nukleobasen umfasst.

4. Verbindung zur Verwendung nach einem der Ansprüche 1-3, wobei die Nukleobasensequenz des Oligonukleotids zu einer gleich langen Region des CLN3-Transkripts zu 100% komplementär ist, gemessen über die gesamte Länge des Oligonukleotids hinweg.

5. Verbindung zur Verwendung nach einem der Ansprüche 1-4, wobei das modifizierte Oligonukleotid mindestens ein modifiziertes Nukleosid umfasst.

6. Verbindung zur Verwendung nach Anspruch 5, wobei mindestens ein modifiziertes Nukleosid einen modifizierten Zuckerrest umfasst.

7. Verbindung zur Verwendung nach Anspruch 6, wobei es sich bei mindestens einem modifizierten Zuckerrest um einen 2'-substituierten Zuckerrest handelt.

8. Verbindung zur Verwendung nach Anspruch 7, wobei der 2'-Substituent mindestens eines 2'-substituierten Zuckerrests ausgewählt ist aus: 2'-OMe, 2'-F und 2'-MOE.

9. Verbindung zur Verwendung nach einem der Ansprüche 1-8, wobei das modifizierte Oligonukleotid mindestens 5 modifizierte Nukleoside, mindestens 10 modifizierte Nukleoside oder mindestens 15 modifizierte Nukleoside umfasst, die jeweils unabhängig einen modifizierten Zuckerrest umfassen.

10. Verbindung zur Verwendung nach Anspruch 9, wobei es sich bei jedem Nukleosid des modifizierten Oligonukleotids um ein modifiziertes Nukleosid handelt, das jeweils unabhängig einen modifizierten Zuckerrest umfasst.

11. Verbindung zur Verwendung nach Anspruch 9 oder Anspruch 10, wobei es sich bei jedem modifizierten Zuckerrest um ein 2'-MOE handelt.

12. Pharmazeutische Zusammensetzung, die eine Verbindung umfasst, umfassend ein modifiziertes Oligonukleotid, das aus 8 bis 30 verknüpften Nukleosiden besteht und eine Nukleobasensequenz aufweist, umfassend eine komplementäre Region, wobei die komplementäre Region mindestens 8 zusammenhängende Nukleobasen umfasst und zu einem gleich langen Teil einer Zielregion eines CLN3-Transkripts zu 100% komplementär ist, wobei:
(i) die Nukleobasensequenz des Oligonukleotids zu einer gleich langen Region des CLN3-Transkripts zu mindestens 90% komplementär ist, gemessen über die gesamte Länge des Oligonukleotids hinweg; und
(ii) das modifizierte Oligonukleotid nicht mehr als 4 zusammenhängende unmodifizierte 2'-Desoxynukleoside umfasst; und
wobei die Verbindung in der Lage ist, das Überspringen eines oder mehrerer Exons des CLN3-Transkripts zu induzieren,
und einen pharmazeutisch annehmbaren Träger oder Verdünner,
zur Verwendung bei der Behandlung von Morbus Batten oder zur Verwendung bei der Verzögerung oder Verhinderung des Ausbruchs von Morbus Batten.

13. Pharmazeutische Zusammensetzung zur Verwendung nach Anspruch 12, wobei die Zusammensetzung ein pharmazeutisch annehmbares Salz der Verbindung umfasst.

14. Pharmazeutische Zusammensetzung zur Verwendung nach Anspruch 13, wobei das pharmazeutisch annehmbare Salz ein Natrium- oder Kaliumsalz ist.

## Revendications

1. Composé comprenant un oligonucléotide modifié constitué de 8 à 30 nucléosides liés et ayant une séquence de bases azotées comprenant une région complémentaire, dans lequel la région complémentaire comprend au moins 8 bases azotées contiguës et est 100 % complémentaire d'une partie de longueur égale d'une région cible d'un produit de transcription de CLN3, dans lequel :
(i) la séquence de bases azotées de l'oligonucléotide est au moins 90 % complémentaire d'une région de longueur égale du produit de transcription de CLN3, comme mesuré sur la longueur totale de l'oligonucléotide ; et
(ii) l'oligonucléotide modifié ne comprend pas plus de 4 2'-désoxynucléosides non modifiés contigus ; et
où le composé est capable d'induire le saut d'un ou plusieurs exons du produit de transcription de CLN3,
pour utilisation dans le traitement de la maladie de Batten ou pour utilisation dans le retardement ou la prévention de l'apparition de la maladie de Batten.

2. Composé pour utilisation selon la revendication 1, dans lequel la région cible du produit de transcription de CLN3 comprend au moins une partie de l'exon 6, l'exon 9, l'intron 5, l'intron 6, l'intron 9 ou l'intron 10 du produit de transcription de CLN3, et dans lequel, facultativement, le composé peut éviter le décalage du cadre de lecture qui conduit à un codon de terminaison prématuré dans l'exon 9 du produit de transcription de CLN3.

3. Composé pour utilisation selon la revendication 1 ou la revendication 2, dans lequel la région complémentaire de l'oligonucléotide modifié comprend au moins 10, au moins 12, au moins 14, au moins 15, au moins 16, au moins 17, au moins 18, au moins 19, ou au moins 20 bases azotées contiguës.

4. Composé pour utilisation selon l'une quelconque des revendications 1 à 3, dans lequel la séquence de bases azotées de l'oligonucléotide est 100 % complémentaire d'une région de longueur égale du produit de transcription de CLN3, comme mesuré sur la longueur totale de l'oligonucléotide.

5. Composé pour utilisation selon l'une quelconque des revendications 1 à 4, dans lequel l'oligonucléotide modifié comprend au moins un nucléoside modifié.

6. Composé pour utilisation selon la revendication 5, dans lequel au moins un nucléoside modifié comprend un fragment de sucre modifié.

7. Composé pour utilisation selon la revendication 6, dans lequel au moins un fragment de sucre modifié est un fragment de sucre substitué en 2'.

8. Composé pour utilisation selon la revendication 7, dans lequel le substituant en 2' d'au moins un fragment de sucre substitué en 2' est choisi parmi : 2'-OMe, 2'-F, et 2'-MOE.

9. Composé pour utilisation selon l'une quelconque des revendications 1 à 8, dans lequel l'oligonucléotide modifié comprend au moins 5 nucléosides modifiés, au moins 10 nucléosides modifiés, ou au moins 15 nucléosides modifiés, chacun comprenant indépendamment un fragment de sucre modifié.

10. Composé pour utilisation selon la revendication 9, dans lequel chaque nucléoside de l'oligonucléotide modifié est un nucléoside modifié, chacun comprenant indépendamment un fragment de sucre modifié.

11. Composé pour utilisation selon la revendication 9 ou la revendication 10, dans lequel chaque fragment de sucre modifié est un 2'-MOE.

12. Composition pharmaceutique comprenant un composé comprenant un oligonucléotide modifié constitué de 8 à 30 nucléosides liés et ayant une séquence de bases azotées comprenant une région complémentaire, dans laquelle la région complémentaire comprend au moins 8 bases azotées contiguës et est 100 % complémentaire d'une partie de longueur égale d'une région cible d'un produit de transcription de CLN3, dans laquelle :
(i) la séquence de bases azotées de l'oligonucléotide est au moins 90 % complémentaire d'une région de longueur égale du produit de transcription de CLN3, comme mesuré sur la longueur totale de l'oligonucléotide ; et
(ii) l'oligonucléotide modifié ne comprend pas plus de 4 2'-désoxynucléosides non modifiés contigus ; et
où le composé est capable d'induire le saut d'un ou plusieurs exons du produit de transcription de CLN3, et un véhicule ou diluant pharmaceutiquement acceptable,
pour utilisation dans le traitement de la maladie de Batten ou pour utilisation dans le retardement ou la prévention de l'apparition de la maladie de Batten.

13. Composition pharmaceutique pour utilisation selon la revendication 12, la composition comprenant un sel pharmaceutiquement acceptable du composé.

14. Composition pharmaceutique pour utilisation selon la revendication 13, dans laquelle le sel pharmaceutiquement acceptable est un sel de sodium ou de potassium.
